(19) Europäisches Patentamt
European Patent Office
Office européen des brevets

(11) EP 4 660 184 A1

(12) **EUROPEAN PATENT APPLICATION**
published in accordance with Art. 153(4) EPC

(43) Date of publication:
**10.12.2025 Bulletin 2025/50**

(21) Application number: **24749440.4**

(22) Date of filing: **26.01.2024**

(51) International Patent Classification (IPC):
***C07C 233/64*** *(2006.01)* ***C07C 15/00*** *(2006.01)*
***C07C 15/12*** *(2006.01)* ***C07D 213/00*** *(2006.01)*
***C07D 401/00*** *(2006.01)* ***C07D 403/00*** *(2006.01)*
***A61K 31/166*** *(2006.01)* ***A61K 31/435*** *(2006.01)*
***A61K 31/495*** *(2006.01)* ***A61K 31/609*** *(2006.01)*
***A61P 25/02*** *(2006.01)* ***A61P 25/04*** *(2006.01)*
***A61P 29/00*** *(2006.01)*

(52) Cooperative Patent Classification (CPC):
**A61K 31/166; A61K 31/435; A61K 31/495; A61K 31/609; A61P 25/02; A61P 25/04; A61P 29/00; C07C 15/00; C07C 15/12; C07C 233/64; C07D 213/00; C07D 401/00; C07D 403/00**

(86) International application number:
**PCT/BR2024/050027**

(87) International publication number:
**WO 2024/159287 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 US 202363482216 P**

(71) Applicants:
- **Eurofarma Laboratórios S.A.**
  **06696-000 São Paulo - SP (BR)**
- **Universidade Federal Do Rio De Janeiro - UFRJ**
  **21941-853 Rio de Janeiro -RJ (BR)**

(72) Inventors:
- **BARREIRO, Gabriela**
  **SP 04514-032 São Paulo (BR)**
- **SANT'ANA, Danilo Pereira De**
  **SP 06709-320 São Paulo (BR)**
- **MONTEIRO, Júlia Lammoglia**
  **SP 06704175 São Paulo (BR)**
- **GAMBA, Luis Eduardo Reina**
  **SP 06404-326 São Paulo (BR)**
- **FRAGA, Carlos Alberto Manssour**
  **(deceased) (BR)**
- **BARREIRO, Eliezer Jesus De Lacerda**
  **(deceased) (BR)**
- **LIMA, Lídia Moreira**
  **RJ 21931-220 Rio de Janeiro (BR)**

(74) Representative: **Engelhard, Markus**
**Boehmert & Boehmert**
**Anwaltspartnerschaft mbB**
**Pettenkoferstrasse 22**
**80336 München (DE)**

(54) **NAV1.7- AND/OR NAV1.8-INHIBITING HYDROXAMATES, PROCESSES FOR THE PREPARATION THEREOF, COMPOSITIONS, USES, METHODS FOR TREATMENT USING SAME, AND KITS**

(57) The present invention relates to Nav 1.7 and/or Nav 1.8 blocking hydroxamates. More specifically, the present invention is related to hydroxamates comprising the Formula (I), in which the substituents $R_1$ to $R_{10}$, as well as their derivatives $R_{11}$ to $R_{20}$, are selected independently of the groups defined in the specification, as well as their preparation processes, compositions comprising at least one of these compounds, uses, treatment methods to treat or prevent pain-related pathologies and kits. The present invention belongs to the fields of medicinal chemistry, organic synthesis, as well as the treatment of pain-related diseases.



Processed by Luminess, 75001 PARIS (FR)

(Cont. next page)

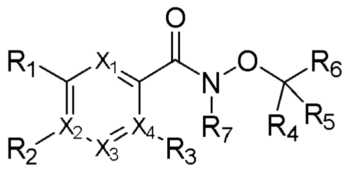 Formula (I)

## Description

## FIELD OF INVENTION

The present invention refers to Nav 1.7 and/or Nav 1.8 blocking hydroxamates, their preparation processes, compositions containing these, uses, kits and treatment methods to treat or prevent pain-related pathologies. The present invention belongs to the fields of medicinal chemistry, organic synthesis, as well as the treatment of pain-related diseases.

## BACKGROUND OF INVENTION

Physiological pain is an important protective mechanism designed to alert the body to real or potential injuries that can put the integrity thereof at risk. Broadly speaking, physiological pain can be classified into nociceptive pain and inflammatory pain. Nociceptive pain is characterized by having a high activation threshold, which remains until the stimulus that generated it is eliminated. Inflammatory pain, which arises as a response to tissue damage, is characterized by having a low activation threshold and is a consequence of the activity of molecular mediators of the inflammatory process in sensitizing nociceptors (Schaible. Langenbecks Arch. Surg. 2004, 389, 237). When these nociceptive processes remain in the absence of noxious stimuli or in response to não noxious stimuli, the protective and repair role of pain loses its functionality, configuring a maladaptive picture of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or central nervous system diseases such as multiple sclerosis or Parkinson's disease (Ducreux. Brain, 2006, 129, 963). Peripheral neuropathic pain, on the other hand, can be caused by trauma, metabolic disorders, chemical neurotoxicity, infection, or tumor invasion, among others. Among the most common syndromes of neuropathic pain are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia, and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. However, the adverse effects and low efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization, allowing the generation and propagation of action potentials in neurons (and other electrically excitable cells), by controlling the flow of sodium ions through the membranes. Structurally, voltage-gated sodium channels are heteromeric transmembrane proteins consisting of one $\alpha$ subunit and two $\beta$ helper subunits. The $\alpha$ subunit is organized into four homologous domains (I-IV), each with six transmembrane segments (S1-S6). The S4 segment of each domain is characterized by presenting a conserved region of arginine residues, which act as sensors of the intra- and extracellular electrical environment of the neuron. This mechanism makes it possible to transform changes in the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine subunits $\alpha$ (Nav 1.1 - Nav 1.9) and four auxiliary subunits $\beta$ ($\beta$1-$\beta$4) have been identified. The $\alpha$ subunits can also be classified according to their susceptibility to blocking by tetrodotoxin (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these subunits $\alpha$ has a different profile of expression and function, so that some of them are essential for the proper functioning of organs such as the heart and/or brain. Thus, the non-selective blockage of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

Broadly speaking, sodium channels are distributed mainly in the central and peripheral nervous system, in neurons and glia. Nav channels 1.1, 1.2, and 1.3 are primarily expressed in the brain. The Nav 1.4 and Nav 1.5 channels are found primarily in skeletal and cardiac muscles, respectively. Nav 1.6 channels are expressed in the central and peripheral nervous systems, while Nav 1.9 channels are selectively expressed in C-type nociceptive fibers in the dorsal root ganglion. On the other hand, the Nav 1.7 and Nav 1.8 channels are mainly found in the peripheral nervous system and

are directly related to the processes of pain transmission (Law. Drug Discovery Today, 2019, 24(7), 1389; Bagal. Channels (Austin), 2015, 9(6), 360; Lera-Ruiz. J. Med. Chem., 2015, 58 (18), 7093).

Nav 1.7 sodium channels are expressed broadly in the olfactory epithelium, sympathetic ganglion, and dorsal root ganglion, predominantly in nociceptive fibers C and A$\delta$. A large amount of evidence supports the important role of Nav 1.7 sodium channels in pain transmission processes. For example, gain-of-function related mutations in the gene (SCN9A), which encodes sodium channel Nav 1.7, are associated with extreme pain disorders such as congenital pain insensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels are most expressed in the peripheral nervous system, widely (but not exclusively) in C-type nociceptive fibers in the dorsal root ganglion. Recent evidence including elevated expression levels of Nav 1.8 in chronic pain states, data with Nav 1.8 *knockout* animals, and analgesic activity of Nav 1.8-specific desensitizing oligodeoxynucleotides, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the sodium channel Nav 1.8 in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

Thus, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels, however, they present a great structural diversity, a fact that does not allow the establishment of a common pharmacophoric group.

The patent literature contains several examples of compounds that act as sodium channel blockers. In particular, Nav 1.7 selective sodium channel blockers are described in US10550080, US9765029, and US10000475. Additionally, some documents describe selective blockers of Nav 1.8 sodium channels such as WO2020261114, WO2020092667, US9163042, WO2014120808, WO2014120815, WO2018213426, WO2019014352, WO2015006280, and US7928107. These documents reveal compounds with different structures from the present invention.

In addition, there are patent documents that describe dual Nav blockers 1.7 and 1.8, including WO2018235851, US8629149, JP2017001991 that reveal, respectively, pyridyl amines, oxopiperazine derivatives and benzoxazolons. However, all these documents reveal compounds with structures and physicochemical characteristics different from the present invention.

In this context, it is advantageous to develop new alternatives of compounds that can act as Nav 1.7 and/or Nav 1.8 blockers that have adequate pharmacological action and, preferably, provide mitigated adverse effects. Therefore, the present invention refers to hydroxamates as an alternative and/or complement to the treatment of pain-related diseases.

## SUMMARY OF THE INVENTION

The present invention discloses hydroxamates with blocking activity of Nav 1.7 and/or 1.8 channels, against pain-related pathologies, as well as related compositions, uses, kits, treatment methods and preparation processes.

The present invention refers to compound(s) of Formula (I):

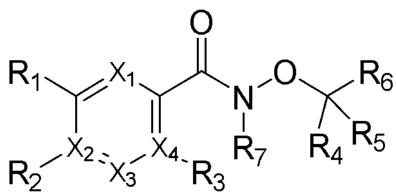

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, COH, or nitrogen; wherein

- when at least one of the substituents $X_2$, $X_4$ is nitrogen or CH, the corresponding R ($R_2$, $R_3$) is null.
- $R_1$ is:

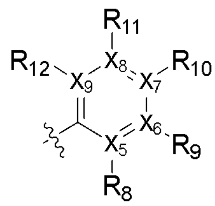
,

wherein:

- $X_5$, $X_6$, $X_7$, $X_8$, and $X_9$ are independently selected from the group consisting of carbon, CH, or nitrogen;

wherein:

- when at least one of the substituents $X_5$-$X_9$ is nitrogen or CH, the corresponding R ($R_8$-$R_{12}$) is null; and
- $R_8$, $R_9$, $R_{10}$, $R_{11}$, and $R_{12}$ are independently selected from the group consisting of hydrogen, halogen, linear or branched $C_1$-$C_6$ alkyl, $C_1$-$C_6$ linear or branched alkoxy, hydroxy, haloalkoxy $C_1$-$C_6$ linear or branched, azetidinyloxy, azetidinylalkoxy $C_1$-$C_6$, alkylazetidinyloxy $C_1$-$C_6$, alkylazetidinylalkoxy $C_1$-$C_6$, alkylsulfinyl $C_1$-$C_6$, alkoxy $C_1$-$C_6$ containing one or more isotopically enriched atoms such as hydrogen isotope $^2H$ but not limited to same;
- $R_2$ and $R_3$ are independently selected from the group consisting of hydrogen, halogen, amine, hydroxy, alkoxy $C_1$-$C_6$ linear or branched, alkyl $C_1$-$C_6$ linear or branched;
- $R_4$ and $R_5$ are independently selected from the group consisting of hydrogen, deuterium, haloalkyl $C_1$-$C_6$ linear or branched, alkyl $C_1$-$C_6$ linear or branched;
- $R_6$ is selected from the group consisting of aryl, pyridinyl, or substituted pyridinyl, or $R_{13}$ or $R_{14}$:

  - $R_{13}$ is:

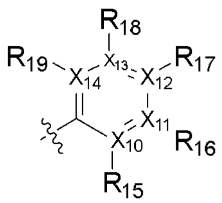
,

  wherein:

  - $X_{10}$, $X_{11}$, $X_{12}$, $X_{13}$ and $X_{14}$ are independently selected from the group consisting of carbon, CH or nitrogen, wherein:

    - when at least one of the substituents $X_{10}$-$X_{14}$ is nitrogen or CH, the corresponding R ($R_{15}$-$R_{19}$) is null,
    - $R_{15}$ $R_{16}$, $R_{17}$, $R_{18}$, and $R_{19}$ are independently selected from the group consisting of hydrogen, halogen, hydroxy, linear or branched $C_1$-$C_6$ alkyl, cycloalkyl $C_3$-$C_6$, $C_1$ -$C_6$ alkoxy linear or branched, cycloalkoxy $C_3$-$C_6$, hydroxyalkyl $C_1$-$C_4$ linear or branched, aminoalkyl $C_1$-$C_6$ linear or branched, alkoxy $C_1$-$C_6$ containing one or more isotopically enriched atoms, such as hydrogen isotope $^2H$, but not limited to the same.
    - $R_{14}$ is selected from the group consisting of:

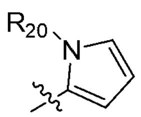
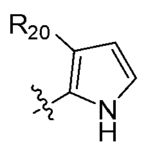
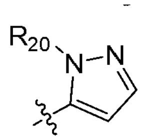
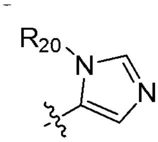
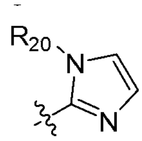

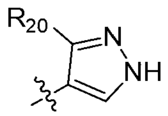 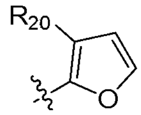 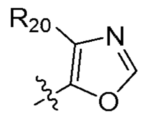 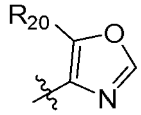 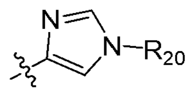

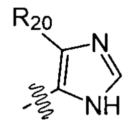 ,

wherein $R_{20}$ is independently selected from the group consisting of hydrogen, halogen, hydroxy, hydroxyalkyl $C_{1-}C_4$ linear or branched, alkyl $C_1$-$C_6$ linear or branched, alkoxy $C_1$-$C_4$ linear or branched, cycloalkoxy $C_3$-$C_7$, haloalkoxy, nitrile, carboxyl, carboxyalkyl ester $C_1$-$C_3$, amine, N-alkylamine, N,N-dialkylamine, aminoalkyl $C_{1-}C_6$ linear or branched, sulfonamide, alkylsulfonylamide $C_1$-$C_6$, N,N-dialkylsulfonylamide $C_1$-$C_6$, sulfonyl, alkylsulfonyl $C_1$-$C_6$, sulfinyl, alkyl sulfinyl $C_1$-$C_6$, azetidiniloxy, azetidinylalkoxy $C_1$-$C_3$, alkoxy $C_1$-$C_2$ linear or branched, hydroxyazetidinyl.

- $R_7$, is independently selected from the group consisting of hydrogen and alkyl $C_1$-$C_6$ linear or branched.

Additionally, the present invention further refers to compositions comprising one or more compound(s) of Formula (I) or a salt, hydrate, solvate and pharmaceutically acceptable isomer thereof; and one or more pharmaceutically acceptable excipients.

In addition, kits in accordance with this invention may comprise such compositions and application devices, which may include ampoules, syringes and others. Alternatively, the kits according to this invention comprise more than one compound of Formula (I) arranged in one or more dosage forms, including without limitation, tablets, accompanied by administration instructions.

The present invention further refers to methods of treatment, prevention, relief, suppression and/or control of diseases related to neuropathic pain. Uses of Formula (I) compound(s) to prepare a drug for the treatment of pathologies related to neuropathic pain are also taught. Finally, the present invention teaches processes for obtaining compound(s) of Formula (I).

## DETAILED DESCRIPTION OF THE INVENTION

The present invention presents, in a first embodiment, the compound of Formula (I):

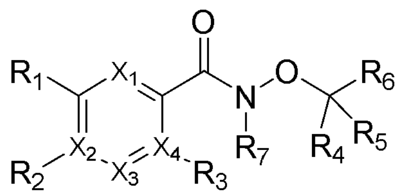

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, COH, or nitrogen;

wherein:

- when at least one of the substituents $X_2$, $X_4$ is nitrogen or CH, the corresponding R ($R_2$, $R_3$) is null.
- $R_1$ is:

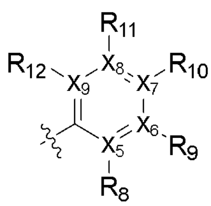 ,

wherein:

- $X_5$, $X_6$, $X_7$, $X_8$, and $X_9$ are independently selected from the group consisting of carbon, CH, or nitrogen;

wherein:

- when at least one of the substituents $X_5$-$X_9$ is N or CH, the corresponding R ($R_8$-$R_{12}$) is null; and $R_8$, $R_9$, $R_{10}$, $R_{11}$, and $R_{12}$ are independently selected from the group consisting of hidrogen, halogen, alkyl $C_1$-$C_6$ linear or branched, alkoxy $C_1$-$C_6$ linear or branched, azetidinyloxy, azetidinylalkoxy $C_1$-$C_6$, alkylazetidiniloxy $C_1$-$C_6$, alkylazetidinylalkoxy $C_1$-$C_6$, alkylsulfinyl $C_1$-$C_6$, trifluoromethoxy, hydroxy, difluoromethoxy, alkoxy $C_1$-$C_6$ containing one or more isotopically enriched atoms such as hydrogen isotope $^2$H, but not limited to the same.
- $R_2$ and $R_3$ are independently selected from the group consisting of hydrogen, halogen, amine, hydroxy, alkoxy $C_1$-$C_6$ linear or branched, alkyl $C_1$-$C_6$ linear or branched;
- $R_4$ and $R_5$ are independently selected from the group consisting of hydrogen, deuterium, haloalkyl $C_1$-$C_6$ linear or branched, alkyl $C_1$-$C_6$ linear or branched;
- $R_6$ is selected from the group consisting of aryl, pyridinyl, or substituted pyridinyl, or $R_{13}$ or $R_{14}$;
- $R_{13}$ is:

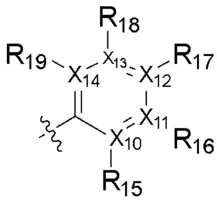

,

wherein:

- $X_{10}$, $X_{11}$, $X_{12}$, $X_{13}$ and $X_{14}$ are independently selected from the group consisting of carbon, CH or nitrogen, wherein:

  - when at least one of the substituents $X_{10}$-$X_{14}$ is nitrogen or CH, the corresponding R ($R_{15}$-$R_{19}$) is null; and
  - $R_{15}$ $R_{16}$, $R_{17}$, $R_{18}$, and $R_{19}$ are independently selected from the group consisting of hydrogen, halogen, hydroxy, linear or branched alkyl $C_1$-$C_6$, cycloalkyl $C_3$-$C_6$, saturated or unsaturated cycloalkoxy-$C_3$-$C_6$, alkoxy $C_1$-$C_6$ linear or branched, hydroxyalkyl $C_1$-$C_6$ linear or branched, $C_1$-$C_6$ linear or branched aminoalkyl, alkoxy $C_1$-$C_6$ containing one or more isotopically enriched atoms, such as hydrogen isotope $^2$H, but not limited to the same;
  - $R_{14}$ is selected from the group consisting of:

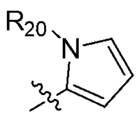
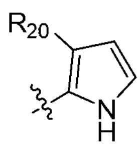
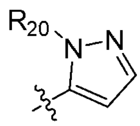
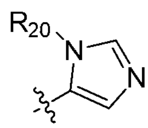
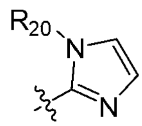

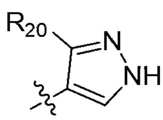
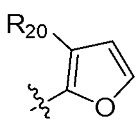
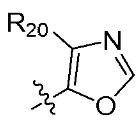
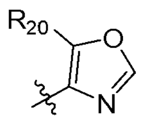
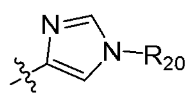

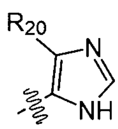

,

wherein $R_{20}$ is independently selected from the group consisting of hydrogen, halogen, hydroxy, hydroxyalkyl $C_1$-$C_4$ linear or branched, alkyl $C_1$-$C_6$ linear or branched, alkoxy $C_1$-$C_6$ linear or branched, cycloalkoxy $C_3$-$C_7$, haloalkoxy, nitrile, carboxyl, carboxyalkyl ester $C_1$-$C_6$, amine, N-alkylamine, N,N-dialkylamine,

aminoalkyl $C_1$-$C_6$ linear or branched, sulfonamide, alkylsulfonylamide $C_1$-$C_6$, N,N-dialkylsulfonylamide $C_1$-$C_6$, sulfonyl, alkylsulfonyl $C_1$-$C_6$, sulfinyl, alkylsulfinyl $C_1$-$C_6$, azetidiniloxy, azetidinylalkoxy $C_1$-$C_6$, linear or branched alkoxy $C_1$-$C_6$, hydroxyazetidinyl.

- $R_7$, is independently selected from the group consisting of hydrogen and alkyl $C_1$-$C_6$ linear or branched.

As described herein, the compounds of the present invention comprise multiple variable groups (R, $X_1$, etc.). As a person skilled in the art will recognize, the group combinations contemplated by this invention are those combinations that result in the formation of stable or chemically viable compounds.

The term "stable" in this context refers to compounds that are not substantially altered when subjected to conditions that allow their production, detection and preferably their recovery, purification and use for one or more of the purposes disclosed herein.

In some embodiments, a stable compound or chemically viable compound is one that is not substantially altered when kept at a temperature of 40 °C or less, in the absence of moisture or other chemically reactive conditions, for at least one week.

In addition, some compounds described in this invention may exist as tautomers, so the individual tautomers, as well as their mixtures, are included in the compounds with structural formula I.

EMBODIMENTS

In an embodiment, $X_1$, $X_2$, $X_3$, and $X_4$ are independently selected from the group consisting of carbon, CH, COH, or nitrogen, where when at least one of the substituents $X_2$, $X_4$ is nitrogen, CH, or COH, the corresponding R ($R_2$, $R_3$) is null; $R_1$ is

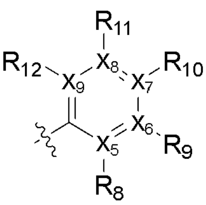

,

where $X_5$, $X_6$, $X_7$, $X_8$, and $X_9$ are independently selected from the group consisting of carbon, CH, or nitrogen, where when at least one of the substituents $X_5$-$X_9$ is nitrogen or CH, the corresponding R ($R_8$-$R_{12}$) is null; $R_8$, $R_9$, $R_{10}$, $R_{11}$, $R_{12}$ are independently selected from the groups consisting of hydrogen, halogen, linear or branched $C_1$-$C_6$ alkyl, alkoxy $C_1$-$C_6$ linear or branched, haloalkoxy $C_1$-$C_6$ linear or branched, hydroxy, azetidinyloxy, azetidinylalkoxy $C_1$-$C_6$, alkylazetidinylalkoxy $C_1$-$C_6$, alkylsulfinyl $C_1$-$C_6$, alkoxy $C_1$-$C_2$ containing one or more isotopically enriched atoms such as hydrogen isotopes $^2$H, but not limited to the same.

In an embodiment, $R_2$ and $R_3$ are independently selected from the group consisting of hydrogen, halogen, amine, hydroxy, linear or branched $C_1$-$C_6$ alkyl, linear or branched $C_1$-$C_6$ alkoxy.

In an embodiment, $R_4$ and $R_5$ are independently selected from the group consisting of hydrogen, deuterium, linear or branched $C_1$-$C_6$haloalkyl, alkyl $C_1$-$C_6$ linear or branched.

In an embodiment, $R_6$ is selected from the group consisting of aryl, pyridinyl, or substituted pyridinyl, or $R_{13}$ or $R_{14}$, where $R_{13}$ is

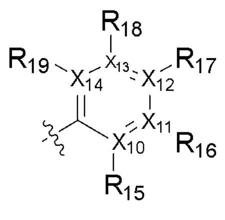

,

wherein $X_{10}$, $X_{11}$, $X_{12}$, $X_{13}$, and $X_{14}$ are independently selected from the group consisting of carbon, CH, or nitrogen; when at least one of the substituents $X_{10}$-$X_{14}$ is nitrogen or CH, the corresponding R ($R_{15}$-$R_{19}$) is null.

In an embodiment $R_{15}$, $R_{16}$, $R_{17}$, $R_{18}$ and $R_{19}$ are independently selected from the group consisting of hydrogen, halogen, hydroxy, linear or branched $C_1$-$C_6$ alkyl, cycloalkyl $C_3$-$C_7$ saturated or unsaturated, linear or branched $C_1$-$C_6$ alkoxy, $C_3$-$C_7$ cycloalkoxy, $C_1$-$C_6$ linear or branched hydroxyalkyl, $C_1$-$C_6$aminoalkyl, linear or branched, $C_1$-$C_2$ alkoxy containing one or more isotopically enriched atoms, such as hydrogen isotope $^2$H, but not limited to the same.

In an embodiment $R_{14}$ is selected from the group consisting of:

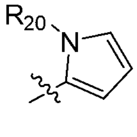
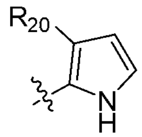
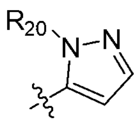
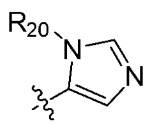
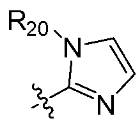

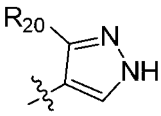
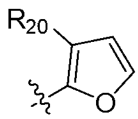
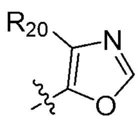
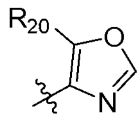
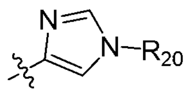

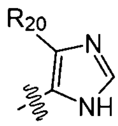 ,

where $R_{20}$ is selected from the group consisting of hydrogen, halogen, hydroxy, hydroxyalkyl $C_1$-$C_6$ linear or branched, alkyl $C_1$-$C_6$ linear or branched, alkoxy $C_1$-$C_6$ linear or branched, cycloalkoxy $C_{>53}$-$C_7$ saturated or unsaturated, haloalkoxy $C_1$-$C_6$ linear or branched, nitrile, carboxyl, carboxyalkyl ester $C_1$-$C_6$, amine, N-alkylamine, N,N-dialkylamine, linear or branched $C_1$-$C_6$ aminoalkyl, sulfonamide, alkylsulfonylamide $C_1$-$C_6$, N,N-dialkylsulfonylamide $C_1$-$C_6$, sulfonyl, alkylsulfinyl $C_1$-$C_6$, sulfinyl, alkyl sulfinyl $C_1$-$C_6$, azetidinyloxy, azetidinylalkoxy $C_1$-$C_6$, alkoxy $C_1$-$C_6$ linear or branched, hydroxyazetidinyl.

In an embodiment, $R_7$ is selected from the group consisting of hydrogen and alkyl $C_1$-$C_6$ linear or branched.

In an embodiment, $X_1$, $X_2$, and $X_4$ are carbon or nitrogen. In a preferred embodiment, $X_3$ is carbon, COH, or nitrogen.

In an embodiment, $R_1$ is

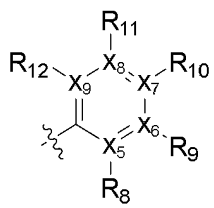 .

In an embodiment, $X_5$, $X_6$, $X_7$, $X_8$, and $X_9$ are independently selected from the group consisting of carbon, CH, and nitrogen. Preferably, $X_5$, $X_7$, and $X_9$ are carbon. Preferably $X_6$ and $X_8$ are carbon or nitrogen.

In a preferred embodiment, $R_8$ is hydrogen or hydroxy.

In a preferred embodiment, $R_9$ is hydrogen, hydroxy, or null when $X_6$ is nitrogen.

In an embodiment, $R_{10}$ is hydroxy, methoxy, ethoxy, propoxy, isopropoxy, n-butoxy, sec-butoxy, tert-butoxy, azetidinyloxy, azetidinylmethoxy, azetidinylethoxy, azetidinylpropoxy, 1-methylazetidine-3-ylmethoxy, 2-methylazetidine3-ylmethoxy, 1-ethylazetidine-3-ylmethoxy, 2-ethylazedine-3-ylmethoxy, 1-methylazetidine3-ylmethoxy, 2-methylazetidine3-ylmetoxy, 1-methylazetine-3-ylpropoxy, chloromethoxy, dichloromethoxy, trichloromethoxy, fluoromethoxy, difluoromethoxy, trifluoromethoxy, methylsulfinyl, ethylsulfinyl, methoxy-$d_1$, methoxy-$d_2$, methoxy-$d_3$, ethoxy-1-$d_1$, ethoxy-2-$d_1$, ethoxy-1,1-$d_2$, ethoxy-1,1-d, ethoxy-1,2-$d_2$, ethoxy-2,2-$d_2$, ethoxy-1,1,1-$d_3$, ethoxy-1,1,2-$d_3$, ethoxy-1,2,2-$d_3$, ethoxy-2,2,2-$d_3$, ethoxy-1,1,2,2-$d_4$, ethoxy-1,2,2,2-$d_4$, ethoxy-$d_5$. Preferably, $R_{10}$ is selected from the group consisting of hydroxy, methoxy, ethoxy, isopropoxy, azetidinyloxy, azetidinylmethoxy, 1-methylazetidine-3-ylmethoxy, 1-methylazetidine3-ylethoxy, methylsulfinyl, ethoxy-$d_5$, ethoxy-1,1-$d_2$, ethoxy-2,2,2-$d_3$, trifluoromethoxy, and difluoromethoxy.

In an embodiment, $R_{11}$ is hydrogen or null when $X_8$ is nitrogen.

In an embodiment, $R_{12}$ is hydrogen, hydroxy, methyl, ethyl, propyl, isopropyl, butyl, isobutyl, sec-butyl, tert-butyl, methoxy, ethoxy, propoxy, isopropoxy, butoxy, isobutoxy, sec-butoxy, and tert-butoxy. In a preferred embodiment, $R_{12}$ is selected from the group consisting of hydrogen, hydroxy, methyl, and methoxy.

In an embodiment, $R_2$ is hydrogen, hydroxy, amine, fluorine, chlorine, bromine, methyl, ethyl, propyl, isopropyl,

butyl, isobutyl, sec-butyl, tert-butyl, methoxy, ethoxy, propoxy, isopropoxy, butoxy, isobutoxy, sec-butoxy, and tert-butoxy, or null when $X_2$ is nitrogen. In a preferred embodiment, $R_2$ is hydrogen, hydroxy, amine, fluorine, or null when $X_2$ is nitrogen.

In an embodiment, $R_3$ is hydrogen, hydroxy, amine, fluorine, chlorine, bromine, methyl, ethyl, propyl, isopropyl, butyl, isobutyl, sec-butyl, tert-butyl, methoxy, ethoxy, propoxy, isopropoxy, butoxy, isobutoxy, sec-butoxy, tert-butoxy, or null when $X_4$ is nitrogen. In a preferred embodiment, $R_3$ is hydrogen, hydroxy, amine, fluorine, or null when $X_4$ is nitrogen.

In an embodiment, $R_4$ is hydrogen, deuterium, methyl, ethyl, propyl, isopropyl, butyl, isobutyl, sec-butyl, or tert-butyl. In a preferred embodiment, $R_4$ is hydrogen, deuterium, or methyl.

In an embodiment, $R_5$ is hydrogen, deuterium, methyl, ethyl, propyl, isopropyl, butyl, isobutyl, sec-butyl, or tert-butyl. In a preferred embodiment, $R_5$ is hydrogen, deuterium, or methyl.

In an embodiment, $R_6$ is aryl, pyridinyl, substituted pyridinyl, $R_{13}$, or $R_{14}$. Preferably, $R_6$ is $R_{13}$ or $R_{14}$.

In a preferred embodiment, $R_{13}$ is

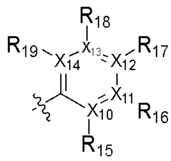
.

In an embodiment, $X_{10}$, $X_{11}$, $X_{12}$, and $X_{13}$ are carbon, CH, or nitrogen. Preferably, they are carbon or nitrogen, wherein, when they are nitrogen, their respective R ($R_{15}$, $R_{16}$, $R_{17}$, $R_{18}$) is null.

In an embodiment $X_{14}$ is carbon, CH, or nitrogen. Preferably, $X_{14}$ is carbon.

In an embodiment, $R_{15}$ is hydrogen or null when $X_{10}$ is nitrogen.

In an embodiment, $R_{16}$ is hydrogen, hydroxy, methyl, ethyl, propyl, isopropyl, butyl, isobutyl, sec-butyl, tert-butyl, methoxy, ethoxy, propoxy, isopropoxy, butoxy, isobutoxy, sec-butoxy, *tert*}-butoxy, methylamine, ethylamine, propylamine, dimethylamine, diethylamine, dipropylamine, cyclopropoxy, cyclobutoxy, cyclopentoxy, cyclohexyloxy, methoxy-$d_1$, methoxy-$d_2$, methoxy-$d_3$, ethoxy-1-$d_1$, ethoxy-2-$d_1$, ethoxy-1,1-$d_2$, ethoxy-1,2-$d_2$, ethoxy-2,2-$d_2$, ethoxy-1,1,1-$d_3$, ethoxy-1,1,2-$d_3$, ethoxy-1,2,2-$d_3$, ethoxy-2,2,2-$d_3$, ethoxy-1,1,2,2-$d_4$, ethoxy-1,2,2,2-$d_4$, ethoxy-d5 or null when $X_{11}$ is nitrogen. In a preferred embodiment, $R_{16}$ is hydrogen, hydroxy, methoxy, isopropoxy, ethylamine, dimethylamine, methyl, cyclobutoxy, methoxy-d3, or null when $X_{11}$ is nitrogen.

In an embodiment $R_{17}$ is hydrogen or null when $X_{12}$ is nitrogen.

In an embodiment $R_{18}$ is hydrogen, methyl, ethyl, propyl, isopropyl, butyl, isobutyl, sec-butyl, *tert*-butyl, methoxy, ethoxy, propoxy, isopropoxy, butoxy, isobutoxy, sec-butoxy, tert-butoxy, or null when $X_{13}$ is nitrogen. In a preferred embodiment, $R_{18}$ is hydrogen, methyl, methoxy, or null when $X_{13}$ is nitrogen.

In an embodiment, $R_{19}$ is hydrogen, hydroxy, fluorine, chlorine, bromine, methyl, ethyl, propyl, isopropyl, butyl, isobutyl, sec-butyl, tert-butyl, cycloheptyl, cyclopropyl, cyclobutyl, cyclopentyl, cyclopentenyl, cycloheptenyl, cyclobutenyl, cyclopropenyl, cyclohexenyl, or null when $X_{14}$ is nitrogen. In a preferred embodiment, $R_{19}$ is hydrogen, fluorine, chlorine, hydroxy, methyl, cyclopropyl, or null when $X_{14}$ is nitrogen.

In an embodiment, $R_{14}$ is

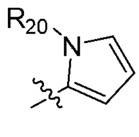 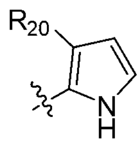 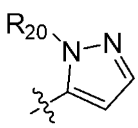 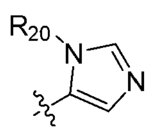 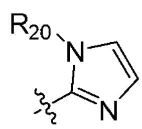

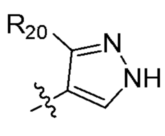 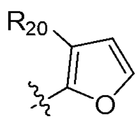 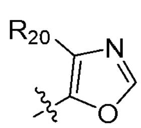 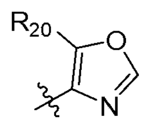 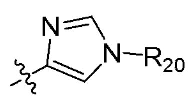

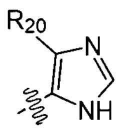
.

In an embodiment, $R_{20}$ is methyl, ethyl, propyl, isopropyl, butyl, isobutyl, *sec*-butyl, *tert*-butyl, nitrile, methoxy, ethoxy, propoxy, isopropoxy, butoxy, isobutoxy, *sec*-butoxy, *tert*-butoxy. In a preferred embodiment, $R_{20}$ is nitrile, methoxy,

methyl, ethyl, isopropyl.

In an implementation of the first embodiment, the Formula (I) compound(s) is selected from the group(s) consisting of:

*N*-((2-fluoro-5-methoxybenzyl)oxy)-6-(4-hydroxyphenyl)pyrazine-2-carboxamide (Compound 1);
*N*-((2-fluoro-5-methoxybenzyl)oxy)-6-(4-methoxyphenyl)pyrazine-2-carboxamide (Compound 2);
*N*-((2-fluoro-5-methoxybenzyl)oxy)-6-(4-isopropoxyphenyl)pyrazine-2-carboxamide (Compound 3);
6-(4-ethoxyphenyl)-*N*-((2-fluorobenzyl)oxy)pyrazine-2-carboxamide (Compound 4);
6-(4-ethoxyphenyl)-*N*-((3-methoxybenzyl)oxy)pyrazine-2-carboxamide (Compound 5);
6-(4-ethoxyphenyl)-*N*-((2-hydroxybenzyl)oxy)pyrazine-2-carboxamide (Compound 6);
6-(4-ethoxyphenyl)-*N*-((3-hydroxybenzyl)oxy)pyrazine-2-carboxamide (Compound 7);
6-(4-ethoxyphenyl)-*N*-((5-methoxypyridine-3-yl)methoxy)pyrazine-2-carboxamide (Compound 8);
6-(4-ethoxyphenyl)-*N*-((2-fluoro-5-methoxybenzyl)oxy)pyrazine-2-carboxamide (Compound 9);
6-(4-ethoxyphenyl)-*N*-((2-fluoro-5-isopropoxybenzyl)oxy)pyrazine-2-carboxamide (Compound 10);
*N*-((2-chloro-5-methoxybenzyl)oxy)-6-(4-ethoxyphenyl)pyrazine-2-carboxamide (Compound 11);
6-(4-ethoxyphenyl)-*N*-((2-fluoro-5-methoxypyridine-3-yl)methoxy)pyrazine-2-carboxamide (Compound 12);
6-(4-ethoxyphenyl)-*N*-((5-fluoro-2-methoxypyridine-4-yl)methoxy)pyrazine-2-carboxamide (Compound 13);
6-(4-ethoxyphenyl)-*N*-((2-fluoro-5-hydroxybenzyl)oxy)pyrazine-2-carboxamide (Compound 14);
*N*-((2-cyclopropyl-5-methoxybenzyl)oxy)-6-(4-ethoxyphenyl)pyrazine-2-carboxamide (Compound 15);
6-(4-ethoxyphenyl)-*N*-((2-hydroxy-5-methoxybenzyl)oxy)pyrazine-2-carboxamide (Compound 16);
*N*-((5-(1-aminoethyl)-2-fluorobenzyl)oxy)-6-(4-ethoxyphenyl)pyrazine-2-carboxamide (Compound 17);
6-(4-ethoxyphenyl)-*N*-((5-methoxy-2-methylpyridine-3-yl)methoxy)pyrazine-2-carboxamide (Compound 18);
6-(4-ethoxyphenyl)-*N*-((5-methoxy-2-methylbenzyl)oxy)pyrazine-2-carboxamide (Compound 19);
*N*-((3,5-dimethoxybenzyl)oxy)-6-(4-ethoxyphenyl)pyrazine-2-carboxamide (Compound 20);
*N*-((3,5-dimethylbenzyl)oxy)-6-(4-ethoxyphenyl)pyrazine-2-carboxamide (Compound 21);
6-(4-(azetidin-3-yloxy)phenyl)-*N*-((2-fluoro-5-methoxybenzyl)oxy)pyrazine-2-carboxamide (Compound 22);
6-(4-(azetidine-3-ylmethoxy)phenyl)-*N*-((2-fluoro-5-methoxybenzyl)oxy)pyrazine-2-carboxamide (Compound 23);
6-(4-(2-(azetidine-3-yl)ethoxy)phenyl)-*N*-((2-fluoro-5-methoxybenzyl)oxy)pyrazine-2-carboxamide (Compound 24);
*N*-((2-fluoro-5-methoxybenzyl)oxy)-6-(4-((1-methylazetidine-3-yl)oxy)phenyl)pyrazine-2-carboxamide (Compound 25);
*N*-((2-fluoro-5-methoxybenzyl)oxy)-6-(4-((1-methylazetidine-3-yl)methoxy)phenyl)pyrazine-2-carboxamide (Compound 26);
*N*-((2-fluoro-5-methoxybenzyl)oxy)-6-(4-(2-(1-methylazetidine-3-yl)ethoxy)phenyl)pyrazine-2-carboxamide (Compound 27);
6-(4-(difluoromethoxy)phenyl)-*N*-((5-methoxypyridine-3-yl)methoxy)pyrazine-2-carboxamide (Compound 28);
*N*-((2-fluoro-5-methoxybenzyl)oxy)-6-(4-(methylsulfinyl)phenyl)pyrazine-2-carboxamide (Compound 29);
*N*-((2-fluoro-5-methoxybenzyl)oxy)-6-(6-methoxypyridine-3-yl)pyrazine-2-carboxamide (Compound 30);
*N*-((2-fluoro-5-methoxybenzyl)oxy)-6-(6-isopropoxypyridine-3-yl)pyrazine-2-carboxamide (Compound 31);
6-(6-ethoxypyridine-3-yl)-*N*-((2-fluorobenzyl)oxy)pyrazine-2-carboxamide (Compound 32);
6-(6-ethoxypyridine-3-yl)-*N*-((3-methoxybenzyl)oxy)pyrazine-2-carboxamide (Compound 33);
6-(6-ethoxypyridine-3-yl)-*N*-((2-hydroxybenzyl)oxy)pyrazine-2-carboxamide (Compound 34);
6-(6-ethoxypyridine-3-yl)-*N*-((3-hydroxybenzyl)oxy)pyrazine-2-carboxamide (Compound 35);
6-(6-ethoxypyridine-3-yl)-*N*-((5-methoxypyridine-3-yl)methoxy)pyrazine-2-carboxamide (Compound 36);
6-(6-ethoxypyridine-3-yl)-*N*-((2-fluoro-5-methoxybenzyl)oxy)pyrazine-2-carboxamide (Compound 37);
6-(6-ethoxypyridine-3-yl)-*N*-((2-fluoro-5-methoxypyridine-3-yl)methoxy)pyrazine-2-carboxamide (Compound 38);
6-(6-ethoxypyridine-3-yl)-*N*-((5-fluoro-2-methoxypyridine-4-yl)methoxy)pyrazine-2-carboxamide (Compound 39);
*N*-((5-(dimethylamino)-2-fluorobenzyl)oxy)-6-(6-ethoxypyridine-3-yl)pyrazine-2-carboxamide (Compound 40);
*N*-((5-(dimethylamino)-2-fluoropyridine-3-yl)methoxy)-6-(6-ethoxypyridine-3-yl)pyrazine-2-carboxamide (Compound 41);
*N*-((5-(dimethylamino)-2-fluoropyridine-3-yl)methoxy)-6-(6-ethoxypyridine-3-yl)pyrazine-2-carboxamide (Compound 42);
6-(6-ethoxypyridine-3-yl)-*N*-((2-fluoro-5-hydroxybenzyl)oxy)pyrazine-2-carboxamide (Compound 43);
6-(6-ethoxypyridine-3-yl)-*N*-((2-hydroxy-5-methoxybenzyl)oxy)pyrazine-2-carboxamide (Compound 44);
*N*-((5-(1-aminoethyl)-2-fluorobenzyl)oxy)-6-(6-ethoxypyridine-3-yl)pyrazine-2-carboxamide (Compound 45);
6-(6-ethoxypyridine-3-yl)-*N*-((5-methoxy-2-methylpyridine-3-yl)methoxy)pyrazine-2-carboxamide (Compound 46);
6-(6-ethoxypyridine-3-yl)-*N*-((5-methoxy-2-methylbenzyl)oxy)pyrazine-2-carboxamide (Compound 47);
6-(6-(ethoxy-d5)pyridine-3-yl)-*N*-((2-fluoro-5-methoxybenzyl)oxy)pyrazine-2-carboxamide (Compound 48);
6-(6-(ethoxy-2,2,2-d3)pyridine-3-yl)-*N*-((2-fluoro-5-methoxybenzyl)oxy)pyrazine-2-carboxamide (Compound 49);

6-(6-(ethoxy-1,1-d2)pyridine-3-yl)-*N*-((2-fluoro-5-methoxybenzyl)oxy)pyrazine-2-carboxamide (Compound 50);
6-(6-(ethoxy-1,1-d2)pyridine-3-yl)-*N*-((2-fluoro-5-methoxybenzyl)oxy)pyrazine-2-carboxamide (Compound 51);
6-(6-(ethoxy-2,2,2-d3)pyridine-3-yl)-*N*-((2-fluoro-5-(methoxy-d3)benzyl)oxy)pyrazine-2-carboxamide (Compound 52);
6-(6-(ethoxy-d5)pyridine-3-yl)-*N*-((2-fluoro-5-(methoxy-d3)benzyl)oxy)pyrazine-2-carboxamide (Compound 53);
6-(6-(ethoxy-d5)pyridine-3-yl)-*N*-((2-fluoro-5-(methoxy-d3)phenyl)methoxy-d2)pyrazine-2-carboxamide (Compound 54);
6-(6-ethoxypyridine-3-yl)-*N*-((2-fluoro-5-(methoxy-d3)benzyl)oxy)pyrazine-2-carboxamide (Compound 55);
6-(6-ethoxypyridine-3-yl)-*N*-((2-fluoro-5-(methoxy-d3)benzyl)oxy)pyrazine-2-carboxamide (Compound 56);
6-(6-ethoxypyridine-3-yl)-*N*-((5-methyloxazole-4-yl)methoxy)pyrazine-2-carboxamide (Compound 57);
6-(6-ethoxypyridine-3-yl)-*N*-((5-methyloxazole-4-yl)methoxy)pyrazine-2-carboxamide (Compound 58);
6-(6-ethoxypyridine-3-yl)-*N*-((4-methyl-1*H*-imidazole-5-yl)methoxy)pyrazine-2-carboxamide (Compound 59);
6-(6-ethoxypyridine-3-yl)-*N*-((1-methyl-1*H*-pyrrole-2-yl)methoxy)pyrazine-2-carboxamide (Compound 60);
6-(6-ethoxypyridine-3-yl)-*N*-((1-ethyl-1*H*-imidazole-2-yl)methoxy)pyrazine-2-carboxamide (Compound 61);
6-(6-ethoxypyridine-3-yl)-*N*-((1-ethyl-1*H*-pyrazole-5-yl)methoxy)pyrazine-2-carboxamide (Compound 62);
6-(6-ethoxypyridine-3-yl)-*N*-((1-ethyl-1*H*-pyrazole-5-yl)methoxy)pyrazine-2-carboxamide (Compound 63);
6-(6-ethoxypyridine-3-yl)-*N*-((1-ethyl-1*H*-pyrazole-5-yl)methoxy)pyrazine-2-carboxamide (Compound 64);
6-(6-ethoxypyridine-3-yl)-*N*-((4-methyloxazole-5-yl)methoxy)pyrazine-2-carboxamide (Compound 65);
6-(6-ethoxypyridine-3-yl)-*N*-((3-methoxyfuran-2-yl)methoxy)pyrazine-2-carboxamide (Compound 66);
6-(6-ethoxypyridine-3-yl)-*N*-((3-methyl-1*H*-pyrrole-2-yl)methoxy)pyrazine-2-carboxamide (Compound 67);
6-(6-ethoxypyridine-3-yl)-*N*-((3-methyl-1*H*-pyrrole-2-yl)methoxy)pyrazine-2-carboxamide (Compound 68);
6-(6-ethoxypyridine-3-yl)-*N*-((3-methyl-1*H*-pyrazole-4-yl)methoxy)pyrazine-2-carboxamide (Compound 69);
*N*-((2-fluorobenzyl)oxy)-6-(6-(trifluoromethoxy)pyridine-3-yl)pyrazine-2-carboxamide (Compound 70);
*N*-((3-methoxybenzyl)oxy)-6-(6-(trifluoromethoxy)pyridine-3-yl)pyrazine-2-carboxamide (Compound 71);
*N*-(pyridine-2-ylmethoxy)-6-(6-(trifluoromethoxy)pyridine-3-yl)pyrazine-2-carboxamide (Compound 72);
*N*-(pyridine-3-ylmethoxy)-6-(6-(trifluoromethoxy)pyridine-3-yl)pyrazine-2-carboxamide (Compound 73);
*N*-(pyridine-4-ylmethoxy)-6-(6-(trifluoromethoxy)pyridine-3-yl)pyrazine-2-carboxamide (Compound 74);
*N*-((2-fluoro-5-methoxybenzyl)oxy)-6-(6-(trifluoromethoxy)pyridine-3-yl)pyrazine-2-carboxamide (Compound 75);
6-(6-(difluoromethoxy)pyridine-3-yl)-*N*-((2-fluoro-5-methoxybenzyl)oxy)pyrazine-2-carboxamide (Compound 76);
6-(6-(difluoromethoxy)pyridine-3-yl)-*N*-((2-fluoro-5-methoxybenzyl)oxy)pyrazine-2-carboxamide (Compound 77);
6-(6-(difluoromethoxy)pyridine-3-yl)-*N*-((5-fluoro-2-methoxypyridine-4-yl)methoxy)pyrazine-2-carboxamide (Compound 78);
6-(6-(difluoromethoxy)pyridine-3-yl)-*N*-((5-fluoro-2-methoxypyridine-4-yl)methoxy)pyrazine-2-carboxamide (Compound 79);
6-(6-(difluoromethoxy)pyridine-3-yl)-*N*-((5-(dimethylamino)-2-fluorobenzyl)oxy)pyrazine-2-carboxamide (Compound 80);
6-(6-(difluoromethoxy)pyridine-3-yl)-*N*-((3-(1-hydroxyethyl)benzyl)oxy)pyrazine-2-carboxamide (Compound 81);
6-(6-(difluoromethoxy)pyridine-3-yl)-*N*-((2-fluoro-5-(methoxy-d3)benzyl)oxy)pyrazine-2-carboxamide (Compound 82);
*N*-((2-fluoro-5-methoxybenzyl)oxy)-6-(6-(methylsulfinyl)pyridine-3-yl)pyrazine-2-carboxamide (Compound 83);
6-(4-ethoxy-2-hydroxyphenyl)-*N*-((2-fluoro-5-methoxybenzyl)oxy)pyrazine-2-carboxamide (Compound 84);
6-(4-ethoxy-3-hydroxyphenyl)-*N*-((2-fluoro-5-methoxybenzyl)oxy)pyrazine-2-carboxamide (Compound 85);
6-(6-ethoxy-4-hydroxypyridine-3-yl)-*N*-((2-fluoro-5-methoxybenzyl)oxy)pyrazine-2-carboxamide (Compound 86);
6-(6-ethoxy-2-oxo-1,2-dihydropyridine-3-yl)-*N*-((2-fluoro-5-methoxybenzyl)oxy)pyrazine-2-carboxamide (Compound 87);
*N*-((3,5-dimethoxybenzyl)oxy)-6-(4-ethoxyphenyl)-5-hydroxypyrazine-2-carboxamide (Compound 88);
6-(4-ethoxyphenyl)-*N*-((2-fluoro-5-methoxybenzyl)oxy)-5-hydroxypyrazine-2-carboxamide (Compound 89);
*N*-((3,5-dimethoxybenzyl)oxy)-6-(4-ethoxyphenyl)-3-hydroxypyrazine-2-carboxamide (Compound 90);
6-(4-ethoxyphenyl)-*N*-((2-fluoro-5-methoxybenzyl)oxy)-3-hydroxypyrazine-2-carboxamide (Compound 91);
6-(6-ethoxypyridine-3-yl)-*N*-((2-fluoro-5-methoxybenzyl)oxy)-5-hydroxypyrazine-2-carboxamide (Compound 92);
6-(6-ethoxypyridine-3-yl)-*N*-((2-fluoro-5-methoxybenzyl)oxy)-3-hydroxypyrazine-2-carboxamide (Compound 93);
5-amino-6-(6-ethoxypyridine-3-yl)-*N*-((2-fluoro-5-methoxybenzyl)oxy)pyrazine-2-carboxamide (Compound 94);
3-amino-6-(6-ethoxypyridine-3-yl)-*N*-((2-fluoro-5-methoxybenzyl)oxy)pyrazine-2-carboxamide (Compound 95);
6-(6-ethoxypyridine-3-yl)-5-fluoro-*N*-((2-fluoro-5-methoxybenzyl)oxy)pyrazine-2-carboxamide (Compound 96);
6-(6-ethoxypyridine-3-yl)-3-fluoro-*N*-((2-fluoro-5-methoxybenzyl)oxy)pyrazine-2-carboxamide (Compound 97);
6'-ethoxy-*N*-((2-fluoro-5-methoxybenzyl)oxy)-[2,3'-bipyridine]-6-carboxamide (Compound 98);
6'-ethoxy-*N*-((2-fluoro-5-methoxypyridin-3-yl)methoxy)-[2,3'-bipyridine]-6-carboxamide (Compound 99);
6'-ethoxy-*N*-((2-fluoro-5-methoxybenzyl)oxy)-[3,3'-bipyridine]-5-carboxamide (Compound 100);

2-(6-ethoxypyridine-3-yl)-*N*-((2-fluoro-5-methoxybenzyl)oxy)pyrimidine-4-carboxamide (Compound 101);
2-(6-ethoxypyridine-3-yl)-*N*-((2-fluoro-5-methoxypyridin-3-yl)methoxy)pyrimidine-4-carboxamide (Compound 102);
4-(6-ethoxypyridine-3-yl)-*N*-((2-fluoro-5-methoxybenzyl)oxy)pyrimidine-2-carboxamide (Compound 103);
4-(6-ethoxypyridine-3-yl)-*N*-((2-fluoro-5-methoxybenzyl)oxy)pyrimidine-2-carboxamide (Compound 104);
4-(6-ethoxypyridine-3-yl)-*N*-((2-fluoro-5-methoxybenzyl)oxy)pyrimidine-2-carboxamide (Compound 105);
6'-(difluoromethoxy)-*N*-((2-fluoro-5-methoxypyridine-3-yl)methoxy)-[2,3'-bipyridine]-6-carboxamide (Compound 106);
6'-(difluoromethoxy)-*N*-((2-fluoro-5-methoxypyridine-3-yl)methoxy)-[2,3'-bipyridine]-6-carboxamide (Compound 107);
6'-(difluoromethoxy)-*N*-((2-fluoro-5-methoxypyridine-3-yl)methoxy)-[2,3'-bipyridine]-6-carboxamide (Compound 108);
6'-(difluoromethoxy)-*N*-((2-fluoro-5-methoxybenzyl)oxy)-[3,3'-bipyridine]-5-carboxamide (Compound 109);
2-(6-(difluoromethoxy)pyridine-3-yl)-*N*-((2-fluoro-5-methoxypyridine-3-yl)methoxy)pyrimidine-4-carboxamide (Compound 110);
2-(6-(difluoromethoxy)pyridine-3-yl)-*N*-((2-fluoro-5-methoxypyridine-3-yl)methoxy)pyrimidine-4-carboxamide (Compound 111);
2-(6-(difluoromethoxy)pyridine-3-yl)-*N*-((2-fluoro-5-methoxypyridine-3-yl)methoxy)pyrimidine-4-carboxamide (Compound 112);
3-amino-6-(6-(difluoromethoxy)pyridine-3-yl)-*N*-((2-fluoro-5-methoxypyridine-3-yl)methoxy)pyrazine-2-carboxamide (Compound 113);
3-amino-6-(6-(difluoromethoxy)pyridine-3-yl)-*N*-((2-fluoro-5-methoxypyridine-3-yl)methoxy)pyrazine-2-carboxamide (Compound 114);
5-amino-6-(6-(difluoromethoxy)pyridine-3-yl)-*N*-((2-fluoro-5-methoxybenzyl)oxy)pyrazine-2-carboxamide (Compound 115);
6-(6-(difluoromethoxy)pyridine-3-yl)-3-fluoro-N-((2-fluoro-5-methoxybenzyl)oxy)pyrazine-2-carboxamide (Compound 116);
6-(6-(difluoromethoxy)pyridine-3-yl)-5-fluoro-N-((2-fluoro-5-methoxybenzyl)oxy)pyrazine-2-carboxamide (Compound 117);
3-(6-(difluoromethoxy)pyridine-3-yl)-N-((2-fluoro-5-methoxybenzyl)oxy)-1,2,4-triazine-5-carboxamide (Compound 118);
5-(6-(difluoromethoxy)pyridine-3-yl)-N-((2-fluoro-5-methoxybenzyl)oxy)-1,2,4-triazine-3-carboxamide (Compound 119);
N-((2-(3,5-dimethoxyphenyl)propan-2-yl)oxy)-6-(4-ethoxyphenyl)pyrazine-2-carboxamide (Compound 120);
*N*-(1-(3,5-dimethoxyphenyl)cyclopropoxy)-6-(4-ethoxyphenyl)pyrazine-2-carboxamide (Compound 121);
(R)-N-(1-(3,5-dimethoxyphenyl)ethoxy)-6-(4-ethoxyphenyl)pyrazine-2-carboxamide (Compound 122);
(S)-N-(1-(3,5-dimethoxyphenyl)ethoxy)-6-(4-ethoxyphenyl)pyrazine-2-carboxamide (Compound 123);
(S)-N-(1-(3,5-dimethoxyphenyl)ethoxy)-6-(4-ethoxyphenyl)pyrazine-2-carboxamide (Compound 124);
(R)-6-(4-ethoxyphenyl)-N-(1-(2-fluoro-5-methoxyphenyl)ethoxy)pyrazine-2-carboxamide (Compound 125);
(S)-6-(4-ethoxyphenyl)-N-(1-(2-fluoro-5-methoxyphenyl)ethoxy)pyrazine-2-carboxamide (Compound 126);
6-(4-ethoxyphenyl)-N-((2-fluoro-5-methoxyphenyl)methoxy-d2)pyrazine-2-carboxamide (Compound 127);
(R)-6-(4-ethoxyphenyl)-N-(1-(3-methoxyphenyl)ethoxy)pyrazine-2-carboxamide (Compound 128);
(S)-6-(4-ethoxyphenyl)-N-(1-(3-methoxyphenyl)ethoxy)pyrazine-2-carboxamide (Compound 129);
6-(4-ethoxyphenyl)-*N*-((3-methoxyphenyl)methoxy-d2)pyrazine-2-carboxamide (Compound 130);
(*R*)-6-(4-ethoxyphenyl)-*N*-(1-(2-fluorophenyl)ethoxy)pyrazine-2-carboxamide (Compound 131);
(*S*)-6-(4-ethoxyphenyl)-*N*-(1-(2-fluorophenyl)ethoxy)pyrazine-2-carboxamide (Compound 132);
6-(4-ethoxyphenyl)-*N*-((2-fluorophenyl)methoxy-d2)pyrazine-2-carboxamide (Compound 133);
6-(6-(difluoromethoxy)pyridine-3-yl)-N-((2-fluoro-5-(methoxy-d3)phenyl)methoxy-d2)pyrazine-2-carboxamide (compound 134);
(R)-6-(6-ethoxypyridine-3-yl)-N-(1-(2-fluoro-5-methoxyphenyl)ethoxy)pyrazine-2-carboxamide (Compound 135);
(S)-6-(6-ethoxypyridine-3-yl)-N-(1-(2-fluoro-5-methoxyphenyl)ethoxy)pyrazine-2-carboxamide (Compound 136);
6-(6-ethoxypyridine-3-yl)-N-((2-(2-fluoro-5-methoxyphenyl)propan-2-yl)oxy)pyrazine-2-carboxamide (Compound 137);
6-(6-ethoxypyridine-3-yl)-N-(1-(2-fluoro-5-methoxyphenyl)cyclopropoxy)pyrazine-2-carboxamide (Compound 138);
6-(6-ethoxypyridine-3-yl)-N-(1-(2-fluoro-5-methoxyphenyl)cyclopropoxy)pyrazine-2-carboxamide (Compound 139);
(R)-6-(6-ethoxypyridine-3-yl)-N-(1-(3-methoxyphenyl)ethoxy)pyrazine-2-carboxamide (Compound 140);
(S)-6-(6-ethoxypyridine-3-yl)-N-(1-(3-methoxyphenyl)ethoxy)pyrazine-2-carboxamide (Compound 141);

6-(6-ethoxypyridine-3-yl)-N-((3-methoxyphenyl)methoxy-d2)pyrazine-2-carboxamide (Compound 142);
(R)-6-(6-ethoxypyridine-3-yl)-N-(1-(2-fluorophenyl)ethoxy)pyrazine-2-carboxamide (Compound 143);
(S)-6-(6-ethoxypyridine-3-yl)-N-(1-(2-fluorophenyl)ethoxy)pyrazine-2-carboxamide (Compound 144);
6-(6-ethoxypyridine-3-yl)-N-((2-fluorophenyl)methoxy-d2)pyrazine-2-carboxamide (Compound 145);
(R)-6-(6-ethoxypyridine-3-yl)-N-(1-(2-fluoro-5-methoxyphenyl)ethoxy-2,2,2-d3)pyrazine-2-carboxamide (Compound 146);
(S)-6-(6-ethoxypyridine-3-yl)-N-(1-(2-fluoro-5-methoxyphenyl)ethoxy-2,2,2-d3)pyrazine-2-carboxamide (Compound 147);
6-(6-ethoxypyridine-3-yl)-N-((2-fluoro-5-methoxybenzyl)oxy)-N-methylpyrazine-2-carboxamide (Compound 148);
6-(6-ethoxypyridine-3-yl)-N-((2-fluoro-5-methoxypyridine-3-yl)methoxy)-N-methylpyrazine-2-carboxamide (Compound 149);
6-(6-(difluoromethoxy)pyridine-3-yl)-N-((2-fluoro-5-methoxybenzyl)oxy)-N-methylpyrazine-2-carboxamide (Compound 150);
6-(6-(difluoromethoxy)pyridine-3-yl)-N-((2-fluoro-5-methoxypyridine-3-yl)methoxy)-N-methylpyrazine-2-carboxamide (Compound 151);
6-(6-ethoxypyridine-3-yl)-N-((3-methoxyphenyl)methoxy-d2)pyrazine-2-carboxamide (Compound 152);
(R*)-6-(6-ethoxypyridine-3-yl)-N-(1-(2-fluorophenyl)ethoxy)pyrazine-2-carboxamide (Compound 153);
(S*)-6-(6-ethoxypyridine-3-yl)-N-(1-(2-fluorophenyl)ethoxy)pyrazine-2-carboxamide (Compound 154);
6-(6-ethoxypyridine-3-yl)-N-((2-fluorophenyl)methoxy-d2)pyrazine-2-carboxamide (Compound 155);
6-(6-ethoxypyridine-3-yl)-N-((2-fluoro-5-methoxybenzyl)oxy)-N-methylpyrazine-2-carboxamide (Compound 156);
6-(6-ethoxypyridine-3-yl)-N-((2-fluoro-5-methoxypyridine-3-yl)methoxy)-N-methylpyrazine-2-carboxamide (Compound 157);
6-(6-(difluoromethoxy)pyridine-3-yl)-N-((2-fluoro-5-methoxybenzyl)oxy)-N-methylpyrazine-2-carboxamide (Compound 158); and
6-(6-(difluoromethoxy)pyridine-3-yl)-N-((2-fluoro-5-methoxypyridine-3-yl)methoxy)-N-methylpyrazine-2-carboxamide (Compound 159).

In an implementation of the first embodiment, the Formula (I) compound(s) are voltage-gated sodium channel blockers Nav 1.7 and/or Nav 1.8. In a preferred embodiment, the Formula (I) compound(s)are dual voltage-gated sodium channel blockers Nav 1.7 and Nav 1.8.

In an implementation of the first embodiment, the compound(s) of Formula (I) may have a basic nature and, consequently, pharmaceutically acceptable salts may be obtained by the addition of organic or inorganic acids. Nonlimiting examples of organic acids that can be used are fumaric, maleic, benzoic, lactic acids, among others. Among the inorganic acids, we can mention hydrochloric acid, phosphoric acid, hydrobromic acid, sulfuric acid, nitric acid, among others.

In an implementation of the first embodiment, the compound(s) of Formula (I) can be obtained in the form of crystals, which can be optionally presented as pharmaceutically acceptable solvates, in which the solvent is incorporated in stoichiometric proportions or not into the crystal lattice. In further embodiments, the crystallization solvent is water, resulting in pharmaceutically acceptable hydrates.

Finally, in an implementation of the first embodiment the Formula (I) compound(s) may present more than one isomer, including without limitation, spatial isomerism, such as geometric and optical isomerism.

**DEFINITIONS:**

In order to clarify or elucidate the terms used in this invention, the following definitions are presented, whereby the scope is not limited thereto.

The term "halogen" refers to the elements of the 7th family of the periodic table, which are: fluorine (F), chlorine (Cl), bromine (Br), iodine (I), astatine (At) and tennessine (Ts).

The term "linear or branched $C_1$-$C_6$ alkyl" refers to saturated hydrocarbon groups of linear or branched chain, such as methyl, ethyl, propyl, isopropyl, butyl, isobutyl, sec-butyl, tert-butyl, but not limited to the same.

The term "linear or branched $C_1$-$C_6$ alkoxy" refers to alkyl groups attached to an oxygen radical, such as methoxy, ethoxy, propoxy, isopropoxy, butoxy, sec-butoxy, *tert*-butoxy, but not limited to the same.

The term "linear or branched haloalkoxy $C_1$-$C_6$" refers to alkyl groups attached to a root oxygen and at least one halogen, such as, but not limited to, chloromethoxy, dichloromethoxy, trichloromethoxy, fluoromethoxy, difluoromethoxy, and trifluoromethoxy.

The term "linear or branched $C_1$-$C_6$" refers to saturated, linear or branched alkyl groups replaced with at least one hydroxyl, such as hydroxymethyl, 1-hydroxyethyl, 2-hydroxyethyl, 1-hydroxypropyl, 2-hydroxypropyl, 2-hydroxypropyl, isopropoxy, but not limited to the same.

The term "COH" refers to a carbon atom bonded to a hydroxyl by means of a single bond.

The term "azetidinylalkoxy $C_1$-$C_3$" refers to the azetidinyl groups attached to an "alkoxy $C_1$-$C_3$" group, e.g., azetidinylmethoxy, azetidinylethoxy, azetidinylpropoxy, and azetidinylbutoxy.

The term "$C_1$-$C_6$ alkylsulfinyl" refers to the sulfinyl groups ($R_2S$=$O$) attached to the $C_1$-$C_6$ alkyl group, e.g., methylsulfinyl, ethylsulfinyl, propylsulfinyl, isopropylsulfinyl, butylsulfinyl, sec-butylsulfinyl, and *tert*-butylsulfinyl.

The term "alkylazetidinylalkoxy $C_1$-$C_6$" refers to azetidinylalkoxy groups linked to "alkyl $C_1$-$C_6$" groups, such as, for example, 1-methylazetidine-3-ylmetoxy, 2-methylazetidine-3-ylethoxy, 1-methylazetidine-3-ylpropoxy, but not being limited thereto.

The term "$C_1$-$C_2$ containing one or more isotopically enriched atoms such as hydrogen isotope $^2$H" refers to exemplified species such as , methoxy-$d_1$, methoxy-$d_2$, methoxy-$d_3$, ethoxy-1-$d_1$, ethoxy-2-$d_1$, ethoxy-1, 1-$d_2$, ethoxy-1,2-$d_2$, ethoxy-2,2-$d_2$, ethoxy-1,1,1-$d_3$, ethoxy-1,1,2-$d_3$, ethoxy-1,2,2-$d_3$, ethoxy-2,2,2-$d_3$, ethoxy-1,1,2,2-$d_4$, ethoxy-1,2,2,2-$d_4$, ethoxy-ds, but not limited to these.

The term "saturated or unsaturated $C_3$-$C_7$ cycloalkyl" refers to alkyl cycle groups such as, but not limited to, cyclopropyl, cyclobutyl, cyclopentyl, cyclopentenyl, cyclohexyl, cyclohexenyl, cycloheptyl, cycloheptenyl, but not being limited thereto.

The term "cycloalkoxy $C_3$-$C_7$" refers to, but is not limited to, the alkyl cycle groups attached to an oxygen radical, such as cyclopropoxy, cyclobutoxy, cyclopentoxy, and cyclohexoxy.

The term "linear or branched $C_1$-$C_6$ aminoalkyl" refers to amines that are attached to one or two linear or branched alkyl groups by means of single bonds such as, but not limited to, methylamine, ethylamine, propylamine, dimethylamine, diethylamine, dipropylamine.

In a second embodiment, the present invention presents a composition comprising a therapeutically effective amount of compound(s) of Formula (I) of the present invention or a pharmaceutically acceptable salt, hydrate, solvate, and isomer thereof; and one or more pharmaceutically acceptable excipients.

Pharmaceutically acceptable excipients are any substance, other than the active pharmaceutical ingredient, that has been evaluated for its safety and that is intentionally added to the dosage form. Such excipients are selected according to the pharmaceutical dosage form of interest, the route of administration thereof, physicochemical compatibility with the active ingredient, and the effect on efficacy.

In addition, these excipients are widely known in the state of the art and are classified according to their function, including without limitation diluents, binders, disintegrants or disaggregators, lubricants, suspending agents, thickeners, solvents, surfactants, sliders, anti-caking agents or flow agents, glazing agents, plasticizers, sweeteners, isotonicity agents, dyes and pigments, preservatives, antioxidants, modifying agents or pH control, complexing agents, chelating agents, flavorings, viscosity modifying agents, opacifiers, permeation promoters, among others.

In an implementation of the second embodiment, the pharmaceutical compositions of the present invention can be administered by several routes including oral, sublingual, nasal, parenteral, injectable, submuscular, topical, transdermal, ocular, rectal, but not limited thereto.

In a third embodiment, the present invention presents the use of compound(s) of Formula (I) or a salt, hydrate, solvate and pharmaceutically acceptable isomer thereof to prepare a drug to treat pathologies related to neuropathic pain.

In an implementation of the third embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention presents a method of treatment, prevention, relief, suppression and/or control of pathologies related to neuropathic pain comprising the administration of an effective amount of Formula (I) compound(s) or a salt, hydrate, solvate and pharmaceutically acceptable isomer thereof.

In an implementation of the fourth embodiment, the method is for the treatment, prevention, relief, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In another implementation of the fourth embodiment, the administration of at least one Formula (I) compound is selected from the group comprising oral, sublingual, nasal, parenteral, injectable, submuscular, topical, transdermal, ocular and rectal routes.

In a fifth embodiment of the invention, processes for obtaining Formula (I) compounds are presented, comprising the following steps:

(a) Formation of the Formula III intermediate:

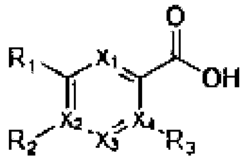

Formula III

from the hydrolysis reaction of a Formula IV intermediate:

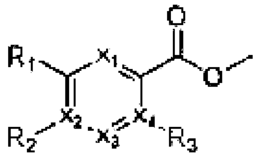

Formula IV

(b) obtaining Formula I compound;

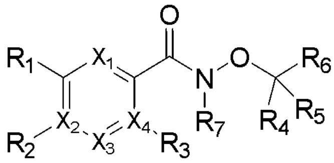

Formula (I)

from the reaction with the Formula II intermediates:

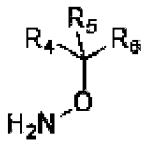

Formula II

and Formula III with or without the presence of a catalyst and a suitable solvent;
wherein,

- $X_1$, $X_2$, $X_3$, and $X_4$ are independently selected from the group consisting of carbon, CH, COH, or nitrogen;

wherein:

- when at least one of the substituents $X_2$, $X_4$ is nitrogen or CH, the corresponding R ($R_2$, $R_3$) is null.
- $R_1$ is:

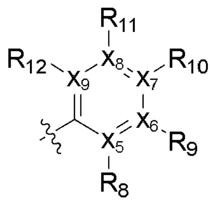

,

wherein:

- $X_5$, $X_6$, $X_7$, $X_8$, and $X_9$ are independently selected from the group consisting of carbon, CH, or nitrogen;

wherein:

- when at least one of the substituents $X_5$-$X_9$ is nitrogen or CH, the corresponding R ($R_8$-$R_{12}$) is null; and $R_8$, $R_9$, $R_{10}$, $R_{11}$, and $R_{12}$ are independently selected from the group consisting of hydrogen, halogen, $C_1$-$C_6$ alkyl linear or branched, alkoxy $C_1$-$C_6$ linear or branched, haloalkoxy $C_1$-$C_6$ linear or branched, hydroxy, azetidinyloxy, azetidinylalkoxy $C_1$-$C_3$, alkylazetidiniloxy $C_1$-$C_6$, alkylazetidinylalkoxy $C_1$-$C_6$, alkylsulfinyl $C_1$-$C_6$, $C_1$-$C_2$ alkoxy containing one or more isotopically enriched atoms such as, but not limited to, the hydrogen isotope $^2$H.
- $R_2$ and $R_3$ are independently selected from the group consisting of hydrogen, halogen, amine, hydroxy, $C_1$-$C_6$ alkoxy linear or branched, alkyl $C_1$-$C_6$ linear or branched;
- $R_4$ and $R_5$ are independently selected from the group consisting of hydrogen, deuterium, haloalkyl $C_1$-$C_6$ linear or branched, alkyl $C_1$-$C_6$ linear or branched;
- $R_6$ is selected from the group consisting of aryl, pyridinyl, or substituted pyridinyl, or $R_{13}$ or $R_{14}$, wherein:

  - $R_{13}$ is:

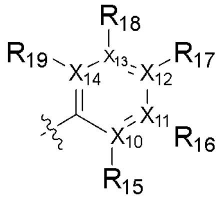,

wherein:

- $X_{10}$, $X_{11}$, $X_{12}$, $X_{13}$ and $X_{14}$ are independently selected from the group consisting of carbon, CH, or nitrogen; wherein:

  - when at least one of the substituents $X_{10-14}$ is nitrogen or CH, the corresponding R ($R_{15}$-$R_{19}$) is null; and
  - $R_{15}$ $R_{16}$, $R_{17}$, $R_{18}$ and $R_{19}$ are independently selected from the group consisting of hydrogen, halogen, hydroxy, alkyl linear or branched $C_1$-$C_6$, cycloalkyl $C_3$-$C_7$, $C_1$-$C_6$ alkoxy linear or branched, cycloalkoxy $C_3$-$C_7$, hydroxyalkyl $C_1$-$C_6$ linear or branched, aminoalkyl $C_1$-$C_6$ linear or branched, alkoxy $C_1$-$C_2$ containing one or more isotopically enriched atoms, such as hydrogen isotope $^2$H, but not limited to the same.
  - $R_{14}$ is selected from the group consisting of:

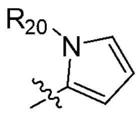
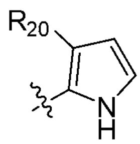
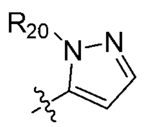
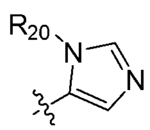
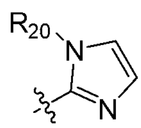
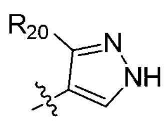
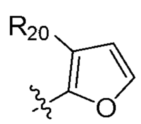
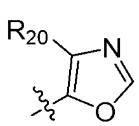
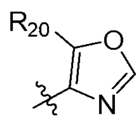
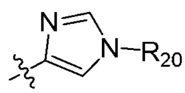
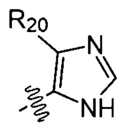;

wherein $R_{20}$ is independently selected from the group consisting of hydrogen, halogen, hydroxy, hydroxyalkyl $C_1$-$C_6$ linear or branched, alkyl $C_1$-$C_6$ linear or branched, alkoxy

$C_1$-$C_6$ linear or branched, cycloalkyl $C_3$-$C_7$, haloalkoxy $C_1$-$C_6$ linear or branched, nitrile, carboxyl, carboxyalkyl ester $C_1$-$C_6$, amine, N-alkylamine, N,N-dialkylamine, aminoalkyl $C_1$-$C_4$ linear or branched, sulfonamide, alkylsulfonylamide $C_1$-$C_6$, N,N-dialkylsulfonylamide $C_1$-$C_6$, sulfonyl, alkylsulfonyl $C_1$-$C_6$, sulfinyl, alkyl sulfinyl $C_1$-$C_6$, azetidiniloxy, azetidinylalkoxy $C_1$-$C_6$, linear or branched alkoxy $C_1$-$C_6$, hydroxyazetidinyl.

- $R_7$, is independently selected from the group consisting of hydrogen and alkyl $C_1$-$C_6$ linear or branched.

In an implementation of the fifth embodiment, the process additionally comprises a step (c) of formation of compound(s) of Formula (I) in which $R_7$ is linear or branched $C_1$-$C_6$ alkyl from the nucleophilic substitution reaction of a compound obtained in step (b), with linear or branched $C_1$-$C_6$ alkyl halides in the presence of inorganic base and polar aprotic solvents.

In an implementation of the fifth embodiment step (b) the coupling agent is selected from T3P or combination between TCFH and NMI or suitable coupling agents and said solvent selected is dichloromethane or classical aprotic solvents.

In a sixth embodiment, the present invention presents kits comprising a pharmaceutical composition of the present invention and an application device.

In an implementation of the sixth embodiment the kits of the present invention may comprise such compositions and application devices, which may include ampoules, syringes and others. Alternatively, the kits of this invention comprise more than one compound of Formula (I) arranged in one or more dosage forms, including without limitation, tablets, accompanied by administration instructions.

The compound(s) of Formula (I) of this invention have been prepared from the synthetic route described in General Scheme 1. However, those skilled in the art will readily notice that additional detailing and/or modifications to the arrangement of one or more steps can be accomplished without departing from the processes taught here. Such variations can be, without limitation, combinations of solvents and catalysts, including stereoselective ones, protective groups, among others.

In the following General Scheme 1, the formation of the Formula II, III and IV intermediates is described, in addition to the formation of the Formula (I) compound(s).

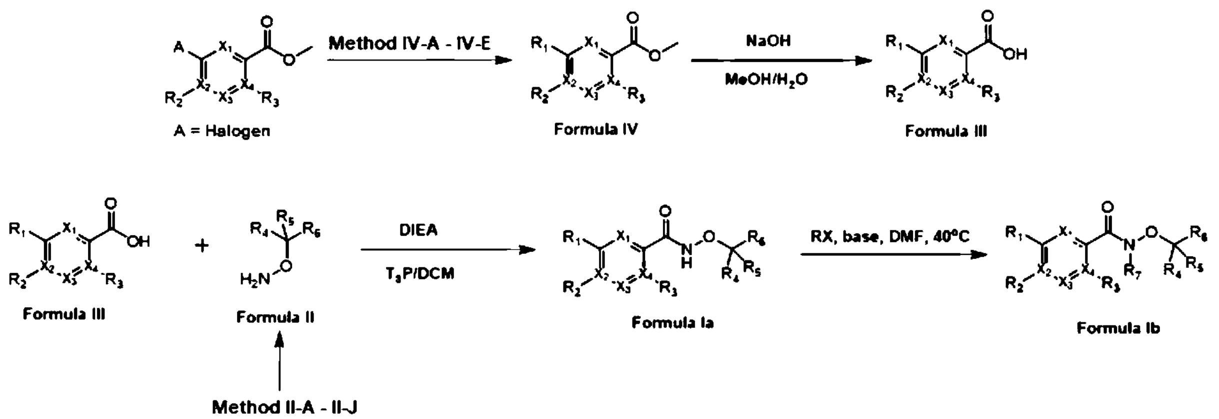


GENERAL SCHEME 1

As illustrated in General Scheme 1, Formula Ia compounds can be prepared from amide coupling reactions between Formula III intermediates with Formula II intermediates in the presence of coupling agents in suitable solvents such as DCM or other aprotic solvents such as DMF. In turn, Formula III intermediates can be prepared by hydrolysis reaction from the corresponding esters (Formula IV) obtained following various methods. Formula II intermediates can be prepared from the corresponding reagents following different methods, some of which can be obtained commercially. The intermediaries obtained by such methodologies are described below.

**FORMULA IV INTERMEDIATES:**

Following General Scheme 1, the methods from IV-A to IV-E for the formation of formula IV intermediates are presented, however, these methods are not exhaustive.

**METHOD IV-A**

**INTERMEDIATES IV-1 TO IV-42:**

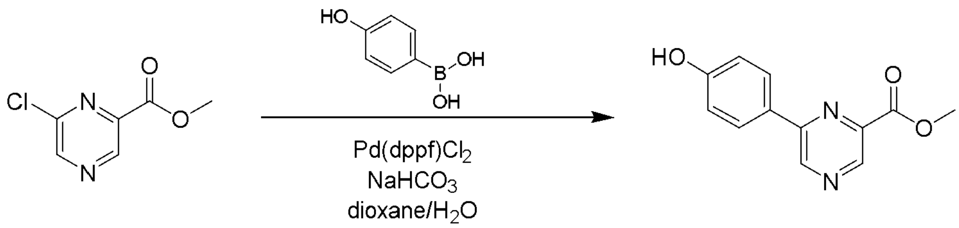


In a round-bottomed flask containing methyl 6-chloropyrazine-2-carboxylate (11.6 mmol) in dioxane (50.0 mL) and $H_2O$ (10.0 mL), (4-hydroxyphenyl)boronic acid (12.8 mmol), $NaHCO_3$ (17.4 mmol), and $Pd(dppf)Cl_2$ (579 μmol) was added under $N_2$ atmosphere. The reaction mixture was kept under agitation at 70 °C for 12 hours. After full consumption of the starting reagent (monitored by CCD), the mixture was concentrated under reduced pressure to remove the solvent. The residue obtained was diluted with $H_2O$ (100 mL) and extracted with AcOEt (100 mL x 3). The organic phase was concentrated under reduced pressure and purified by column chromatography (petroleum ether/AcOEt: 50/1 → 0/1). 1.5 g (56% yield) of the corresponding ester were obtained in the form of a white solid. This procedure is used to obtain the intermediates of Table 1.

**TABLE 1. INTERMEDIATES OBTAINED FROM THE CORRESPONDING REAGENTS FOLLOWING METHOD IV-A.**

| Intermediate | Structure | Intermediate | Structure |
|---|---|---|---|
| IV-1 | 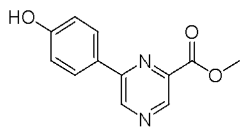 | IV-21 | 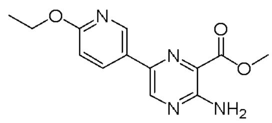 |
| IV-2 | 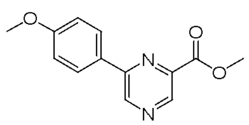 | IV-22 | 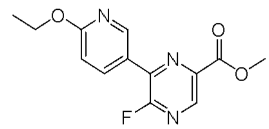 |
| IV-3 | 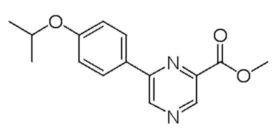 | IV-23 | 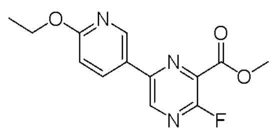 |
| IV-4 | 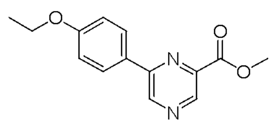 | IV-24 | 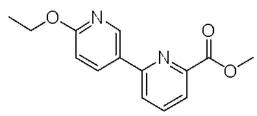 |
| IV-5 | 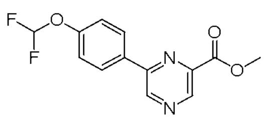 | IV-25 | 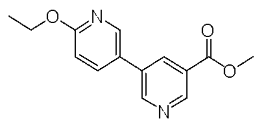 |
| IV-6 | 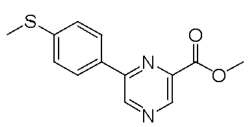 | IV-26 | 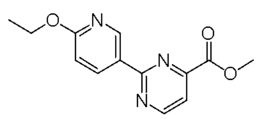 |
| IV-7 | 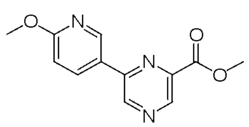 | IV-27 | 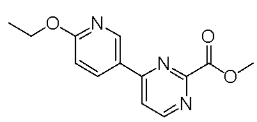 |

(continued)

| Intermediate | Structure | Intermediate | Structure |
|---|---|---|---|
| IV-8 | 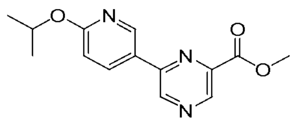 | IV-28 | 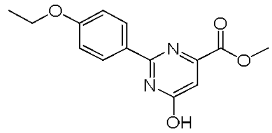 |
| IV-9 | 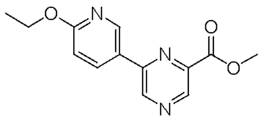 | IV-29 | 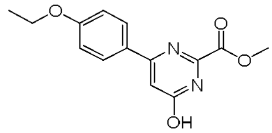 |
| IV-10 | 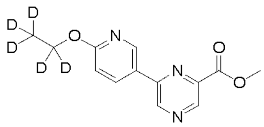 | IV-30 | 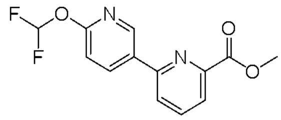 |
| IV-11 | 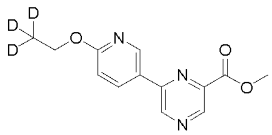 | IV-31 | 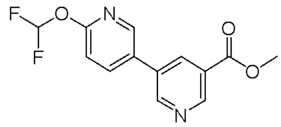 |
| IV-12 | 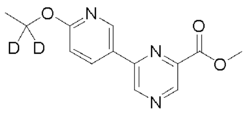 | IV-32 | 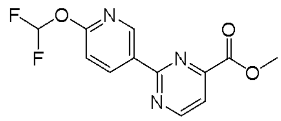 |
| IV-13 | 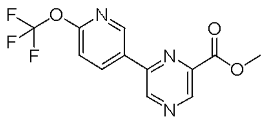 | IV-33 | 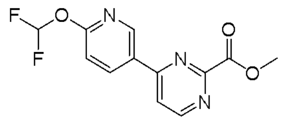 |
| IV-14 | 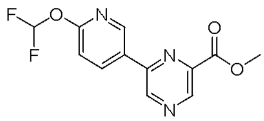 | IV-34 | 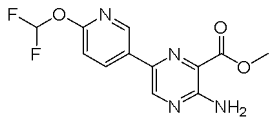 |
| IV-15 | 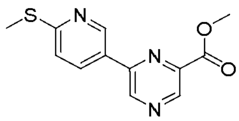 | IV-35 | 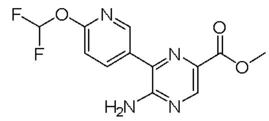 |
| IV-16 | 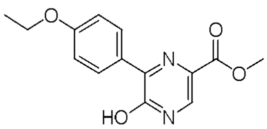 | IV-36 | 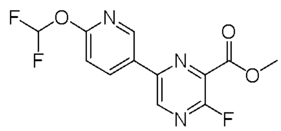 |
| IV-17 | 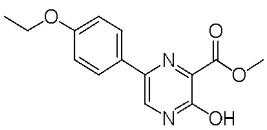 | IV-37 | 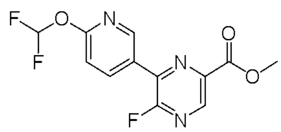 |
| IV-18 | 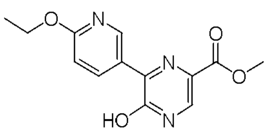 | IV-40 | 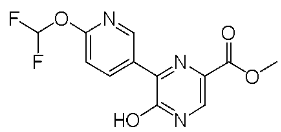 |

(continued)

| Intermediate | Structure | Intermediate | Structure |
|---|---|---|---|
| IV-19 | 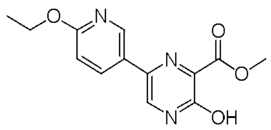 | IV-41 | 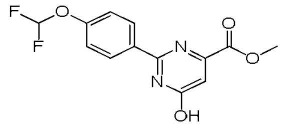 |
| IV-20 | 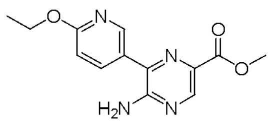 | IV-42 | 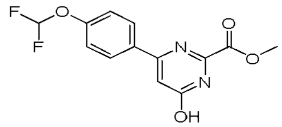 |

**METHOD IV-B**

**STEP IV-B-I:**

**PRECURSORS IV-43I TO IV-46I:**

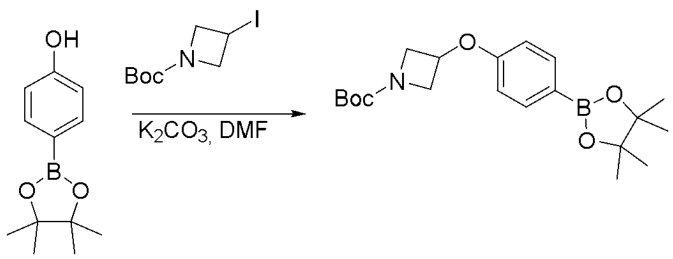


In a round-bottomed flask containing 4-(4,4,5,5-tetramethyl-1,3,2-dioxaborolan-2-yl)phenol (4.54 mmol) in DMF (15 mL) at 0 °C, $K_2CO_3$ (9.09 mmol) and tert-butyl 3-iodoazetidin-1-carboxylate (5.45 mmol) were added. The reaction mixture was kept under agitation at 80 °C for 12 hours. After total consumption of the starting reagent (monitored by CCD), the mixture was diluted with $H_2O$ (100 mL) and extracted with AcOEt (100 mL x 2). The organic phase was washed with saturated sodium chloride solution *(brine)* (5.0 mL x 1), dried under $Na_2SO_4$, filtered and concentrated under reduced pressure. The residue obtained was purified by column chromatography. 1.1 g (38.4% yield) of the product of interest were obtained in the form of a brown solid.

**TABLE 2. PRECURSORS OBTAINED FROM THE CORRESPONDING REACTANTS FOLLOWING *STEP IV-B-I.***

| Intermediate | Structure |
|---|---|
| IV-43i | 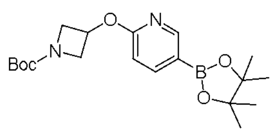 |
| IV-44i | 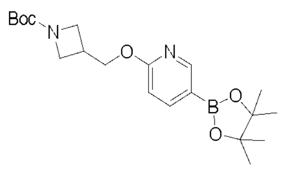 |
| IV-45i | 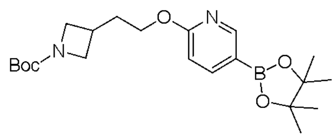 |

(continued)

| Intermediate | Structure |
|---|---|
| IV-46i | 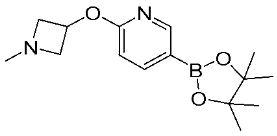 |

**STEP IV-B-II:**

**INTERMEDIATES IV-43 TO IV-46:**

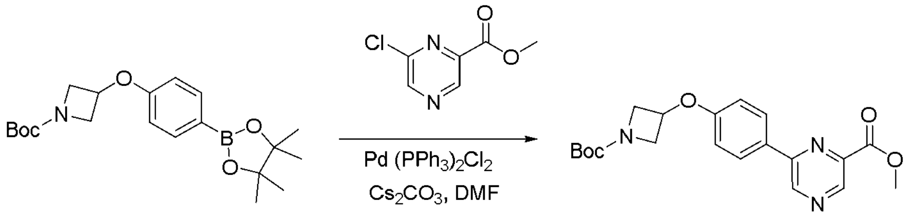


In a round-bottomed flask containing 3-(4-(4,4,5,5-tetramethyl-1,3,2-dioxaborolan-2-yl)phenoxy)tert-butyl azetidin-1-carboxylate (2.40 mmol) and methyl 6-chloropyrazine-2-carboxylate (3.12 mmol) in DMF (20 mL), Cs2CO3 (4.80 mmol) was added, and the mixture was de-gasified for 30 minutes. Next, Pd(dppf)$Cl_2$ (0.19 mmol) was added under inert atmosphere. The reaction mixture was kept under agitation at 55 °C for 12 hours. After total consumption of the starting reagent (monitored by CCD), the mixture was diluted with $H_2O$ (100.0 mL) and extracted with AcOEt (100.0 mL x 2). The organic phase was washed with saturated sodium chloride solution *(brine),* dried on anhydrous sodium sulfate, filtered, concentrated under reduced pressure and purified by column chromatography. 510 mg (34.2% yield) of the product of interest were obtained in the form of a white solid.

**TABLE 3. PRECURSORS OBTAINED FROM THE CORRESPONDING REAGENTS FOLLOWING STEP IV-B-II.**

| Intermediate | Structure |
|---|---|
| IV-43 | 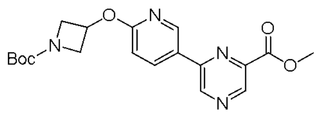 |
| IV-44 | 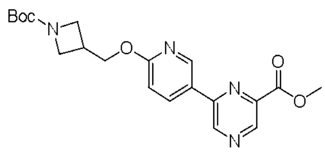 |
| IV-45 | 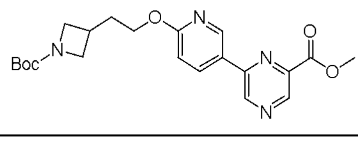 |
| IV-46 | 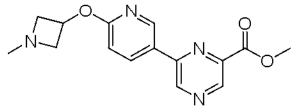 |

**METHOD IV-C**

**INTERMEDIATE 47:**

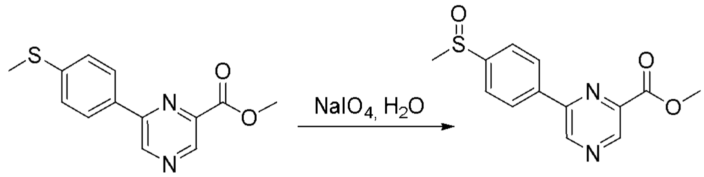


In a round-bottomed flask containing methyl 6-(4-(methylthio)phenyl)pyrazine-2-carboxylate (1.15 mmol) in $H_2O$ (10 mL), sodium periodate (1.09 mmol) was added at 25 °C. The reaction mixture was kept under agitation at 55 °C for 3 hours. After total consumption of the starting reagent (monitored by CCD), the mixture was diluted with $H_2O$ (20 mL) and extracted with AcOEt (50 mL x 3). The organic phase was washed with saturated sodium chloride solution *(brine),* dried under $Na_2SO_4$, filtered and concentrated under reduced pressure. The residue obtained was purified by column chromatography. 311 mg (80% yield) of the product of interest were obtained in the form of a white solid.

**TABLE 4. PRECURSORS OBTAINED FROM THE CORRESPONDING REAGENTS FOLLOWING METHOD C.**

| Intermediate | Structure |
|---|---|
| IV-47 | 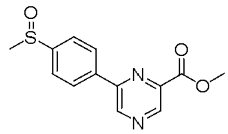 |
| IV-48 | 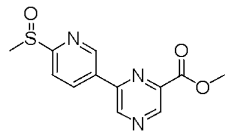 |

**METHOD IV-D**

**STEP IV-D-I:**

**PRECURSOR IV-49I:**

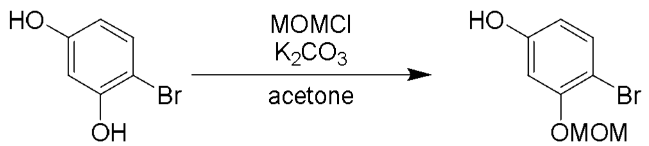


In a round-bottomed flask containing MOMCl (79.4 mmol) in acetone (30.0 mL), 4-bromobenzene-1,3-diol (79.4 mmol) and $K_2CO_3$ (119 mmol) in acetone (100 mL) at 20-30 °C under $N_2$ atmosphere was added. The reaction mixture remained at 60 °C for 12 hours under an atmosphere of $N_2$. After total consumption of the starting reagent (monitored by CCD), the mixture was cooled to 20-30 °C and filtered. The residue was washed with AcOEt (60.0 mL). The resulting filtrate was concentrated under reduced pressure and purified by column chromatography (petroleum ether/AcOEt: 50/1 $\rightarrow$ 5/1). 10 g (51.73% yield) of the product of interest were obtained in the form of a yellow solid.

**STEP IV-D-II:**

**PRECURSOR IV-49II:**

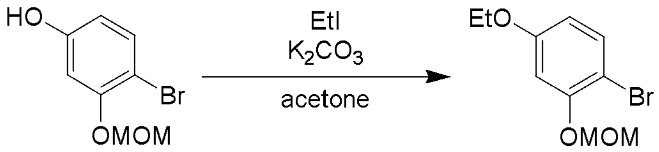


In a round-bottomed flask containing 4-bromo-3-(methoxymethoxy)phenol (42.9 mmol) and $K_2CO_3$ (64.39 mmol) in MeCN (70.0 mL), iodoethane (55.8 mmol) was added at 20-30 °C under an atmosphere of $N_2$. The reaction mixture was kept under agitation at 80 °C for 12 hours. After total consumption of the starting reagent (monitored by LC-MS and CCD), the mixture was filtered, and the residue was washed with AcOEt (20.0 mL x 2). The organic phase obtained was concentrated under reduced pressure and purified by column chromatography (petroleum ether/AcOEt: 50/1 $\rightarrow$ 5/1). 7.9 g (70.51% yield) of the product of interest were obtained in the form of a yellow solid.

**STEP II-D-III:**

**PRECURSOR IV-49III:**

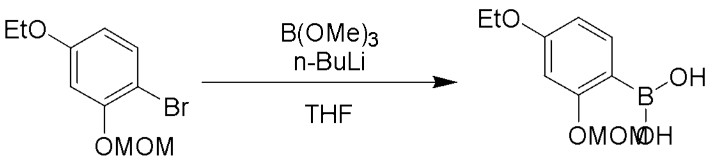


In a round-bottomed flask containing 4-bromo-3-(methoxymethoxy)phenol (21.5 mmol) in THF (40.0 mL), n-BuLi (2.50 M, 17.2 mL) was added at -70 °C under $N_2$ atmosphere. The reaction mixture was kept under agitation at -70 °C for 1 hour. Next, trimethylborate (85.8 mmol) was added at -70 °C. The mixture was kept under agitation at 20 °C for 1 hour. After total consumption of the starting reagent (monitored by LC-MS), the mixture was diluted with $H_2O$ (10.0 mL) at 0 °C and extracted with AcOEt (10.0 mL x 3). The organic phase obtained was concentrated under reduced pressure. 4.80 g (99.0% yield) of the product of interest were obtained in the form of a brown oil.

**STEP II-D-IV:**

**PRECURSOR IV-49IV:**

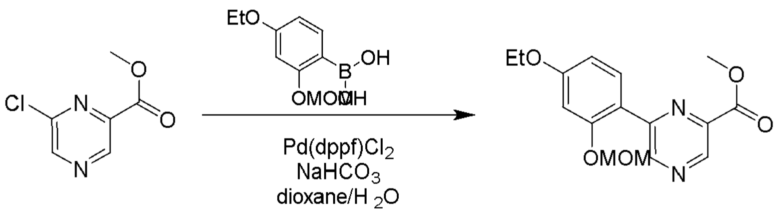


In a round-bottomed flask containing methyl 6-chloropyrazine-2-carboxylate (21.2 mmol), (4-ethoxy-2-(methoxymethoxy)phenyl)boronic acid (12.7 mmol), and $NaHCO_3$ (31.8 mmol) in dioxane (20.0 mL) and $H_2O$ (4.00 mL), $Pd(dppf)Cl_2$ (1.06 mmol) was added at 20-30 °C under $N_2$ atmosphere. The reaction mixture remained at 60 °C for 12 hours under an atmosphere of $N_2$. After total consumption of the starting reagent (monitored by CCD and LC-MS), the mixture was concentrated under reduced pressure. The residue obtained was diluted with $H_2O$ (20.0 mL) and extracted with AcOEt (20.0 mL x 3). The organic phase was washed with saturated sodium chloride solution *(brine)* (20.0 mL), dried on anhydrous sodium sulfate, filtered and concentrated under reduced pressure. The residue was purified by column chromatography (petroleum ether/AcOEt: 10/1 $\rightarrow$ 0/1). 3.50 g (51.7% yield) of the product of interest were obtained in the form of a yellow solid.

**STEP II-D-V:**

INTERMEDIATE IV-49:

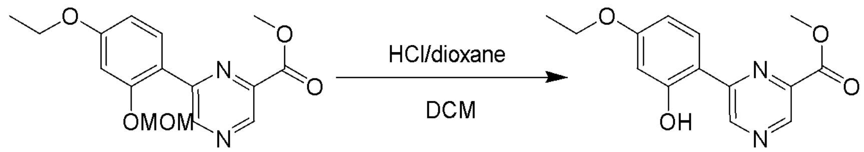


In a round-bottomed flask containing methyl 6-(4-ethoxy-2-(methoxymethoxy)phenyl)pyrazine-2-carboxylate (11.0 mmol), in DCM (20.0 mL), HCl/dioxane (54.8 mmol) was added. The reaction mixture remained under agitation at 30 °C for 1 hour. After total consumption of the starting reagent (monitored by CCD and LC-MS), the mixture was concentrated under reduced pressure. 3.00 g (99.5% yield) of the product of interest were obtained in the form of an orange solid, which was used in the next step without further purification.

**METHOD IV-E**

**STEP IV-E-I:**

**IV-50I - IV-56I PRECURSORS:**

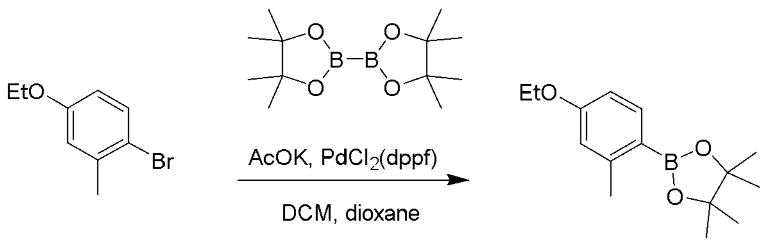


In a round-bottomed flask containing 1-bromo-4-ethoxy-2-methylbenzene (2.32 mmol) and 4,4,4',4',5,5,5',5'-octamethyl-2,2'-bi(1,3,2-dioxaborlan) (3.48 mmol) in 1,4-dioxane (7.5 mL), potassium acetate (5.81 mmol) was added at room temperature and the mixture was purged with $N_2$ for 10 minutes. Next, Pd(dppf)$Cl_2$/DCM (0.23 mmol) was added. The reaction mixture was kept under agitation at 80 °C for 2 hours. After total consumption of the starting reagent, the mixture was filtered with celite. The filtrate was diluted with $H_2O$ (50 mL) and extracted with n-hexane (50 mL x 3). The organic phase was washed with $H_2O$ (25 mL), dried on $Na_2SO_4$, filtered and concentrated under reduced pressure. The residue obtained was purified by column chromatography (100% n-hexane). 460 mg (37.74% yield) of the product of interest were obtained in the form of a yellow oil.

**TABLE 5. PRECURSORS OBTAINED FROM THE CORRESPONDING REAGENTS FOLLOWING STEP IV-E-I:**

| Intermediate | Structure |
|---|---|
| IV-50 | 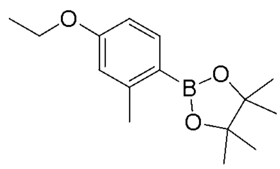 |
| IV-51 | 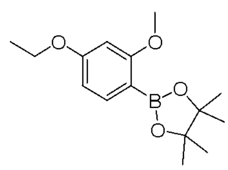 |

(continued)

| Intermediate | Structure |
|---|---|
| IV-52 | 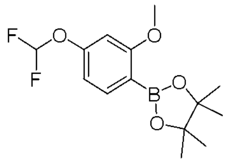 |
| IV-53 | 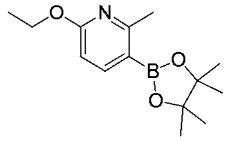 |
| IV-54 | 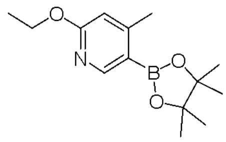 |
| IV-55 | 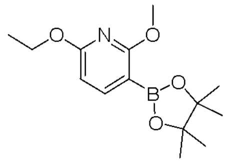 |
| IV-56 | 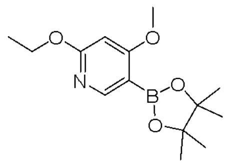 |

**STEP IV-E-II:**

**IV-50 - IV-56 INTERMEDIATES:**

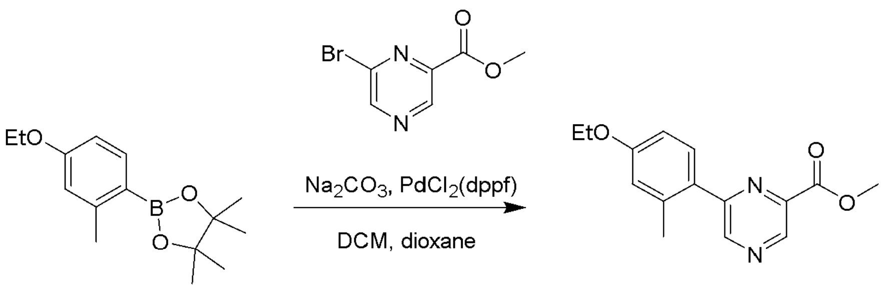


In a round-bottomed flask containing 2-(4-ethoxy-2-methylphenyl)-4,4,5,5-tetramethyl-1,3,2-dioxaborolan (1.75 mmol) and methyl 6-bromopyrazine-2-carboxylate (1.75 mmol) in 1,4-dioxane (5 mL), a solution of $Na_2CO_3$ (2M, 5 mL) was added at room temperature and the mixture was purged with $N_2$ for 10 minutes. Next, Pd(dppf)$Cl_2$/DCM (0.17 mmol) was added. The reaction mixture was kept under agitation at 50 °C for 1 hour. After total consumption of the starting reagent, the mixture was filtered with celite. The filtrate was diluted with $H_2O$ (50 mL) and extracted with n-hexane (50 mL x 3). The organic phase was washed with $H_2O$ (25 mL), dried on $Na_2SO_4$, filtered and concentrated under reduced pressure. The residue obtained was purified by column chromatography (n-hexane/AcOEt: 99/1). 330 mg (69.07% yield) of the product of interest were obtained in the form of a yellow oil.

**TABLE 6. PRECURSORS OBTAINED FROM THE CORRESPONDING REAGENTS FOLLOWING STEP IV-E-II**

| Intermediate | Structure |
|---|---|
| IV-50 | 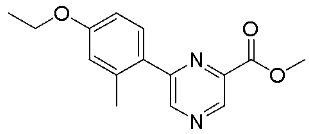 |
| IV-51 | 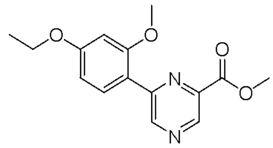 |
| IV-52 | 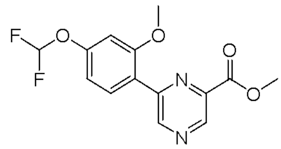 |
| IV-53 | 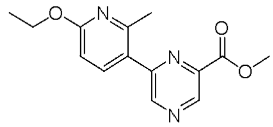 |
| IV-54 | 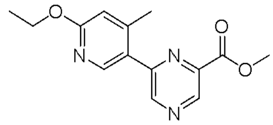 |
| IV-55 | 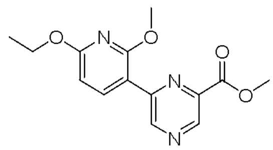 |
| IV-56 | 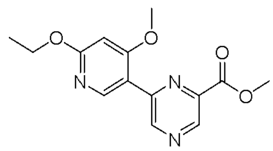 |

**FORMULA III INTERMEDIATES:**

Following General Scheme 1, the intermediates of Formula III are presented

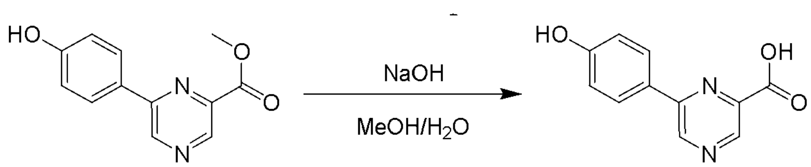


In a round-bottomed flask containing methyl 6-(4-hydroxyphenyl)pyrazine-2-carboxylate (3.65 mmol) and in MeOH (10.0 mL), NaOH (7.29 mmol) and $H_2O$ (2.00 mL) were added. The reaction mixture remained at 20 °C for 3 hours. After total consumption of the starting reagent (monitored by LC-MS and HPLC), the mixture was concentrated under reduced pressure and the pH of the residue obtained was adjusted to 5 with HCl solution (2 N). The mixture was then filtered and purified by preparative HPLC ([$H_2O$ (HCl) /ACN] 25% $\rightarrow$ 55% B) . 200 mg (21.0% yield) of the compound of interest (Table 7) were obtained in the form of a yellow solid.

**TABLE 7. INTERMEDIATES OBTAINED FROM THE CORRESPONDING REAGENTS FOLLOWING THE METHOD DESCRIBED IN OBTAINING THE COMPOUNDS OF FORMULA III.**

| Intermediate | Structure | Intermediate | Structure |
|---|---|---|---|
| III-1 | 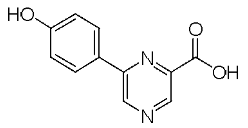 | III-27 | 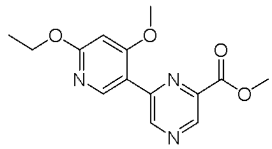 |
| III-2 | 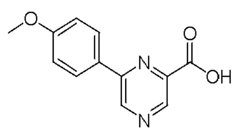 | III-28 | 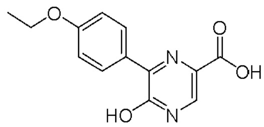 |
| III-3 | 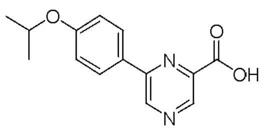 | III-29 | 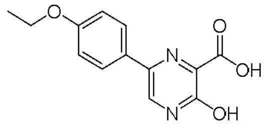 |
| III-4 | 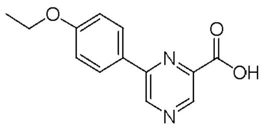 | III-30 | 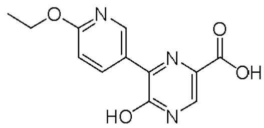 |
| III-5 | 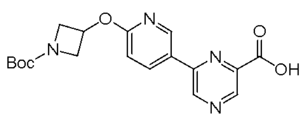 | III-31 | 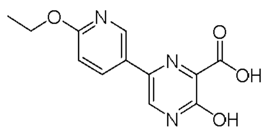 |
| III-6 | 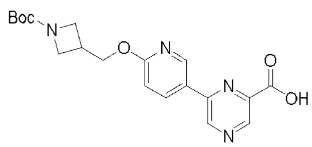 | III-32 | 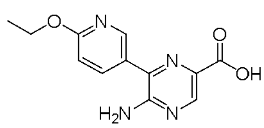 |
| III-7 | 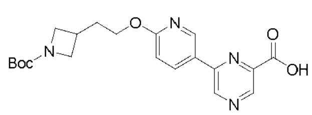 | III-33 | 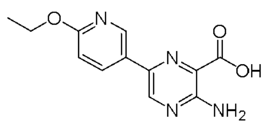 |
| III-8 | 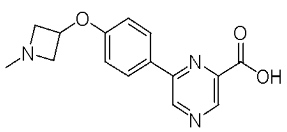 | III-34 | 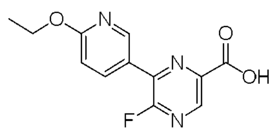 |
| III-9 | 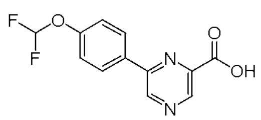 | III-35 | 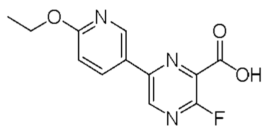 |
| III-10 | 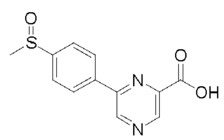 | III-36 | 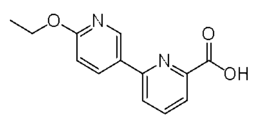 |
| III-11 | 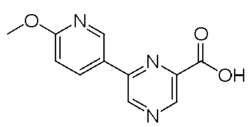 | III-37 | 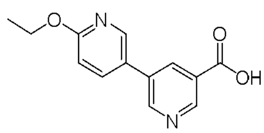 |

(continued)

| Intermediate | Structure | Intermediate | Structure |
|---|---|---|---|
| III-12 | 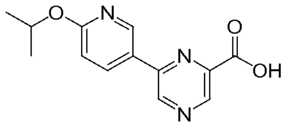 | III-38 | 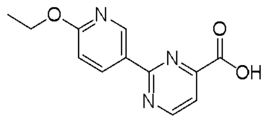 |
| III-13 | 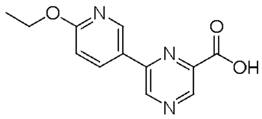 | III-39 | 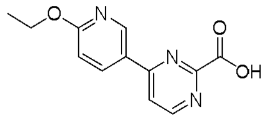 |
| III-14 | 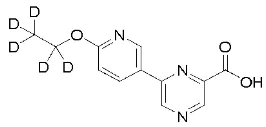 | III-40 | 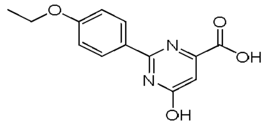 |
| III-15 | 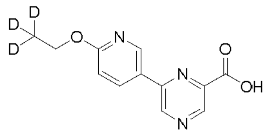 | III-41 | 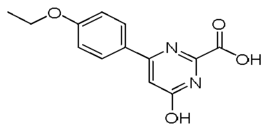 |
| III-16 | 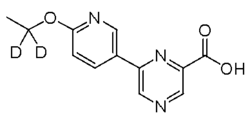 | III-42 | 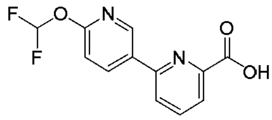 |
| III-17 | 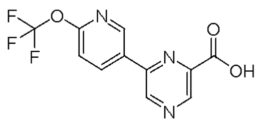 | III-43 | 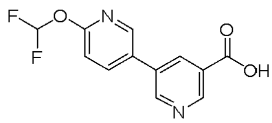 |
| III-18 | 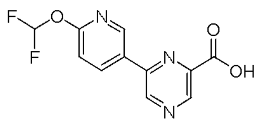 | III-44 | 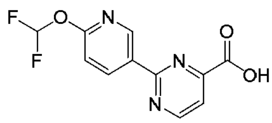 |
| III-19 | 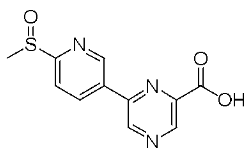 | III-45 | 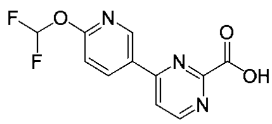 |
| III-20 | 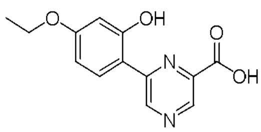 | III-46 | 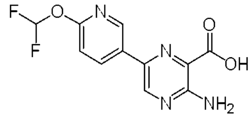 |
| III-21 | 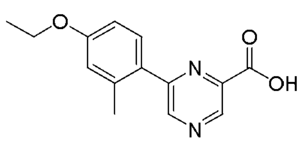 | III-47 | 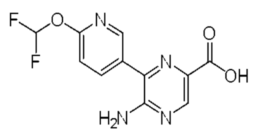 |
| III-22 | 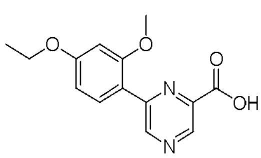 | III-48 | 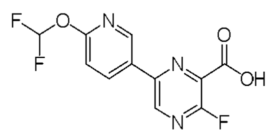 |

(continued)

| Intermediate | Structure | Intermediate | Structure |
|---|---|---|---|
| III-23 | 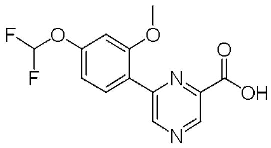 | III-49 | 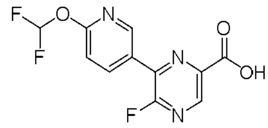 |
| III-24 | 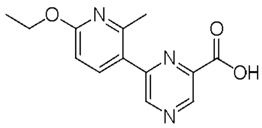 | III-50 | 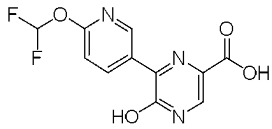 |
| III-25 | 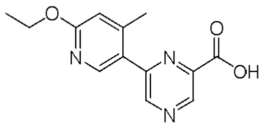 | III-51 | 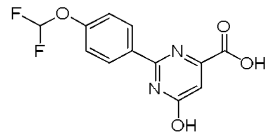 |
| III-26 | 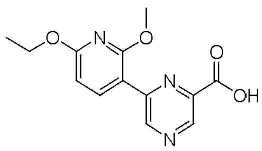 | III-52 | 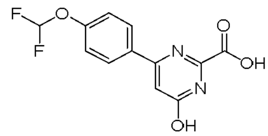 |

**OBTAINING FORMULA II COMPOUNDS**

Following General Scheme 1, the example methods II-A to II-X, for the formation of formula II intermediates are presented without limitation below.

**METHOD II-A**

**STEP II-A-I:**

**PRECURSOR II-1I:**

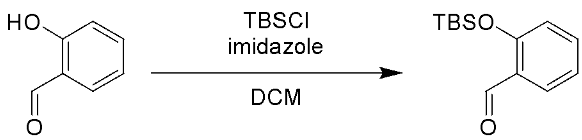


TBSCl (45.0 mmol) at 0-10 °C was added to a round-bottomed flask containing 2-hydroxybenzaldehyde (40.9 mmol) and imidazole (49.1 mmol) in anhydrous DCM (30.0 mL). Subsequently, the reaction mixture was kept under agitation at 20-30 °C for 2 hours. After total consumption of the starting reagent (monitored by CCD), the reaction mixture was diluted with $H_2O$ (30.0 mL) and extracted with DCM (20.0 mL x 2). Subsequently, the organic phase was concentrated under reduced pressure. 10 g of the product of interest were obtained in the form of a colorless oil.

**STEP II-A-II:**

**PRECURSOR II-1II:**

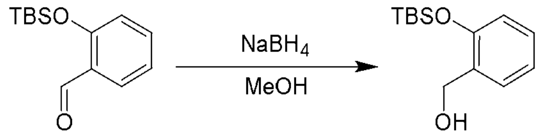


In a round-bottomed flask containing 2-((tert-butyldimethylsilyl)oxy)benzaldehyde (42.3 mmol) in MeOH (70.0 mL), $NaBH_4$ (36.0 mmol) was added slowly, under an atmosphere of $N_2$ at 0-20 °C. Subsequently, the reaction mixture was kept under agitation at 20-30 °C for 1 hour. After total consumption of the starting reagent (monitored by CCD), the reaction mixture was cooled to 0-10 °C and slowly diluted with acetone (10.0 mL). The mixture was kept under agitation at 0-10 °C for 30 minutes. Subsequently, the mixture was concentrated, and the residue was diluted with $H_2O$ (50.0 mL) and extracted with AcOEt (50.0 mL x 3). The organic phase was concentrated under reduced pressure and purified by column chromatography (petroleum ether/AcOEt: 100/1 $\rightarrow$ 5/1). 5.90 g (55.4 % yield) of the product of interest were obtained in the form of a yellow oil.

**STEP II-A-III:**

**PRECURSOR II-1III:**

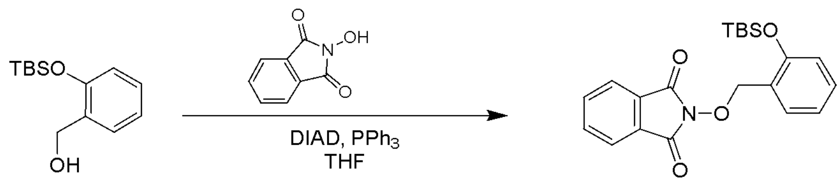


In a flask containing (2-((tert-butyldimethylsilyl)oxy)phenyl)methanol (21.8 mmol), 2-hydroxysoindoline-1,3-dione (28.36 mmol) and $PPh_3$ (32.7 mmol) in anhydrous THF (30.0 mL) DEAD (32.7 mmol) at 0-10 °C was added. Subsequently, the reaction mixture was kept under agitation at 20-30 °C for 16 hours. After total consumption of the starting reagent (monitored by CCD and LC-MS), the mixture was concentrated and purified by column chromatography (petroleum ether/AcOEt: 100/1 $\rightarrow$ 5/1). 5.30 g (63.3% yield) of the product of interest were obtained in the form of a white solid.

**STEP II-A-IV:**

**INTERMEDIATE II-1:**

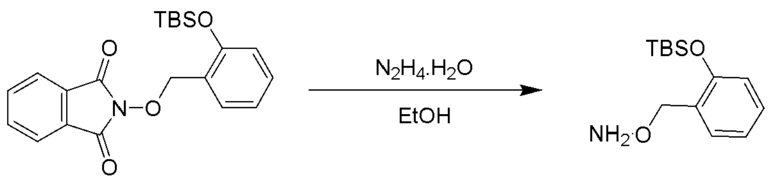


In a round-bottomed flask containing 2-((2-((tert-butyldimethylsilyl)oxy)benzyl)oxy)oxy)isoindoline-1,3-dione (5.47 mmol) in EtOH (36.0 mL), $NH_2NH_2H_2O$ (8.20 mmol) was added. Subsequently, the reaction mixture was kept under agitation at 90 °C for 2 hours. After total consumption of the starting reagent (monitored by CCD), the reaction mixture was filtered and concentrated under reduced pressure. The residue was purified by column chromatography (petroleum ether/AcOEt: 10/1 $\rightarrow$ 1/1). 0.99 g (72.8% yield) of the product of interest were obtained in the form of a colorless oil.

**METHOD B**

**STEP II-B-I:**

**PRECURSOR II-2I:**

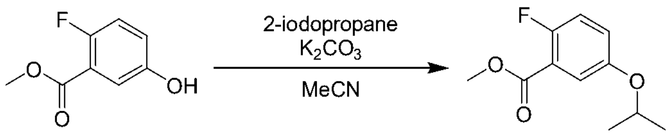


In a round-bottomed flask containing methyl 2-fluoro-5-hydroxybenzoate (11.7 mmol) in MeCN (12.0 mL), 2-iodopropane (23.5 mmol) and $K_2CO_3$ (23.5 mmol) were added. Subsequently, the reaction mixture was kept under agitation at 80 °C for 16 hours. After total consumption of the starting reagent (monitored by LC-MS), the reaction mixture was cooled to 25 °C and diluted with $H_2O$ (10.0 mL) and extracted with AcOEt (8.00 mL x 2). Subsequently, the organic phase was dried on anhydrous sodium sulfate, filtered and concentrated under reduced pressure. 2.10 g (84.1% yield) of the product of interest were obtained in the form of a yellow oil, which was used for the next step without further purification.

**STEP II-B-II:**

**PRECURSOR II-2II:**

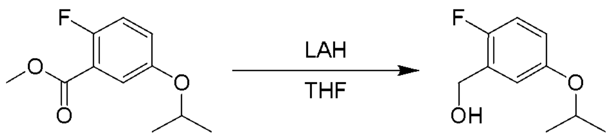


In a round-bottomed flask containing methyl 2-fluoro-5-isopropoxybenzoate (8.81 mmol) in THF (15.0 mL), $LiAlH_4$ (2.50 M, 10.5 mL) was added at 0 °C under $N_2$ atmosphere. Subsequently, the reaction mixture was kept in agitation at 0-20 °C for 2 hours. After total consumption of the starting reagent (monitored by LC-MS), $Na_2SO_4 \cdot 10H_2O$ (1.50 g) were added at 0 °C. The mixture was then filtered and concentrated under reduced pressure. 1.56 g (96.1% yield) of the product of interest were obtained in the form of a colorless oil, which was used for the next step without further purification.

**STEP II-B-III:**

**PRECURSOR II-2III:**

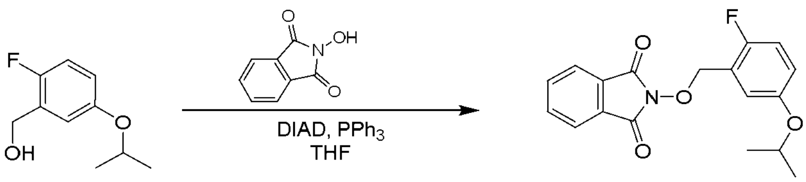


In a round-bottomed flask containing (2-fluoro-5-isopropoxyphenyl)methanol (9.23 mmol) in THF (17.0 mL), 2-hydroxysoindoline-1,3-dione (10.1 mmol), $PPh_3$ (13.8 mmol) and DIAD (13.8 mmol) were added at 0 °C. Subsequently, the reaction mixture was kept under agitation at 20 °C for 16 hours. After total consumption of the starting reagent (monitored by CCD), the reaction mixture was filtered. The residue was purified by column chromatography (petroleum ether/AcOEt: 10/1 $_{\rightarrow}$ 1/1). 1.8 g (59.23% yield) of the product of interest were obtained in the form of a white solid.

**STEP II-B-IV:**

## INTERMEDIATE II-2I:

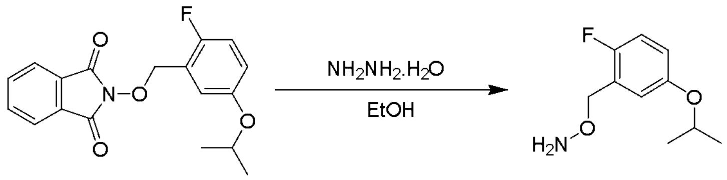


In a round-bottomed flask containing 2-((2-fluoro-5-isopropoxybenzyl)oxy)isoindoline-1,3-dione (5.47 mmol) in EtOH (36.0 mL), $NH_2NH_2H_2O$ (8.20 mmol) was added. Subsequently, the reaction mixture was kept under agitation at 90 °C for 2 hours. After total consumption of the starting reagent (monitored by CCD), the reaction mixture was filtered and concentrated under reduced pressure. The residue was purified by column chromatography (petroleum ether/AcOEt: 10/1 $\rightarrow$ 1/1). 0.99 g (72.8% yield) of the product of interest were obtained in the form of a colorless oil.

**METHOD C**

**STEP II-C-I:**

**PRECURSOR II-3I:**

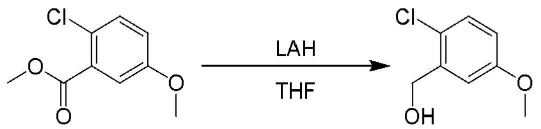


In a round-bottomed flask containing methyl 2-chloro-5-methoxybenzoate (5.98 mmol) in THF (3.00 mL), $LiAlH_4$ (2.50 M, 7.18 mL) was added at 0 °C. Subsequently, the reaction mixture was kept in agitation at 25 °C for 2 hours. After full consumption of the starting reagent (monitored by CCD), $Na_2SO_4 \cdot 10H$ was added to the reaction mixture $_2O$ (1.50 g) at 0 °C. The mixture was then filtered and concentrated under reduced pressure. 1.02 g (98.7% yield) of the product of interest were obtained in the form of a colorless oil, which was used for the next step without further purification.

**STEP II-C-II:**

**PRECURSOR II-3II:**

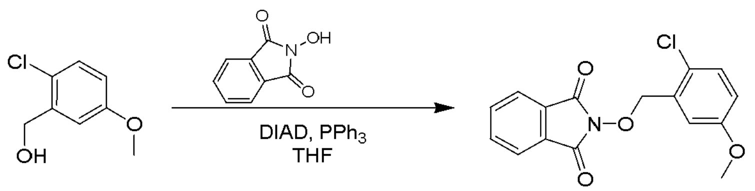


In a round-bottomed flask containing (2-chloro-5-methoxyphenyl)methanol (5.91 mmol) in THF (15.0 mL), 2-hydroxysoindoline-1,3-dione (6.50 mmol), $PPh_3$ (8.86 mmol), and DIAD (8.86 mmol) were added at 0 °C. Subsequently, the reaction mixture was kept under agitation at 20 °C for 16 hours. After total consumption of the starting reagent (monitored by CCD), the reaction mixture was filtered. The residue was purified by column chromatography (petroleum ether/AcOEt: 10/1 $\rightarrow$ 1/1). 1.2 g (63.91% yield) of the product of interest were obtained in the form of a white solid.

**STEP II-C-III:**

INTERMEDIATE II-3:

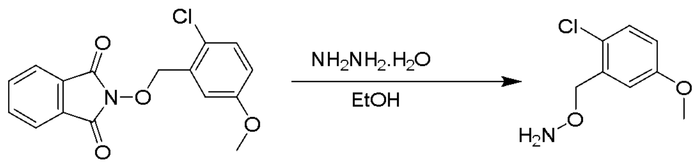


In a round-bottomed flask containing 2-((2-chloro-5-methoxybenzyl)oxy)isoindoline-1,3-dione (3.78 mmol) in EtOH (24.0 mL), $NH_2NH_2H_2O$ (5.67 mmol) was added. Subsequently, the reaction mixture was kept under agitation at 90 °C for 2 hours. After total consumption of the starting reagent (monitored by CCD), the reaction mixture was filtered and concentrated under reduced pressure. The residue was purified by column chromatography (petroleum ether/AcOEt: 10/1 → 3/1). 680 mg (83.2% yield) of the product of interest were obtained in the form of a colorless oil.

**METHOD D**

**STEP II-D-I:**

INTERMEDIATE II-4I:

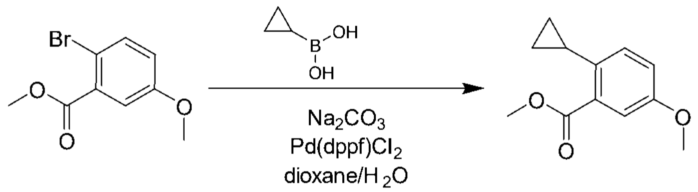


In a round-bottomed flask containing methyl 2-bromo-5-methoxybenzoate (20.4 mmol) in dioxane (17.0 mL), cyclopropylboronic acid (61.2 mmol), $H_2O$ (102 mmol), $NaHCO_3$ (40.8 mmol), and $Pd(dppf)Cl_2$ (2.04 mmol) was added under $N_2$ atmosphere. Subsequently, the reaction mixture was kept under agitation at 80 °C for 16 hours. After total consumption of the starting reagent (monitored by CCD), the reaction mixture was diluted in $H_2O$ (20.0 mL) and extracted with AcOEt (15.0 mL x 2). Subsequently, the organic phase was concentrated under reduced pressure and purified by column chromatography (petroleum ether/AcOEt: 10/1 → 2/1). 2.00 g (30.1% yield) of the product of interest were obtained in the form of a white solid.

**STEP II-D-II:**

INTERMEDIATE II-4II:

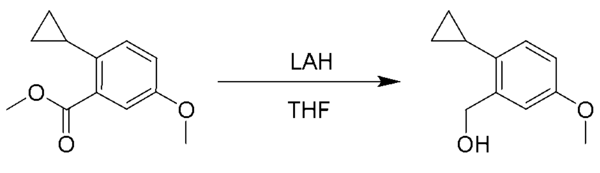


In a round-bottomed flask containing methyl 2-cyclopropyl-5-methoxybenzoate (7.27 mmol) in THF (10.0 mL), $LiAlH_4$ (2.50 M, 3.49 mL) was added at 0 °C under an atmosphere of $N_2$. Subsequently, the reaction mixture was kept in agitation at 20 °C for 2 hours. After full consumption of the starting reagent (monitored by CCD), $Na_2SO_4 \cdot 10H$ was added to the reaction mixture $_2O$ (5.00 g) at 0 °C. The mixture was then filtered and concentrated under reduced pressure. 1.23 g (94.8% yield) of the product of interest were obtained in the form of a colorless oil, which was used for the next step without further purification.

**STEP II-D-III:**

INTERMEDIATE II-4III:

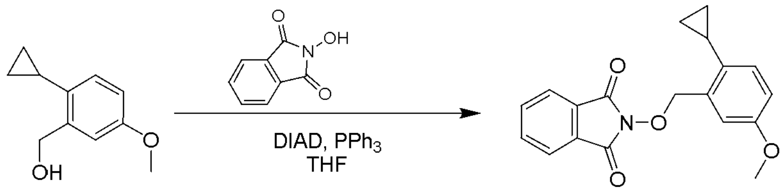


In a round-bottomed flask containing (2-cyclopropyl-5-methoxyphenyl)methanol (6.90 mmol) in THF (10.0 mL), 2-hydroxysoindoline-1,3-dione (7.59 mmol), DEAD (10.3 mmol), and $PPh_3$ (10.3 mmol) were added at 0 °C. Subsequently, the reaction mixture was kept under agitation at 20 °C for 16 hours. After total consumption of the starting reagent (monitored by CCD), the reaction mixture was filtered. The residue was purified by column chromatography (petroleum ether/AcOEt: 10/1 → 1/1). 1.20 g (53.7% yield) of the product of interest were obtained in the form of a white solid.

**STEP II-D-IV:**

INTERMEDIATE II-4:

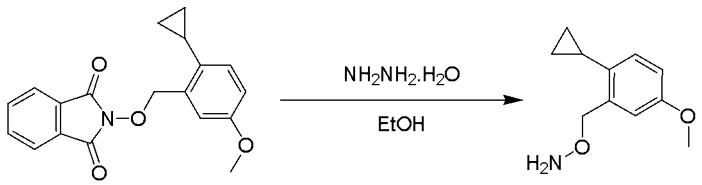


In a round-bottomed flask containing 2-((2-cyclopropyl-5-methoxybenzyl)oxy)isoindoline-1,3-dione (3.71 mmol) in EtOH (7.50 mL), $NH_2NH_2H_2O$ (5.57 mmol) was added. Subsequently, the reaction mixture was kept under agitation at 90 °C for 2 hours. After total consumption of the starting reagent (monitored by CCD), the reaction mixture was filtered and concentrated under reduced pressure. The residue was purified by column chromatography (petroleum ether/AcOEt: 10/1 → 1/1). 630 mg (87.8% yield) of the product of interest were obtained in the form of a colorless oil.

**METHOD E**

**STEP II-E-I:**

**PRECURSOR II-5I:**

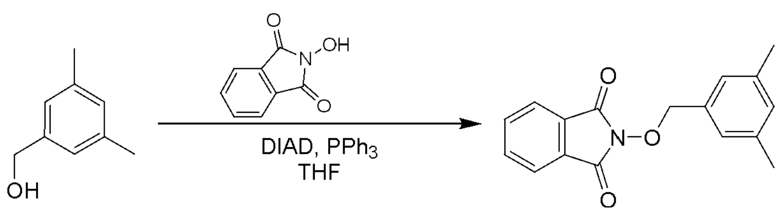


In a round-bottomed flask containing (3,5-dimethylphenyl)methanol (7.34 mmol) in THF (10.0 mL), 2-hydroxysoindoline-1,3-dione (8.08 mmol), $PPh_3$ (11.0 mmol), and DIAD (11.0 mmol) were added at 0 °C. Subsequently, the reaction mixture was kept under agitation at 20 °C for 16 hours. After total consumption of the starting reagent (monitored by CCD), the reaction mixture was filtered. The residue was purified by column chromatography (petroleum ether/AcOEt: 10/1 → 1/1). 1.50 g (72.6% yield) of the product of interest were obtained in the form of a white solid.

**STEP II-AND-II:**

**INTERMEDIATE II-5II:**

INTERMEDIATE II-5II:

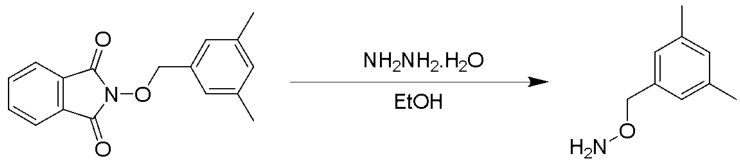


In a round-bottomed flask containing 2-((3,5-dimethylbenzyl)oxy)isoindoline-1,3-dione (3.78 mmol) in EtOH (24.0 mL), $NH_2NH_2H_2O$ (5.67 mmol) was added. Subsequently, the reaction mixture was kept under agitation at 90 °C for 2 hours. After total consumption of the starting reagent (monitored by CCD), the reaction mixture was filtered and concentrated under reduced pressure. The residue was purified by column chromatography (petroleum ether/AcOEt: 10/1 → 1/1). 700 mg (83.2% yield) of the product of interest were obtained in the form of a colorless oil.

**METHOD F**

**STEP II-F-I:**

**PRECURSOR II-6I:**

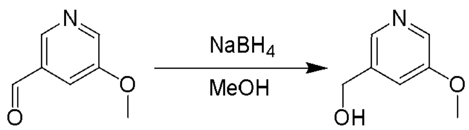


In a round-bottomed flask containing 5-methoxniycotineldehyde (7.29 mmol) in MeOH (10.0 mL), $NaBH_4$ (7.40 mmol) was added slowly at 0 °C. Subsequently, the reaction mixture was kept in agitation at 25 °C for 0.5 hours. After total consumption of the starting reagent (monitored by CCD and LC-MS), the mixture was cooled to 0-10 °C and diluted in acetone (10.0 mL) slowly. The mixture was placed under agitation at 0-10 °C for 30 minutes and subsequently concentrated. The residue was diluted in $H_2O$ (50.0 mL) and extracted with AcOEt (50.0 mL x 3). The organic phase was concentrated under reduced pressure. 1.00 g (98.5% yield) of the product of interest were obtained in the form of a yellow oil, which was used for the next step without further purification.

**STEP II-F-II:**

**PRECURSOR II-6II:**

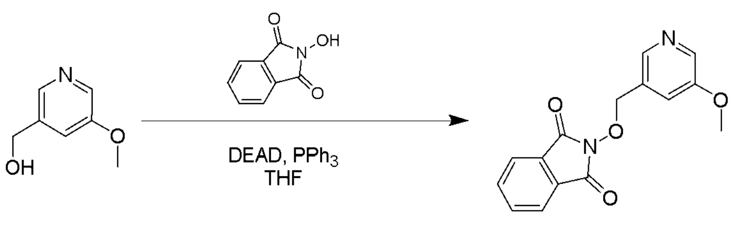


In a round-bottomed flask containing (5-methoxypyridine-3-yl)methanol (33.7 mmol), $PPh_3$ (50.7 mmol) in THF (25.0 mL), DEAD (9.21 mmol) was added at 0 °C under $N_2$ atmosphere. Subsequently, the reaction mixture was kept under

agitation at 30 °C for 16 hours. After total consumption of the starting reagent (monitored by CCD), the reaction mixture was concentrated under reduced pressure. The residue was placed under agitation with MeOH (20.0 mL) at 30 °C for 30 minutes. 7.20 g (74.9% yield) of the product of interest were obtained in the form of a white solid.

**STEP II-F-III:**

INTERMEDIATE II-6:

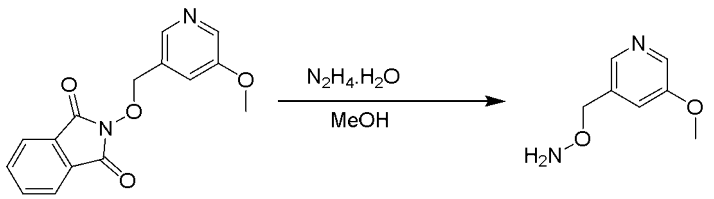


In a round-bottomed flask containing 2-((5-methoxypyridine-3-yl)methoxy)isoindoline-1,3-dione (29.2 mmol) in EtOH (50.0 mL), $NH_2NH_2H_2O$ (35.8 mmol) was added. Subsequently, the reaction mixture was kept under agitation at 90 °C for 2 hours. After total consumption of the starting reagent (monitored by CCD), the reaction mixture was filtered and washed with EtOH (10.0 mL x 3). The residue was purified by column chromatography (petroleum ether/AcOEt: 10/1 → 0/1). 4.00 g (88.9% yield) of the product of interest were obtained in the form of a yellow oil.

**METHOD G**

**STEP II-G-I:**

**PRECURSOR II-7I:**

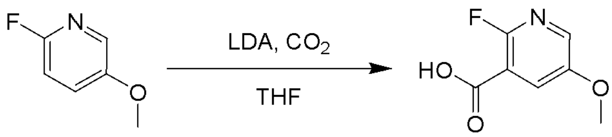


In a round-bottomed flask containing 2-fluoro-5-methoxypyridine (39.3 mmol) in THF (30.0 mL), a solution of LDA (2 M, 29.5 mL) was added dropwise at -60 °C. The reaction mixture remained at -60 °C under agitation for 2 hours. After total consumption of the starting reagent (monitored by CCD), an HCl solution (2N, 50.0 mL) at 0 °C was added. Subsequently, the reaction mixture was extracted with AcOEt (50.0 mL x 3). The organic phase was washed with saturated sodium chloride solution (*brine*) (50.0 mL), dried on anhydrous sodium sulfate, filtered and concentrated under reduced pressure. The residue was purified by column chromatography (petroleum ether/AcOEt: 100/1 → 10/1). 6.00 g (89.1% yield) of the product of interest were obtained in the form of a brown solid.

**STEP II-G-II:**

**PRECURSOR II-7II:**

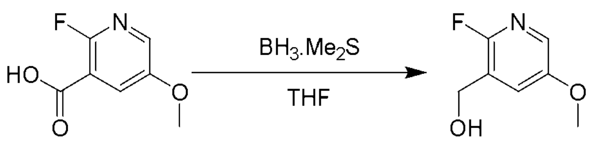


In a round-bottomed flask containing 2-fluoro-5-methoxynicotinic acid (35.1 mmol) in THF (60.0 mL), $BH_3MeS$ (10 M, 17.5 mL) was added dropwise at 0 °C. The reaction mixture remained at 20 °C under agitation for 16 hours. After

total consumption of the starting reagent (monitored by LC-MS), MeOH (50.0 mL) was added slowly at 0 °C and the reaction mixture was concentrated. Subsequently, 1-butanol (50.0 mL) was added, the reaction mixture was kept under agitation at 110 °C for 12 hours, and then concentrated. 5.50 g (99.8% yield) of the product of interest were obtained in the form of a brown oil, which was used in the next step without further purification.

**STEP II-G-III:**

**PRECURSOR II-7III:**

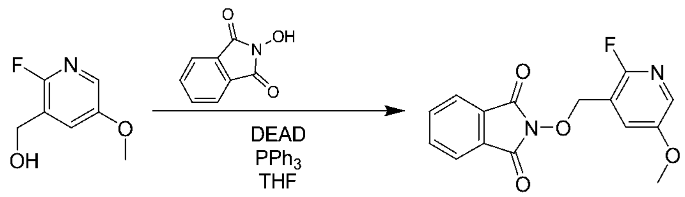


In a round-bottomed flask containing (2-fluoro-5-methoxypyridin-3-yl)methanol (35.0 mmol), 2-hydroxysoindoline-1,3-dione (42.0 mmol), and $PPh_3$ (52.5 mmol) in THF (35.0 mL), DEAD (52.5 mmol) was added at 0 °C. Subsequently, the reaction mixture was kept under agitation at 20 °C for 16 hours. After total consumption of the starting reagent (monitored by LC-MS), the reaction mixture was diluted in $H_2O$ (50.0 mL) and extracted with AcOEt (30.0 mL x 3). Subsequently, the organic phase was washed with saturated sodium chloride solution (*brine*) (50.0 mL), dried on anhydrous sodium sulfate, filtered and concentrated under reduced pressure. The residue was purified by column chromatography (petroleum ether/AcOEt: 100/1 → 10/1). 5.20 g (78.6% yield) of the product of interest were obtained in the form of a white solid.

**STEP II-G-IV:**

INTERMEDIATE II-7IV:

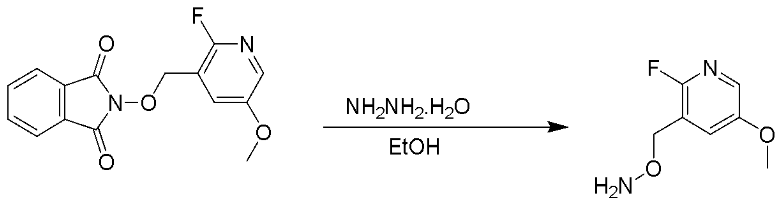


In a round-bottomed flask containing 2-((2-fluoro-5-methoxypyridin-3-yl)methoxy)isoindoline-1,3-dione (4.30 mmol) in EtOH (10.0 mL), $NH_2NH_2H_2O$ (12.0 mmol) was added. Subsequently, the reaction mixture was kept under agitation at 90 °C for 2 hours. After total consumption of the starting reagent (monitored by CCD), the reaction mixture was filtered and washed with EtOH (5.00 mL x 3). The residue was purified by column chromatography (petroleum ether/AcOEt: 10/1 → 0/1). 700 mg (94.5% yield) of the product of interest were obtained in the form of a yellow oil.

**METHOD H**

**STEP II-H-I:**

**PRECURSOR II-8I:**

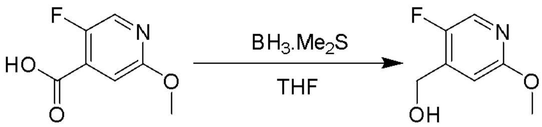


In a round-bottomed flask containing 5-fluoro-2-methoxysonicotinic acid (11.69 mmol, 2.00 g) in THF (10.0 mL), $BH_3MeS$ (10 M, 5.84 mL) was added dropwise at 0 °C. The reaction mixture remained at 0-20 °C under agitation for 16 hours. After total consumption of the starting reagent (monitored by LC-MS), MeOH (50.0 mL) was added slowly at 0 °C and the reaction mixture was concentrated. Subsequently, 1-butanol (50.0 mL) was added, the reaction mixture was kept under agitation at 110 °C for 12 hours, and then concentrated. 1.80 g (98.0% yield) of the product of interest were obtained, which was used in the next step without further purification.

**STEP II-H-II:**

**PRECURSOR II-8II:**

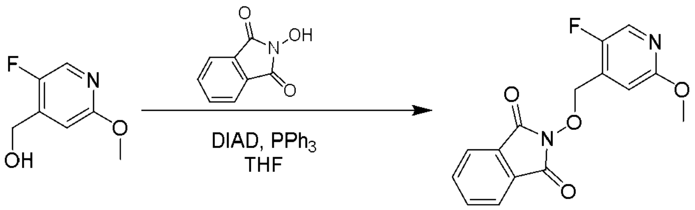


In a round-bottomed flask containing (5-fluoro-2-methoxypyridin-4-yl)methanol (11.45 mmol, 1.80 g), 2-hydroxysoindoline-1,3-dione (13.73 mmol, 2.24 g), and $PPh_3$ (17.19 mmol, 4.51 g) in THF (10.0 mL), DEAD (17.17 mmol, 2.99 g) was added at 0 °C. Subsequently, the reaction mixture was kept under agitation at 0-20 °C for 16 hours. After total consumption of the starting reagent (monitored by LC-MS), the reaction mixture was diluted in $H_2O$ (50.0 mL) and extracted with AcOEt (30.0 mL x 3). Subsequently, the organic phase was washed with saturated sodium chloride solution (*brine*) (50.0 mL), dried on anhydrous sodium sulfate, filtered and concentrated under reduced pressure. The residue was purified by column chromatography (petroleum ether/AcOEt: 100/1 → 10/1). 1.60 g (33.0% yield) of the product of interest were obtained.

**STEP II-H-III:**

**INTERMEDIATE II-8:**

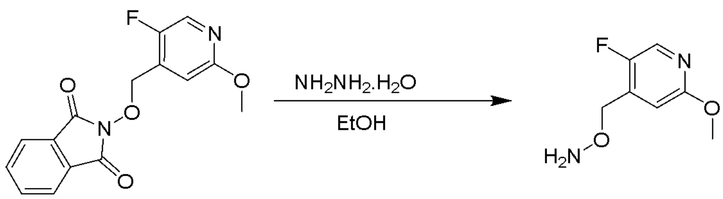


In a round-bottomed flask containing 2-((5-fluoro-2-methoxypyridin-4-yl)methoxy)isoindoline-1,3-dione (5.29 mmol, 1.60 g) in EtOH (10.0 mL), $NH_2NH_2H_2O$ (6.39 mmol, 0.320 g) was added. Subsequently, the reaction mixture was kept under agitation at 90 °C for 2 hours. After total consumption of the starting reagent (monitored by CCD), the reaction mixture was filtered and washed with EtOH (5.00 mL x 3). The residue was purified by column chromatography (petroleum ether/AcOEt: 10/1 → 0/1). 750 mg (82.3% yield) of the product of interest were obtained in the form of a yellow oil.

**METHOD I**

**STEP II-I-I:**

**PRECURSORS II-9I - II-20I:**

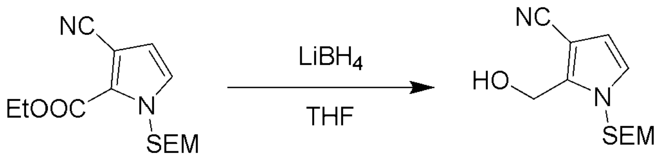


In a round-bottomed flask containing 2-((5-fluoro-2-methoxypyridin-4-yl)methoxy)isoindoline-1,3-dione (1.80 mmol, 6.11 g) in THF (10.0 mL), $LiNH_4$ (8.83e-1 eq, 2.70 g) was added. Subsequently, the reaction mixture was kept under agitation at 60 °C for 2 hours. After total consumption of the starting reagent (monitored by CCD), the reaction mixture was filtered and extracted with AcOEt (20.0 mL x 3). The organic phase was washed with anhydrous sodium sulfate, filtered and concentrated at reduced pressure. 1.70 g of the product of interest were obtained in the form of a colorless oil, which was used in the next step for further purification.

**TABLE 8. PRECURSORS OBTAINED FROM THE CORRESPONDING REAGENTS FOLLOWING STEP II-I-I**

| Intermediate | Structure |
|---|---|
| II-9i | 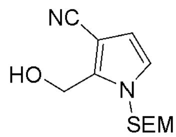 |
| II-10i | 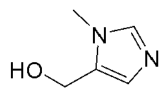 |
| II-11i | 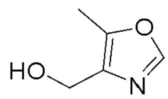 |
| II-12i | 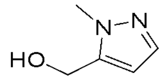 |
| II-13i | 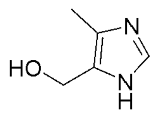 |
| II-14i | 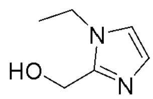 |
| II-15i | 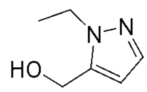 |
| II-16i | 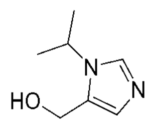 |
| II-17i | 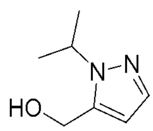 |

(continued)

| Intermediate | Structure |
|---|---|
| II-18i | 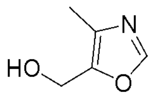 |
| II-19i | 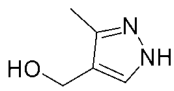 |
| II-20i | 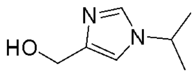 |

**STEP II-I-II:**

**PRECURSORS II-9II - II-20II:**

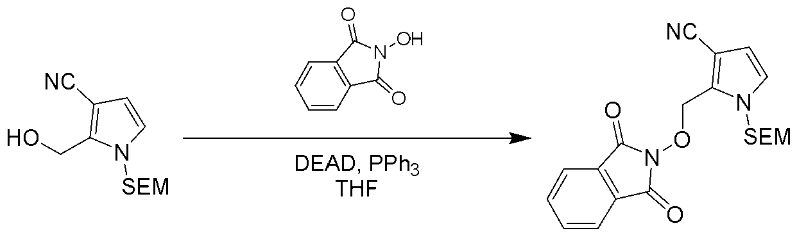


In a flask containing 2-(hydroxymethyl)-1-((2-(trimethylsilyl)ethoxy)methyl)-1*H*-pyrrole-3-carbonitrile (6.34 mmol), 2-hydroxysoindoline-1,3-dione (8.24 mmol) and $PPh_3$ (9.51 mmol) in THF (20.0 mL) was added DEAD (9.51 mmol) dropwise under an atmosphere of $N_2$ at 0-10 °C. Subsequently, the reaction mixture was kept under agitation at 20-30 °C for 16 hours. After total consumption of the starting reagent (monitored by LC-MS), the reaction mixture was diluted with $H_2O$ (30.0 mL) and extracted with AcOEt (30.0 mL x 3). The organic phase was dried on anhydrous sodium sulfate, filtered and concentrated under reduced pressure. The residue obtained was placed under agitation with MeOH (2.00 mL) at 0-10 °C for 30 minutes. The mixture was filtered and washed with MeOH (2.00 mL). 1.60 g (63.4% yield) of the product of interest were obtained in the form of a white solid.

**TABLE 9. PRECURSORS OBTAINED FROM THE CORRESPONDING REAGENTS FOLLOWING STEP II-I-II.**

| Intermediate | Structure |
|---|---|
| II-9ii | 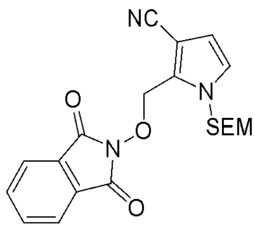 |
| II-10ii | 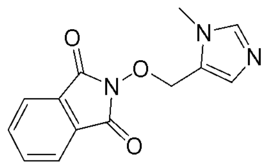 |

(continued)

| Intermediate | Structure |
|---|---|
| II-11ii | 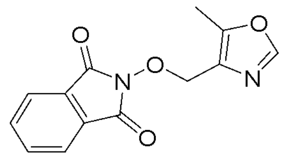 |
| II-12ii | 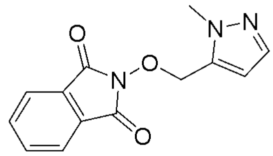 |
| II-13ii | 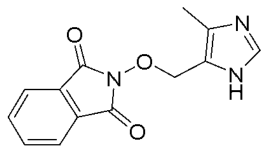 |
| II-14ii | 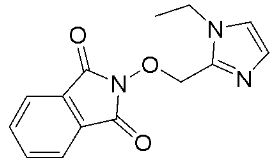 |
| II-15ii | 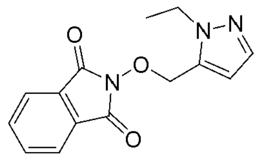 |
| II-16ii | 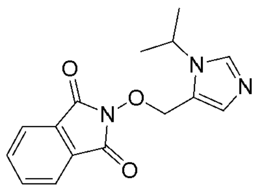 |
| II-17ii | 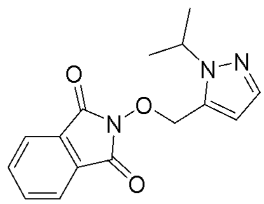 |
| II-18ii | 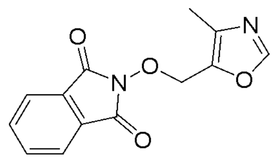 |
| II-19ii | 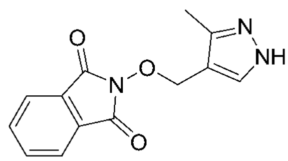 |

(continued)

| Intermediate | Structure |
|---|---|
| II-20ii | 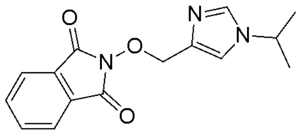 |

**STEP II-I-III:**

INTERMEDIATES II-9 - II-20:

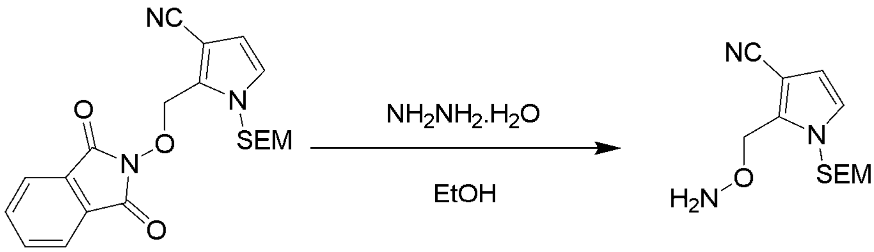


In a round-bottomed flask containing 2-(((1,3-dioxoisoindoline-2-yl)oxy)methyl)-1-((2-(trimethylsilyl)ethoxy)methyl)-1*H*-pyrrole-3-carbonitrile (2.16 mmol) in EtOH (10.0 mL), $NH_2NH_2H_2O$ (5.59 mmol) was added. Subsequently, the reaction mixture was kept under agitation at 90 °C for 2 hours. After total consumption of the starting reagent (monitored by CCD), the reaction mixture was cooled to 20-30 °C, filtered, washed with EtOH (10.0 mL) and concentrated. 570 mg (98.5% yield) of the product of interest were obtained in the form of a colorless oil, which was used in the next step without further purification.

**TABLE 10. PRECURSORS OBTAINED FROM THE CORRESPONDING REAGENTS FOLLOWING STEP II-I-III.**

| Intermediate | Structure |
|---|---|
| II-9 | 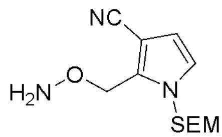 |
| II-10 | 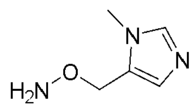 |
| II-11 | 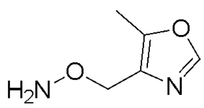 |
| II-12 | 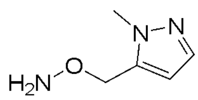 |
| II-13 | 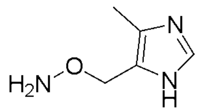 |
| II-14 | 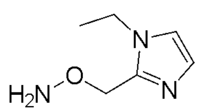 |

(continued)

| Intermediate | Structure |
|---|---|
| II-15 | 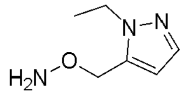 |
| II-16 | 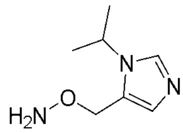 |
| II-17 | 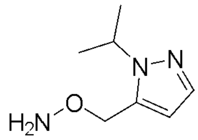 |
| II-18 | 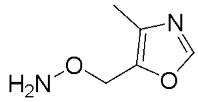 |
| II-19 | 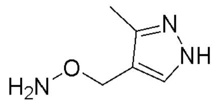 |
| II-20 | 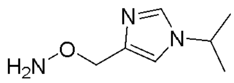 |

**J METHOD**

**STEP II-J-I:**

**PRECURSOR II-21I:**

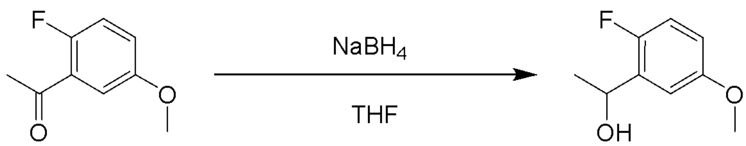


In a round-bottomed flask containing 1-(2-fluoro-5-methoxyphenyl)etan-1-one (10.7 mmol) in THF (90.0 mL), $NaBH_4$ (15.9 mmol) at 0 °C was added. Subsequently, the reaction mixture was kept in agitation at 25 °C for 1 hour. After total consumption of the starting reagent (monitored by CCD), the reaction mixture was diluted in $H_2O$ (50.0 mL), AcOEt (40.0 mL) at 0 °C and extracted with AcOEt (40.0 mL x 3). The organic phase was washed with saturated sodium chloride solution (*brine*) (20.0 mL x 2), dried in anhydrous sodium sulfate, filtered and concentrated under reduced pressure. 1.75 g (96.1% yield) of the product of interest were obtained in the form of a white oil, which was used for the next step without further purification.

**STEP II-J-II:**

**PRECURSOR II-21II:**

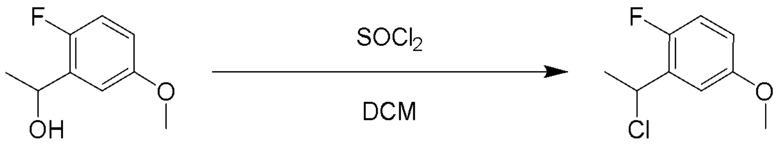


In a round-bottomed flask containing 1-(2-fluoro-5-methoxyphenyl)etan-1-ol (8.23 mmol) in DCM (20 mL), $SOCl_2$ (16.4 mmol) was added at 0 °C. Subsequently, the reaction mixture was kept in agitation at 25 °C for 16 hours. After full consumption of the starting reagent (monitored by CCD), the concentrated reaction mixture under reduced pressure. 1.50 g (96.7% yield) of the product of interest were obtained in the form of a brown oil, which was used for the next step without further purification.

**STEP II-J-III:**

**PRECURSOR II-21III:**

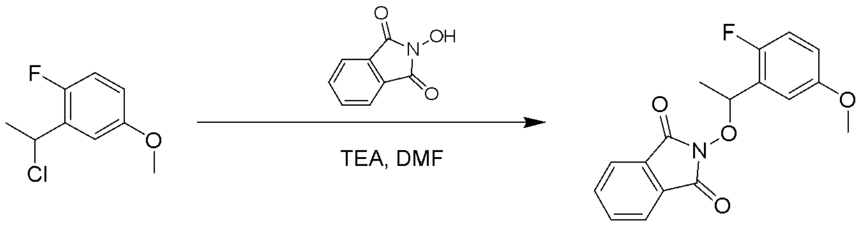


In a round-bottomed flask containing 2-(1-chloroethyl)-1-fluoro-4-methoxybenzene (11.9 mmol) and compound 7-b (7.95 mmol) in DMF (15.0 mL), TEA (15.9 mmol) was added at 25 °C. Subsequently, the reaction mixture was kept in agitation at 60 °C for 12 hours. After total consumption of the starting reagent (monitored by CCD), the reaction mixture was diluted with $H_2O$ (5.00 mL) at 0-10 °C and AcOEt (5.00 mL) was added. The mixture was extracted with AcOEt (5.00 mL x 2). The organic phase was washed with saturated sodium chloride solution (*brine*) (5.00 mL x 2), dried in anhydrous sodium sulfate, filtered and concentrated under reduced pressure. The residue was purified by column chromatography (petroleum ether/AcOEt: 10/1 → 0/1). 1.80 g (71.79% yield) of the product of interest were obtained in the form of a white solid.

**STEP II-J-III:**

INTERMEDIATE II-21:

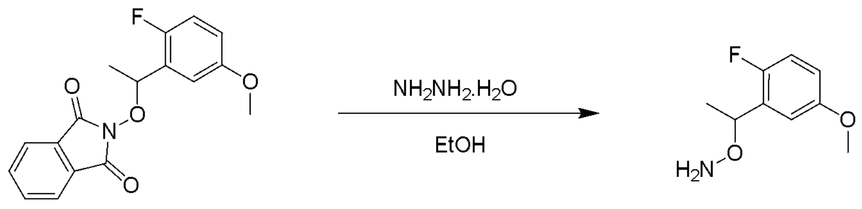


In a round-bottomed flask containing 2-(1-(2-fluoro-5-methoxyphenyl)ethoxy)isoindoline-1,3-dione (5.71 mmol) in EtOH (20.0 mL), $NH_2NH_2H_2O$ (8.56 mmol) at 25 °C was added. Subsequently, the reaction mixture was kept under agitation at 90 °C for 2 hours. After total consumption of the starting reagent (monitored by CCD), the reaction mixture was filtered, washed with EtOH (30 mL x 3) and concentrated under reduced pressure. 950 mg (89.8% yield) of the product of interest were obtained in the form of a yellow oil.

## EXAMPLES

The following examples, described in detail, serve to illustrate the embodiments of this invention without, however, having any limitation character to the scope of protection thereof.

The General Formula I compound(s) of this invention were obtained from the amide coupling between the Formula III intermediates with the Formula II intermediates described above, synthesized according to the various methodologies, described above and schematized in General Scheme 1, but not limited to these.

## EXAMPLE 1. N-((2-FLUORO-5-METHOXYBENZYL)OXY)-6-(4-HYDROXYPHENYL)PYRAZINE-2-CARBOXAMIDE

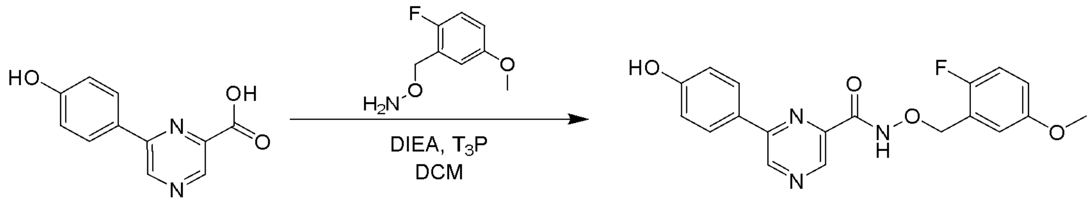


In a round-bottomed flask, 6-(4-hydroxyphenyl)pyrazine-2-carboxylic acid (768 μmol), O-[(2-fluoro-5-methoxyphenyl)methyl]hydroxylamine (768 μmol), T3P (3.87 mmol, 50% purity), DIEA (1.15 mmol) in DCM (10 mL) were added. The reaction mixture was kept under agitation for 16 hours at 20 °C. Upon conclusion of the reaction, the mixture was diluted with water and poured with DCM (3 x 10 mL). The organic phases were washed with anhydrous $Na_2SO_4$ and concentrated under reduced pressure. The product was crushed with methanol (10 mL) at 20oC for 20 min. The product of interest **1** was obtained in the form of a yellow solid.

The compounds **2** - **156,** as shown in table 11, are obtained by the procedure described for example 1 (compound 1), changing the corresponding intermediates II and III.

**TABLE 11. COMPOUNDS OBTAINED FROM THE CORRESPONDING REACTANTS FOLLOWING METHOD I-A.**

| Compound Nº | Structure | $^1$H-NMR / MS(m/z) [M+H]$^+$ |
|---|---|---|
| 1 | 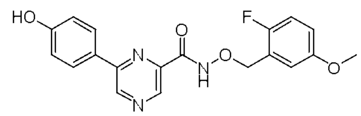 *N*-((2-fluoro-5-methoxybenzyl)oxy)-6-(4-hydroxyphenyl) pyrazine-2-carboxamide | $^1$H-NMR, 400 MHz, DMSO-$d_6$ $\delta$ = 12.31 (s, 1H), 10.15 - 9.96 (m, 1H), 9.41 (s, 1H), 9.00 (s, 1H), 8.31 (br d, J = 8.6 Hz, 2H), 7.27 - 7.16 (m, 2H), 7.08 - 6.93 (m, 3H), 5.10 (br s, 2H), 3.82 (s, 3H). [M+H]$^+$: 370.1 |
| 2 | 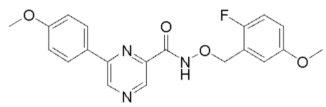 *N*-((2-fluoro-5-methoxybenzyl)oxy)-6-(4-methoxyphenyl) pyrazine-2-carboxamide | $^1$H-NMR, 400 MHz, DMSO-$d_6$ $\delta$ = 12.30 (s, 1H), 9.42 (s, 1H), 8.98 (s, 1H), 8.36 (d, J = 8.80 Hz, 2H), 7.20 to 7.12 (m, 2H), 7.11 to 7.09 (m, 2H), 7.00 to 6.95 (m, 1H), 5.05 (s, 2H), 3.86 (s, 3H), 3.76 (s, 3H). [M+H]$^+$: 384.1. |
| 3 | 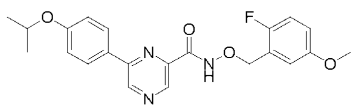 *N*-((2-fluoro-5-methoxybenzyl)oxy)-6-(4-isopropoxyphenyl) pyrazine-2-carboxamide | $^1$H-NMR, 400 MHz, DMSO-$d_6$ $\delta$ = 12.30 (s, 1H), 9.41 (s, 1H), 8.98 (s, 1H), 8.34 (d, J = 8.80 Hz, 2H), 7.20 to 7.16 (m, 2H), 7.15 (d, J = 3.60 Hz, 2H), 7.09 to 7.00 (m, 1H), 5.05 (s, 2H), 4.79 to 4.76 (m, 1H), 3.76 (s, 3H), 1.32 (d, J = 6.00 Hz, 6H). [M+H]$^+$: 412.1. |

(continued)

| Compound Nº | Structure | $^1$H-NMR / MS(m/z) [M+H]$^+$ |
|---|---|---|
| 4 | 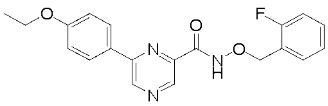 6-(4-ethoxyphenyl)-*N*-((2-fluorobenzyl)oxy)pyrazine-2-carboxamide | $^1$H-NMR, 400 MHz, Methanol-d$_4$ $\delta$ = 9.24 (s, 1H), 8.99 (s, 1H), 8.26 - 8.06 (m, 2H), 7.61 (dt, J = 1.7, 7.5 Hz, 1H), 7.48 - 7.34 (m, 1H), 7.24 - 7.18 (m, 1H), 7.13 (t, J = 8.9 Hz, 1H), 7.09 - 6.99 (m, 2H), 5.14 (s, 2H), 4.13 (q, J = 7.0 Hz, 2H), 1.43 (t, J = 7.0 Hz, 3H). [M+H]$^+$: 368.1. |
| 5 | 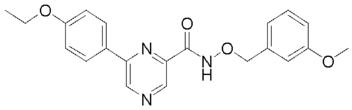 6-(4-ethoxyphenyl)-*N*-((3-methoxybenzyl)oxy)pyrazine-2-carboxamide | $^1$H-NMR, 400 MHz, DMSO-d$_6$ $\delta$ = 12.28 (s, 1H), 9.52 - 9.37 (m, 1H), 9.09 - 8.94 (m, 1H), 8.35 (d, J = 8.8 Hz, 2H), 7.36 - 7.29 (m, 1H), 7.13 - 7.04 (m, 4H), 6.94 (dd, J = 2.0, 8.3 Hz, 1H), 4.98 (s, 2H), 4.14 (q, J = 7.0 Hz, 2H), 3.78 (s, 3H), 1.37 (t, J = 7.0 Hz, 3H). [M+H]$^+$: 380.2. |
| 6 | 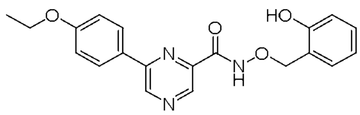 6-(4-ethoxyphenyl)-*N*-((2-hydroxybenzyl)oxy)pyrazine-2-carboxamide | $^1$H-NMR, 400 MHz, DMSO-d$_6$ $\delta$ = 12.31 - 12.10 (m, 1H), 9.49 (br d, J = 3.3 Hz, 1H), 9.20 (s, 1H), 8.78 (s, 1H), 8.15 (d, J = 8.8 Hz, 2H), 7.14 (d, J = 6.5 Hz, 1H), 7.02 - 6.93 (m, 1H), 6.86 (d, J = 8.9 Hz, 2H), 6.68 - 6.55 (m, 2H), 4.77 (s, 2H), 3.91 (q, J = 6.9 Hz, 2H), 1.14 (t, J = 7.0 Hz, 3H). [M+H]$^+$: 366.1. |
| 7 | 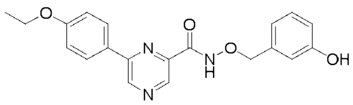 6-(4-ethoxyphenyl)-*N*-((3-hydroxybenzyl)oxy)pyrazine-2-carboxamide | $^1$H-NMR, 400 MHz, DMSO-d$_6$ $\delta$ = 12.27 (s, 1H), 9.48 (s, 1H), 9.42 (s, 1H), 8.99 (s, 1H), 8.36 (d, J = 8.8 Hz, 2H), 7.19 (t, J = 7.8 Hz, 1H), 7.09 (d, J = 8.8 Hz, 2H), 6.94 - 6.88 (m, 2H), 6.78 - 6.72 (m, 1H), 4.90 (s, 2H), 4.13 (q, J = 6.9 Hz, 2H), 1.37 (t, J = 7.0 Hz, 3H). [M+H]$^+$: 366.1. |
| 8 | 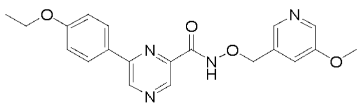 6-(4-ethoxyphenyl)-*N*-((5-methoxypyridine-3-yl)methoxy) pyrazine-2-carboxamide | $^1$H-NMR, 400 MHz, DMSO-d$_6$ $\delta$ = 1.37 (t, J=6.94 Hz, 3 H) 3.85 (s, 3 H) 4.13 (q, J=6.96 Hz, 2 H) 5.05 (s, 2 H) 7.09 (d, J=8.88 Hz, 2 H) 7.54 (s, 1 H) 8.25 - 8.38 (m, 4 H) 8.98 (s, 1 H) 9.42 (s, 1 H) 12.32 (s, 1 H). [M+H]$^+$: 381.2. |
| 9 | 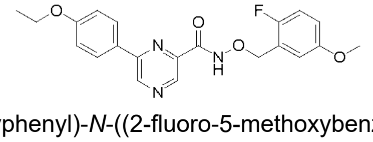 6-(4-ethoxyphenyl)-*N*-((2-fluoro-5-methoxybenzyl)oxy)pyrazine-2-carboxamide | $^1$H-NMR, 400 MHz, DMSO-d$_6$ $\delta$ = 12.30 (s, 1H), 9.42 (s, 1H), 8.98 (s, 1H), 8.35 (dd, J = 2.00, 7.00 Hz, 1H), 7.20-7.14 (m, 2H), 7.10-7.08 (m, 2H), 6.99-6.97 (m, 1H), 5.04 (s, 2H), 4.14 (q, J = 6.80 Hz, 2H), 3.76 (s, 3H), 1.37 (t, J = 6.80 Hz, 3H). [M+H]$^+$: 398.5. |

(continued)

| Compound № | Structure | $^1$H-NMR / MS(m/z) [M+H]$^+$ |
|---|---|---|
| 10 | 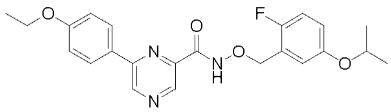 6-(4-ethoxyphenyl)-*N*-((2-fluoro-5-isopropoxybenzyl)oxy) pyrazi ne-2-carboxamide | $^1$H-NMR, 400 MHz, DMSO-$d_6$ $\delta$ = 12.28 (s, 1H), 9.41 (s, 1H), 8.96 (s, 1H), 8.34 (d, J = 8.8 Hz, 2H), 7.17 - 7.04 (m, 4H), 6.98 - 6.91 (m, 1H), 5.02 (s, 2H), 4.54 (td, J = 6.1, 12.1 Hz, 1H), 4.13 (q, J = 6.9 Hz, 2H), 1.37 (t, J = 6.9 Hz, 3H), 1.21 (d, J = 6.0 Hz, 6H). [M+H]$^+$: 426.1. |
| 11 | 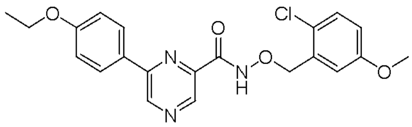 *N*-((2-chloro-5-methoxybenzyl)oxy)-6-(4-ethoxyphenyl)pyrazine-2-carboxamide | $^1$H-NMR, 400 MHz, DMSO-$d_6$ $\delta$ = 12.33 (s, 1H), 9.41 (s, 1H), 8.98 (s, 1H), 8.34 (d, J = 8.8 Hz, 2H), 7.40 (d, J = 8.8 Hz, 1H), 7.28 (d, J = 3.1 Hz, 1H), 7.08 (d, J = 8.8 Hz, 2H), 6.97 (dd, J = 3.2, 8.8 Hz, 1H), 5.10 (s, 2H), 4.13 (q, J = 7.0 Hz, 2H), 3.78 (s, 3H), 1.37 (t, J = 7.0 Hz, 3H). [M+H]$^+$: 414.0. |
| 12 | 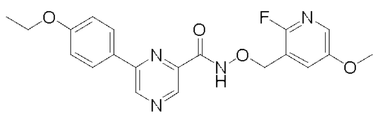 6-(4-ethoxyphenyl)-N-((2-fluoro-5-methoxypyridine-3-yl) methoxy)pyrazine-2-carboxamide | $^1$H-NMR, 400 MHz, DMSO-$d_6$ $\delta$ = 12.32 (s, 1H), 9.42 (s, 1H), 8.97 (s, 1H), 8.34 (br d, J = 8.8 Hz, 2H), 7.96 - 7.90 (m, 1H), 7.78 (dd, J = 3.0, 7.6 Hz, 1H), 7.09 (d, J = 8.9 Hz, 2H), 5.05 (s, 2H), 4.14 (q, J = 6.9 Hz, 2H), 3.84 (s, 3H), 1.38 (t, J = 7.0 Hz, 3H). [M+H]$^+$: 399.1. |
| 13 | 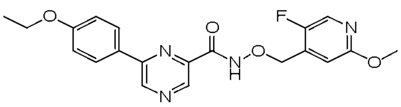 6-(4-ethoxyphenyl)-*N*-((5-fluoro-2-methoxypyridine-4-yl) methoxy)pyrazine-2-carboxamide | $^1$H-NMR, 400 MHz, DMSO-$d_6$ $\delta$ = 12.37 (s, 1H), 9.42 (s, 1H), 8.98 (s, 1H), 8.34 (d, J = 8.8 Hz, 2H), 8.17 (d, J = 1.2 Hz, 1H), 7.14 - 7.03 (m, 3H), 5.11 (s, 2H), 4.14 (q, J = 7.0 Hz, 2H), 3.85 (s, 3H), 1.37 (t, J = 7.0 Hz, 3H). [M+H]$^+$: 399.1. |
| 14 | 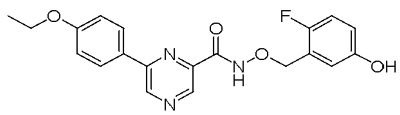 6-(4-ethoxyphenyl)-*N*-((2-fluoro-5-hydroxybenzyl)oxy)pyrazine-2-carboxamide | $^1$H-NMR, 400 MHz, DMSO-$d_6$ $\delta$ = 12.31 (br s, 1H), 9.57 - 9.37 (m, 2H), 8.98 (s, 1H), 8.36 (br d, J = 8.8 Hz, 2H), 7.11 - 7.00 (m, 3H), 6.95 (dd, J = 3.0, 5.8 Hz, 1H), 6.81 - 6.71 (m, 1H), 4.96 (s, 2H), 4.13 (q, J = 6.9 Hz, 2H), 1.37 (t, J = 7.0 Hz, 3H). [M+H]$^+$: 384.1. |
| 15 | 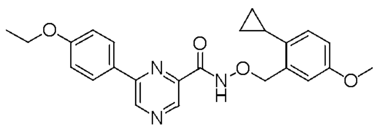 *N*-((2-cyclopropyl-5-methoxybenzyl)oxy)-6-(4-ethoxyphenyl)pyrazine-2-carboxamide | $^1$H-NMR, 400 MHz, DMSO-$d_6$ $\delta$ = 12.31 (br s, 1H), 9.42 (s, 1H), 9.00 (s, 1H), 8.36 (d, J = 8.9 Hz, 2H), 7.14 - 7.01 (m, 3H), 6.97 - 6.92 (m, 1H), 6.84 (dd, J = 2.8, 8.5 Hz, 1H), 5.16 (s, 2H), 4.14 (q, J = 7.0 Hz, 2H), 3.74 (s, 3H), 2.36 - 2.22 (m, 1H), 1.37 (t, J = 6.9 Hz, 3H), 0.96 - 0.85 (m, 2H), 0.66 - 0.52 (m, 2H). [M+H]$^+$: 420.4. |

(continued)

| Compound № | Structure | $^1$H-NMR / MS(m/z) $[M+H]^+$ |
|---|---|---|
| 16 | 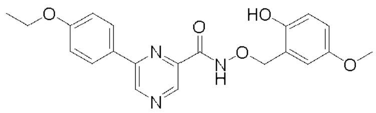 6-(4-ethoxyphenyl)-*N*-((2-hydroxy-5-methoxybenzyl)oxy) pyrazine-2-carboxamide | $^1$H-NMR, 400 MHz, DMSO-$d_6$ $\delta$ = 12.83 - 11.99 (m, 1H), 9.77 - 8.89 (m, 2H), 8.37 (d, J = 8.9 Hz, 2H), 7.18 - 6.94 (m, 3H), 6.79 (s, 2H), 4.98 (s, 2H), 4.14 (q, J = 7.0 Hz, 2H), 3.70 (s, 3H), 1.38 (t, J = 6.9 Hz, 3H). $[M+H]^+$: 396.1. |
| 17 | 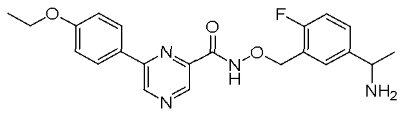 *N*-((5-(1-aminoethyl)-2-fluorobenzyl)oxy)-6-(4-ethoxyphenyl)pyrazine-2-carboxamide | |
| 18 | 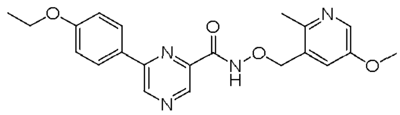 6-(4-ethoxyphenyl)-*N*-((5-methoxy-2-methylpyridine-3-yl)methoxy)pyrazine-2-carboxamide | |
| 19 | 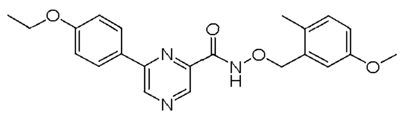 6-(4-ethoxyphenyl)-*N*-((5-methoxy-2-methylbenzyl)oxy)pyrazine-2-carboxamide | |
| 20 | 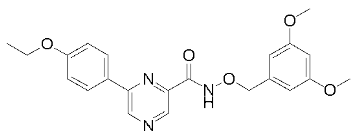 *N*-((3,5-dimethoxybenzyl)oxy)-6-(4-ethoxyphenyl)pyrazine-2-carboxamide | $^1$H-NMR, 400 MHz, DMSO-$d_6$ $\delta$ = 12.27 (s, 1H), 9.42 (s, 1H), 8.98 (s, 1H), 8.35 (d, J = 8.9 Hz, 2H), 7.19 - 6.93 (m, 2H), 6.67 (d, J = 2.3 Hz, 2H), 6.48 (t, J = 2.2 Hz, 1H), 4.94 (s, 2H), 4.14 (q, J = 6.9 Hz, 2H), 3.75 (s, 6H), 1.37 (t, J = 7.0 Hz, 3H). $[M+H]^+$: 410.1. |
| 21 | 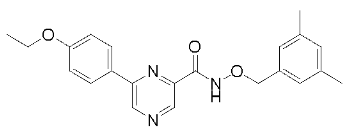 *N*-((3,5-dimethylbenzyl)oxy)-6-(4-ethoxyphenyl)pyrazine-2-carboxamide | $^1$H-NMR, 400 MHz, DMSO-$d_6$ $\delta$ = 12.27 (s, 1H), 9.42 (s, 1H), 8.99 (s, 1H), 8.36 (d, J = 8.8 Hz, 2H), 7.13 - 7.06 (m, 4H), 7.00 (s, 1H), 4.91 (s, 2H), 4.14 (q, J = 7.0 Hz, 2H), 2.29 (s, 6H), 1.37 (t, J = 7.0 Hz, 3H). $[M+H]^+$: 378.1. |
| 22 | 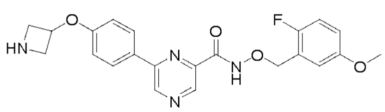 6-(4-(azetidine-3-yloxy)phenyl)-*N*-((2-fluoro-5-methoxybenzyl)oxy)pyrazine-2-carboxamide | $^1$H-NMR, 400 MHz, DMSO-$d_6$ $\delta$ = 9.42 (s, 1H), 8.99 (s, 1H), 8.35 (d, J = 9.20 Hz, 2H), 7.20-7.14 (m, 2H), 7.00-6.96 (m, 3H), 5.12 (t, J = 6.00 Hz, 1H), 5.04 (s, 2H), 3.93-3.89 (m, 2H), 3.76-3.75 (m, 3H), 3.63-3.59 (m, 2H). $[M+H]^+$: 425.2. |
| 23 | 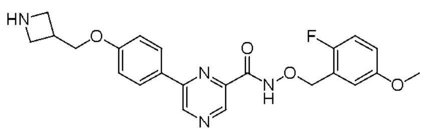 6-(4-(azetidine-3-ylmethoxy)phenyl)-*N*-((2-fluoro-5-methoxybenzyl)oxy)pyrazine-2-carboxamide | $^1$H-NMR, 400 MHz, DMSO-$d_6$ $\delta$ = 9.44 (s, 1H), 9.00 (s, 1H), 8.40-8.38 (m, 2H), 7.20-7.14 (m, 4H), 7.00-6.96 (m, 1H), 5.05 (s, 2H), 4.25-4.24 (m, 2H), 4.03 (t, J = 9.20 Hz, 2H), 3.84-3.76 (m, 5H), 3.26-3.21 (m, 1H). $[M+H]^+$: 439.2. |

(continued)

| Compound № | Structure | $^1$H-NMR / MS(m/z) [M+H]$^+$ |
|---|---|---|
| 24 | 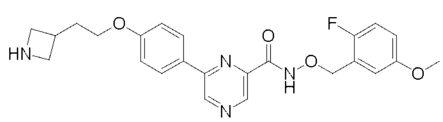 6-(4-(2-(azetidine-3-yl)ethoxy)phenyl)-*N*-((2-fluoro-5-methoxybenzyl)oxy)pyrazine-2-carboxamide | $^1$H-NMR, 400 MHz, DMSO-$d_6$ $\delta$ = 12.30 (s, 1H), 9.43 (s, 1H), 8.61-8.49 (m, 2H), 8.38-8.35 (m, 2H), 7.20-7.15 (m, 2H), 7.09-7.07 (m, 2H), 7.00-6.97 (m, 1H), 5.05 (s, 1H), 4.11-4.01 (m, 4H), 3.82-3.80 (m, 5H), 3.04-2.96 (m, 1H), 2.10-2.06 (m, 2H), 1.51 (s, 1H). [M+H]$^+$: 453.2. |
| 25 | 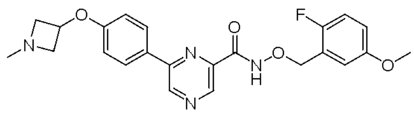 *N*-((2-fluoro-5-methoxybenzyl)oxy)-6-(4-((1-methylazetidine-3-yl)oxy)phenyl)pyrazine-2-carboxamide | $^1$H-NMR, 400 MHz, DMSO-$d_6$ $\delta$ = 11.68 (s, 1H), 9.41 (s, 1H), 8.98 (s, 1H), 8.35 (d, J = 8.0 Hz, 2H), 7.20 to 7.14 (m, 2H), 7.00 to 6.95 (m, 3H), 5.04 (s, 2H), 4.92 to 4.86 (m, 1H), 3.79 to 3.76 (m, 5H), 3.02 to 2.99 (m, 2H), 2.31 (s, 3H), 1.90 (s, 3H). [M+H]$^+$: 439.2. |
| 26 | 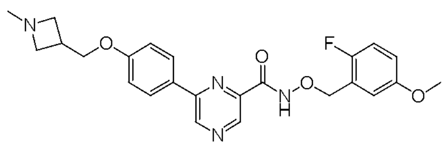 *N*-((2-fluoro-5-methoxybenzyl)oxy)-6-(4-((1-methylazetidine-3-yl)methoxy)phenyl)pyrazine-2-carboxamide | |
| 27 | 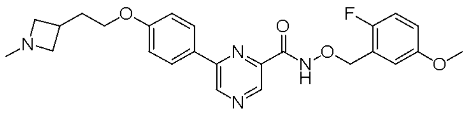 *N*-((2-fluoro-5-methoxybenzyl)oxy)-6-(4-(2-(1-methylazetidine-3-yl)ethoxy)phenyl)pyrazine-2-carboxamide | |
| 28 | 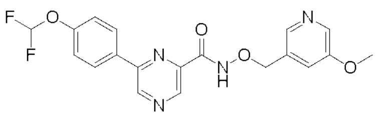 6-(4-(difluoromethoxy)phenyl)-*N*-((5-methoxypyridine-3-yl) methoxy)pyrazine-2-carboxamide | $^1$H-NMR, 400 MHz, DMSO-$d_6$ $\delta$ = 12.38 (s, 1H), 9.50 (s, 1H), 9.07 (s, 1H), 8.48 (d, J = 8.9 Hz, 2H), 8.30 (d, J = 3.3 Hz, 2H), 7.66 - 7.18 (m, 4H), 5.07 (s, 2H), 3.86 (s, 3H). [M+H]$^+$: 403.4. |
| 29 | 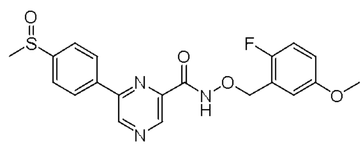 *N*-((2-fluoro-5-methoxybenzyl)oxy)-6-(4-(methylsulfinyl) phenyl)py razine-2-carboxamide | $^1$H-NMR, 400 MHz, DMSO-$d_6$ $\delta$ = 12.40 (s, 1H), 9.57 (s, 1H), 9.13 (s, 1H), 8.60 (d, J = 8.40 Hz, 2H), 7.89-7.86 (m, 2H), 7.21-7.00 (m, 2H), 6.99-6.97 (m, 1H), 5.06 (s, 2H), 3.76 (s, 3H), 2.83 (s, 3H). [M+H]$^+$: 416.2. |
| 30 | 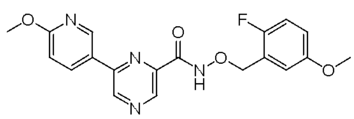 *N*-((2-fluoro-5-methoxybenzyl)oxy)-6-(6-methoxypyridine-3-yl)pyrazine-2-carboxamide | $^1$H-NMR, 400 MHz, DMSO-$d_6$ $\delta$ = 12.35 (s, 1H), 9.50 (s, 1H), 9.22 (d, J = 2.00 Hz, 1H), 9.05 (s, 1H), 8.74 to 8.71 (m, 1H), 7.20 to 7.14 (m, 2H), 7.03 to 6.98 (m, 2H), 5.04 (s, 2H), 3.96 (s, 3H), 0.75 (s, 3H). [M+H]$^+$: 385.0. |

(continued)

| Compound № | Structure | $^1$H-NMR / MS(m/z) [M+H]$^+$ |
|---|---|---|
| 31 | 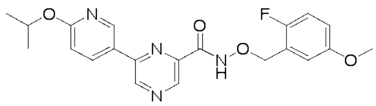 *N*-((2-fluoro-5-methoxybenzyl)oxy)-6-(6-isopropoxypyridine-3-yl)pyrazine-2-carboxamide | $^1$H-NMR, 400 MHz, DMSO-$d_6$ $\delta$ = 12.34 (s, 1H), 9.48 (s, 1H), 9.21-9.20 (m, 1H), 9.04 (s, 1H), 8.72-8.69 (m, 1H), 7.20-7.14 (m, 2H), 6.99-6.94 (m, 2H), 5.40-5.31 (m, 1H), 5.04 (s, 2H), 3.76 (s, 3H), 1.36-1.34 (m, 6H). [M+H]$^+$: 413.1. |
| 32 | 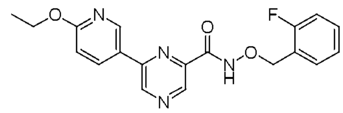 6-(6-ethoxypyridine-3-yl)-*N*-((2-fluorobenzyl)oxy)pyrazine -2-carboxamide | $^1$H-NMR, 400 MHz, DMSO-$d_6$ $\delta$ = 12.37 (s, 1H), 9.49 (s, 1H), 9.21 (d, J = 2.00 Hz, 1H), 9.04 (s, 1H), 8.74-8.71 (m, 1H), 7.62-7.57 (m, 1H), 7.47-7.43 (m, 1H), 7.28-7.24 (m, 2H), 7.00-6.98 (m, 1H), 5.07 (s, 2H), 4.44-4.38 (m, 2H), 1.38-1.35 (m, 3H). [M+H]$^+$: 369.4. |
| 33 | 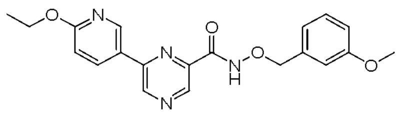 6-(6-ethoxypyridine-3-yl)-*N*-((3-methoxybenzyl)oxy)pyrazin e-2-carboxamide | $^1$H-NMR, 400 MHz, DMSO-$d_6$ $\delta$ = 12.33 (s, 1H), 9.49 (s, 1H), 9.21-9.20 (m, 1H), 9.05 (s, 1H), 8.74-8.71 (m, 1H), 7.35-7.31 (m, 1H), 7.09-7.06 (m, 2H), 7.00-6.95 (m, 2H), 4.99 (s, 2H), 4.41 (q, J = 6.80 Hz, 2H), 3.78 (s, 3H), 1.37 (t, J = 6.80 Hz, 3H). [M+H]$^+$: 381.1. |
| 34 | 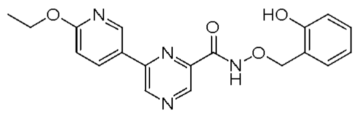 6-(6-ethoxypyridine-3-yl)-*N*-((2-hydroxybenzyl)oxy)pyrazine-2-carboxamide | |
| 35 | 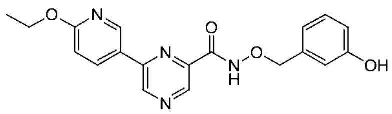 6-(6-ethoxypyridine-3-yl)-*N*-((3-hydroxybenzyl)oxy)pyrazine-2-carboxamide | |
| 36 | 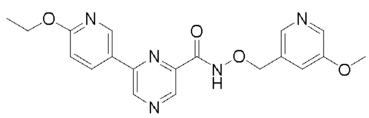 6-(6-ethoxypyridine-3-yl)-*N*-((5-methoxypyridine-3-yl)methoxy)pyrazine-2-carboxamide | $^1$H-NMR, 400 MHz, DMSO-$d_6$ $\delta$ = 1.36 (t, J=7.07 Hz, 3 H) 3.85 (s, 3 H) 4.40 (q, J=7.09 Hz, 2 H) 5.05 (s, 2 H) 6.98 (d, J=8.63 Hz, 1 H) 7.53 (dd, J=2.44, 1.81 Hz, 1 H) 8.28 (dd, J=3.75, 2.25 Hz, 2 H) 8.70 (dd, J=8.75, 2.50 Hz, 1 H) 9.04 (s, 1 H) 9.19 (d, J=2.25 Hz, 1 H) 9.48 (s, 1 H) 12.36 (s, 1 H). [M+H]$^+$: 382.4. |
| 37 | 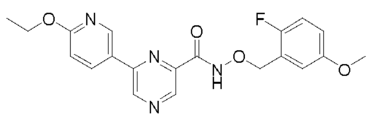 6-(6-ethoxypyridine-3-yl)-*N*-((2-fluoro-5-methoxybenzyl) oxy)pyrazine -2-carboxamide | $^1$H-NMR, 400 MHz, DMSO-$d_6$ $\delta$ = 12.36 (s, 1H), 9.40 (s, 1H), 9.15 (d, J = 2.40 Hz, 1H), 9.00 (s, 1H), 8.66 (dd, J = 2.40, 8.60 Hz, 1H), 7.18 to 7.13 (m, 2H), 6.98 to 6.93 (m, 2H), 5.01 (s, 2H), 4.40 (q, J = 6.80 Hz, 2H), 3.75 (s, 3H), 1.36 (t, J = 7.20 Hz, 3H). [M+H]$^+$: 398.5. |

(continued)

| Compound № | Structure | $^1$H-NMR / MS(m/z) [M+H]$^+$ |
|---|---|---|
| 38 | 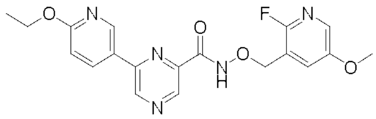 6-(6-ethoxypyridine-3-yl)-*N*-((2-fluoro-5-methoxypyridine-3-yl)methoxy)pyrazine-2-carboxamide | $^1$H-NMR, 400 MHz, DMSO-$d_6$ $\delta$ = 12.36 (br s, 1H), 9.49 (s, 1H), 9.19 (br d, J = 1.6 Hz, 1H), 9.03 (s, 1H), 8.70 (dd, J = 2.2, 8.7 Hz, 1H), 7.91 (br s, 1H), 7.78 (dd, J = 2.8, 7.7 Hz, 1H), 6.99 (d, J = 8.8 Hz, 1H), 5.05 (s, 2H), 4.41 (q, J = 7.0 Hz, 2H), 3.84 (s, 3H), 1.37 (t, J = 7.0 Hz, 3H). [M+H]$^+$: 400.1. |
| 39 | 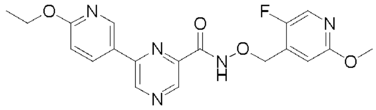 6-(6-ethoxypyridine-3-yl)-*N*-((5-fluoro-2-methoxypyridine-4-yl)methoxy)pyrazine-2-carboxamide | $^1$H-NMR, 400 MHz, DMSO-$d_6$ $\delta$ = 12.41 (s, 1H), 9.50 (s, 1H), 9.20 (d, J = 2.2 Hz, 1H), 9.04 (s, 1H), 8.70 (dd, J = 2.4, 8.7 Hz, 1H), 8.18 (s, 1H), 7.10 (d, J = 4.8 Hz, 1H), 6.99 (d, J = 8.8 Hz, 1H), 5.11 (s, 2H), 4.41 (q, J = 6.9 Hz, 2H), 3.85 (s, 3H), 1.37 (t, J = 7.0 Hz, 3H). [M+H]$^+$: 400.1. |
| 40 | 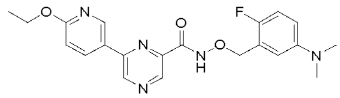 *N*-((5-(dimethylamino)-2-fluorobenzyl)oxy)-6-(6-ethoxypyridine-3-yl)pyrazine-2-carboxamide | $^1$H-NMR, 400 MHz, DMSO-$d_6$ $\delta$ = 12.32 (s, 1H), 9.49 (s, 1H), 9.21 (s, 1H), 9.04 (s, 1H), 8.74-8.71 (m, 1H), 7.00-6.91 (m, 3H), 6.90-6.89 (m, 1H), 5.02 (s, 2H), 4.42 (q, J = 7.20 Hz, 2H), 2.87 (s, 6H), 1.37 (t, J = 6.80 Hz, 3H) . [M+H]$^+$: 412.2. |
| 41 | 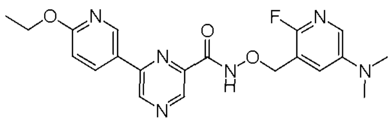 *N*-((5-(dimethylamino)-2-fluoropyridine-3-yl)methoxy)-6-(6-ethoxypyridine-3-yl)pyrazine-2-carboxamide | $^1$H-NMR, 400 MHz, DMSO-$d_6$ $\delta$ = 12.32 (s, 1H), 9.49 (s, 1H), 9.19 (d, J = 2.40 Hz, 1H), 9.03 (s, 1H), 8.72-8.69 (m, 1H), 7.62-7.61 (m, 1H), 7.51-7.48 (m, 1H), 6.98 (d, J = 8.40 Hz, 1H), 5.02 (s, 2H), 4.41 (q, J = 7.20 Hz, 2H), 2.89 (s, 6H), 1.37 (t, J = 7.20 Hz, 3H). [M+H]$^+$: 413.2. |
| 42 | 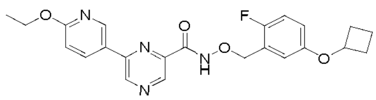 *N*-((5-cyclobutoxy-2-fluorobenzyl)oxy)-6-(6-ethoxypyridine-3-yl)pyrazine-2-carboxamide | $^1$H-NMR, 400 MHz, DMSO-$d_6$ $\delta$ = 12.33 (s, 1H), 9.49 (s, 1H), 9.21-9.20 (m, 1H), 9.05 (s, 1H), 8.74-8.71 (m, 1H), 7.35-7.31 (m, 1H), 7.09-7.06 (m, 2H), 7.00-6.95 (m, 2H), 4.99 (s, 2H), 4.41 (q, J = 6.80 Hz, 2H), 3.78 (s, 3H), 1.37 (t, J = 6.80 Hz, 3H). [M+H]$^+$: 381.1. |
| 43 | 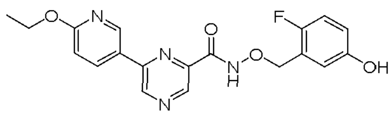 6-(6-ethoxypyridine-3-yl)-*N*-((2-fluoro-5-hydroxybenzyl)oxy)pyrazine-2-carboxamide | |
| 44 | 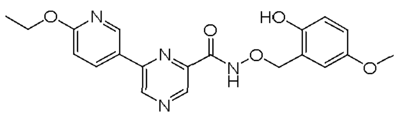 6-(6-ethoxypyridine-3-yl)-*N*-((2-hydroxy-5-methoxybenzyl)oxy)pyrazine-2-carboxamide | |

(continued)

| Compound No | Structure | $^1$H-NMR / MS(m/z) [M+H]$^+$ |
|---|---|---|
| 45 | 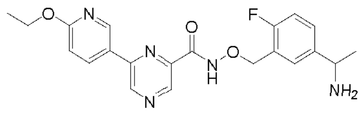 *N*-((5-(1-aminoethyl)-2-fluorobenzyl)oxy)-6-(6-ethoxypyridine-3-yl)pyrazine-2-carboxamide | |
| 46 | 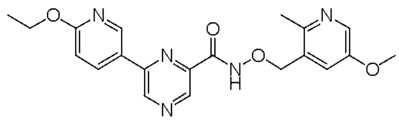 6-(6-ethoxypyridine-3-yl)-*N*-((5-methoxy-2-methylpyridine-3-yl)methoxy)pyrazine-2-carboxamide | |
| 47 | 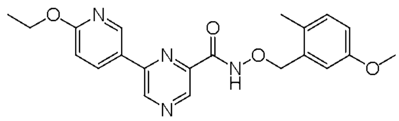 6-(6-ethoxypyridine-3-yl)-*N*-((5-methoxy-2-methylbenzyl)oxy)pyrazine-2-carboxamide | |
| 48 | 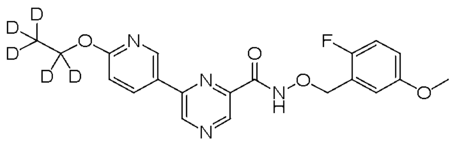 6-(6-(ethoxy-$D_5$)pyridine-3-yl)-*N*-((2-fluoro-5-methoxybenzyl)oxy)pyrazin e-2-carboxamide | $^1$H-NMR, 400 MHz, DMSO-$d_6$ $\delta$ = 12.41 - 12.28 (m, 1H), 9.54 - 8.97 (m, 3H), 8.77 - 8.64 (m, 1H), 7.24 - 7.08 (m, 2H), 7.01 - 6.90 (m, 2H), 5.04 (s, 2H), 3.81 - 3.66 (m, 3H). [M+H]$^+$: 404.1. |
| 49 | 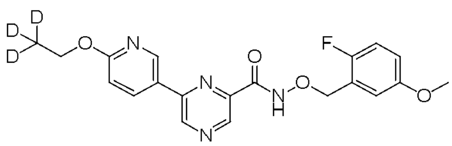 6-(6-(ethoxy-2,2,2-$d_3$)pyridine-3-yl)-N-((2-fluoro-5-methoxybenzyl)oxy)pyrazin e-2-carboxamide | $^1$H-NMR, 400 MHz, DMSO-$d_6$ $\delta$ = 12.34 (s, 1H), 9.48 (s, 1H), 9.20 (d, J = 2.3 Hz, 1H), 9.04 (s, 1H), 8.71 (dd, J = 2.5, 8.8 Hz, 1H), 7.21 - 7.10 (m, 2H), 7.02 - 6.90 (m, 2H), 5.04 (s, 2H), 4.39 (s, 2H), 3.75 (s, 3H). [M+H]$^+$: 402.0. |
| 50 | 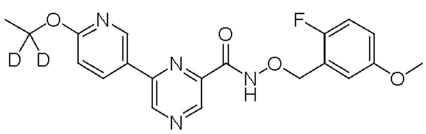 6-(6-(ethoxy-1,1-$d_2$)pyridine-3-yl)-*N*-((2-fluoro-5-methoxybenzyl)oxy)pyrazin e-2-carboxamide | $^1$H-NMR, 400 MHz, DMSO-$d_6$ $\delta$ = 12.34 (s, 1H), 9.48 (s, 1H), 9.20 (d, J = 2.1 Hz, 1H), 9.04 (s, 1H), 8.71 (dd, J = 2.5, 8.8 Hz, 1H), 7.21 - 7.10 (m, 2H), 6.97 (br d, J = 8.8 Hz, 2H), 5.04 (s, 2H), 3.75 (s, 3H), 1.34 (s, 3H). [M+H]$^+$: 401.0. |
| 51 | 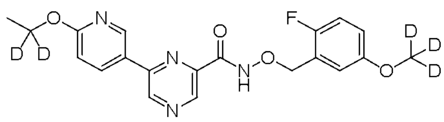 6-(6-(ethoxy-1,1-$d_2$)pyridine-3-yl)-*N*-((2-fluoro-5-(methoxy-$d_3$)benzyl)oxy)pyrazine-2-carboxamide | $^1$H-NMR, 400 MHz, DMSO-$d_6$ $\delta$ = 12.34 (s, 1H), 9.48 (s, 1H), 9.20 (d, J = 2.3 Hz, 1H), 9.04 (s, 1H), 8.71 (dd, J = 2.4, 8.8 Hz, 1H), 7.21 - 7.11 (m, 2H), 7.00 - 6.93 (m, 2H), 5.04 (s, 2H), 1.34 (s, 3H). [M+H]$^+$: 404.1. |
| 52 | 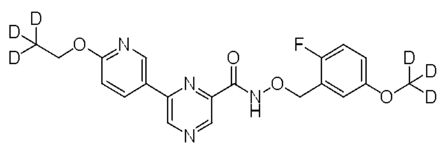 6-(6-(ethoxy-2,2,2-$d_3$)pyridine-3-yl)-*N*-((2-fluoro-5-(methoxy-$d_3$)benzyl)oxy)pyrazine-2-carboxamide | $^1$H-NMR, 400 MHz, DMSO-$d_6$ $\delta$ = 12.34 (s, 1H), 9.48 (s, 1H), 9.19 (d, J = 2.3 Hz, 1H), 9.04 (s, 1H), 8.70 (dd, J = 2.5, 8.8 Hz, 1H), 7.22 - 7.10 (m, 2H), 7.01 - 6.91 (m, 2H), 5.03 (s, 2H), 4.39 (s, 2H). [M+H]$^+$: 405.0. |

(continued)

| Compound № | Structure | $^1$H-NMR / MS(m/z) [M+H]$^+$ |
|---|---|---|
| 53 | 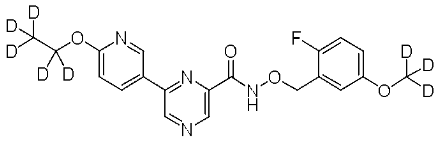 6-(6-(ethoxy-$d_5$)pyridine-3-yl)-*N*-((2-fluoro-5-(methoxy-$d_3$) benzyl)oxy)pyrazine-2-carboxamide | $^1$H-NMR, 400 MHz, DMSO-d$_6$ $\delta$ = 12.35 (s, 1H), 9.57 - 8.95 (m, 3H), 8.76 - 8.63 (m, 1H), 7.26 - 7.08 (m, 2H), 7.05 - 6.93 (m, 2H), 5.05 (s, 2H). [M+H]$^+$: 407.5. |
| 54 | 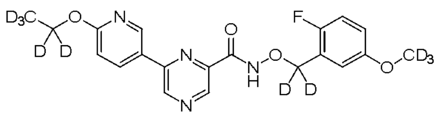 6-(6-(ethoxy-$d_5$)pyridine-3-yl)-*N*-((2-fluoro-5-(methoxy-$d_3$) phenyl)methoxy-$d_2$)pyrazine-2-carboxamide | $^1$H-NMR, 400 MHz, DMSO-d$_6$ $\delta$ = 12.33 (s, 1H), 9.48 (s, 1H), 9.20 (s, 1H), 9.07 - 9.01 (m, 1H), 8.71 (dd, J = 2.4, 8.6 Hz, 1H), 7.23 - 7.09 (m, 2H), 7.02 - 6.92 (m, 2H). [M+H]$^+$: 409.5. |
| 55 | 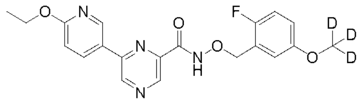 6-(6-ethoxypyridine-3-yl)-*N*-((2-fluoro-5-(methoxy-$d_3$)benzyl)oxy)pyrazine-2-carboxamide | $^1$H-NMR, 400 MHz, DMSO-d$_6$ $\delta$ = 12.34 (s, 1H), 9.48 (s, 1H), 9.20 (d, J = 2.4 Hz, 1H), 9.04 (s, 1H), 8.71 (dd, J = 2.5, 8.8 Hz, 1H), 7.20 - 7.12 (m, 2H), 6.99 - 6.93 (m, 2H), 5.04 (s, 2H), 4.40 (q, J = 7.0 Hz, 2H), 1.36 (t, J = 7.0 Hz, 3H). [M+H]$^+$: 402.0. |
| 56 | 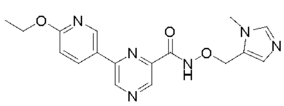 6-(6-ethoxypyridine-3-yl)-*N*-((1-methyl-1*H*-midazole-5-yl) methoxy)pyrazine-2-carboxamide | $^1$H-NMR, 400 MHz, DMSO-d$_6$ $\delta$ = 12.27 (s, 1H), 9.49 (s, 1H), 9.20 (d, J = 2.4 Hz, 1H), 9.04 (s, 1H), 8.72 (dd, J = 2.4, 8.7 Hz, 1H), 7.68 (s, 1H), 7.04 - 6.93 (m, 2H), 4.98 (s, 2H), 4.41 (q, J = 7.1 Hz, 2H), 3.83 (s, 3H), 1.37 (t, J = 7.1 Hz, 3H). [M+H]$^+$: 355.0. |
| 57 | 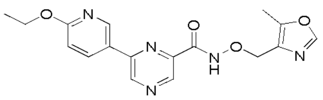 6-(6-ethoxypyridine-3-yl)-*N*-((5-methyloxazole-4-yl)methoxy)pyrazine-2-carboxamide | $^1$H-NMR, 400 MHz, DMSO-d$_6$ $\delta$ = 12.27 (s, 1H), 9.48 (s, 1H), 9.20 (d, J = 2.4 Hz, 1H), 9.04 (s, 1H), 8.73 (dd, J = 2.5, 8.8 Hz, 1H), 8.23 (s, 1H), 6.98 (d, J = 8.8 Hz, 1H), 4.87 (s, 2H), 4.41 (q, J = 7.0 Hz, 2H), 2.36 (s, 3H), 1.36 (t, J = 7.0 Hz, 3H). [M+H]$^+$: 356.0. |
| 58 | 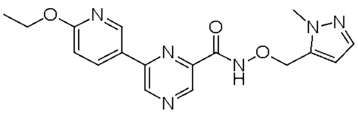 6-(6-ethoxypyridine-3-yl)-*N*-((1-methyl-1*H*-pyrazole-5-yl) methoxy)pyrazine-2-carboxamide | $^1$H-NMR, 400 MHz, DMSO-d$_6$ $\delta$ = 12.37 (s, 1H), 9.49 (s, 1H), 9.20 (d, J = 2.3 Hz, 1H), 9.05 (s, 1H), 8.71 (dd, J = 2.4, 8.7 Hz, 1H), 7.38 (d, J = 1.8 Hz, 1H), 6.99 (d, J = 8.8 Hz, 1H), 6.37 (d, J = 1.8 Hz, 1H), 5.05 (s, 2H), 4.41 (q, J = 7.0 Hz, 2H), 4.01 (s, 3H), 1.36 (t, J = 7.0 Hz, 3H). [M+H]$^+$: 355.4. |

(continued)

| Compound Nº | Structure | $^1$H-NMR / MS(m/z) [M+H]$^+$ |
|---|---|---|
| 59 | 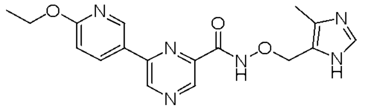 6-(6-ethoxypyridine-3-yl)-*N*-((4-methyl-1*H*-imidazole-5-yl) methoxy)pyrazine-2-carboxamide | $^1$H-NMR, 400 MHz, DMSO-$d_6$ $\delta$ = 13.73 (br d, J = 5.0 Hz, 1H), 12.25 (br s, 1H), 9.49 (s, 1H), 9.19 (d, J = 2.3 Hz, 1H), 9.02 (s, 1H), 8.82 - 8.62 (m, 2H), 6.99 (d, J = 8.8 Hz, 1H), 4.99 (s, 2H), 4.41 (q, J = 7.0 Hz, 2H), 2.28 (s, 3H), 1.36 (t, J = 7.1 Hz, 3H). [M+H]$^+$: 355.4. |
| 60 | 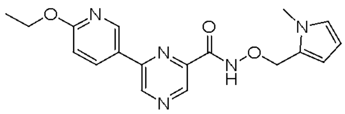 6-(6-ethoxypyridine-3-yl)-*N*-((1-methyl-1*H*-pyrrole-2-yl)methoxy)pyrazine-2-carboxamide | |
| 61 | 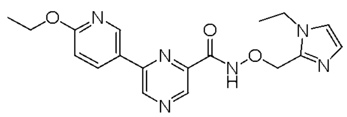 6-(6-ethoxypyridine-3-yl)-*N*-((1-ethyl-1*H*-imidazole-2-yl) methoxy)pyrazine-2-carboxamide | $^1$H-NMR, 400 MHz, DMSO-$d_6$ $\delta$ = 12.62 - 12.17 (m, 1H), 9.50 (s, 1H), 9.21 (d, J = 2.1 Hz, 1H), 9.06 (s, 1H), 8.73 (dd, J = 2.4, 8.7 Hz, 1H), 7.31 (s, 1H), 6.99 (d, J = 8.8 Hz, 1H), 6.90 (s, 1H), 5.02 (s, 2H), 4.41 (q, J = 7.1 Hz, 2H), 4.28 (q, J = 7.3 Hz, 2H), 1.39 (td, J = 7.1, 18.3 Hz, 6H). [M+H]$^+$: 369.1. |
| 62 | 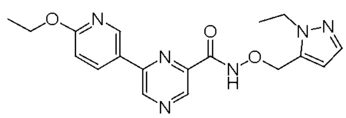 6-(6-ethoxypyridine-3-yl)-*N*-((1-ethyl-1*H*-pyrazole-5-yl) methoxy)pyrazine-2-carboxamide | $^1$H-NMR, 400 MHz, DMSO-$d_6$ $\delta$ = 12.40 (s, 1H), 9.50 (s, 1H), 9.21 (d, J = 2.4 Hz, 1H), 9.06 (s, 1H), 8.72 (dd, J = 2.5, 8.8 Hz, 1H), 7.43 (d, J = 1.8 Hz, 1H), 6.99 (d, J = 8.8 Hz, 1H), 6.37 (d, J = 1.8 Hz, 1H), 5.04 (s, 2H), 4.40 (qd, J = 7.2, 9.6 Hz, 4H), 1.39 (td, J = 7.1, 19.0 Hz, 6H). [M+H]$^+$: 369.0. |
| 63 | 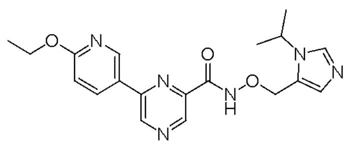 6-(6-ethoxypyridine-3-yl)-*N*-((1-isopropyl-1*H*-imidazole-5-yl)methoxy)pyrazine-2-carboxamide | $^1$H-NMR, 400 MHz, DMSO-$d_6$ $\delta$ = 12.32 (s, 1H), 9.50 (s, 1H), 9.21 (d, J = 2.3 Hz, 1H), 9.06 (s, 1H), 8.72 (dd, J = 2.5, 8.8 Hz, 1H), 7.90 (d, J = 0.6 Hz, 1H), 7.01 - 6.98 (m, 2H), 4.97 (s, 2H), 4.86 (td, J = 6.7, 13.4 Hz, 1H), 4.41 (q, J = 7.0 Hz, 2H), 1.50 (d, J = 6.8 Hz, 6H), 1.37 (t, J = 7.1 Hz, 3H). [M+H]$^+$: 383.0. |
| 64 | 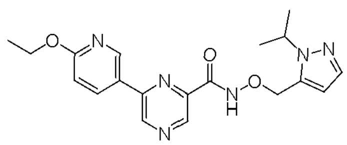 6-(6-ethoxypyridine-3-yl)-*N*-((1-isopropyl-1*H*-pyrazole-5-yl) methoxy)pyrazine-2-carboxamide | $^1$H-NMR, 400 MHz, DMSO-$d_6$ $\delta$ = 12.41 (s, 1H), 9.50 (s, 1H), 9.21 (d, J = 2.3 Hz, 1H), 9.07 (s, 1H), 8.72 (dd, J = 2.4, 8.7 Hz, 1H), 7.45 (d, J = 1.4 Hz, 1H), 6.99 (d, J = 8.8 Hz, 1H), 6.35 (d, J = 1.6 Hz, 1H), 5.17 - 4.96 (m, 3H), 4.41 (q, J = 7.0 Hz, 2H), 1.44 (d, J = 6.5 Hz, 6H), 1.36 (t, J = 7.0 Hz, 3H). [M+H]$^+$: 383.1. |

(continued)

| Compound № | Structure | $^1$H-NMR / MS(m/z) [M+H]$^+$ |
|---|---|---|
| 65 | 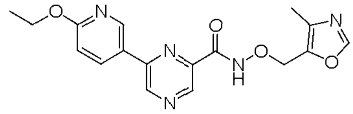 6-(6-ethoxypyridine-3-yl)-*N*-((4-methyloxazole-5-yl)methoxy)pyrazine-2-carboxamide | $^1$H-NMR, 400 MHz, DMSO-$d_6$ $\delta$ = 12.28 (s, 1H), 9.48 (s, 1H), 9.19 (d, J = 2.4 Hz, 1H), 9.02 (s, 1H), 8.70 (dd, J = 2.5, 8.8 Hz, 1H), 8.31 (s, 1H), 6.98 (d, J = 8.8 Hz, 1H), 5.03 (s, 2H), 4.40 (q, J = 7.0 Hz, 2H), 2.11 (s, 3H), 1.36 (t, J = 7.0 Hz, 3H). [M+H]$^+$: 356.4. |
| 66 | 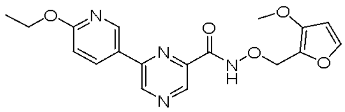 6-(6-ethoxypyridine-3-yl)-*N*-((3-methoxyfuran-2-yl)methoxy)pyrazine-2-carboxamide | |
| 67 | 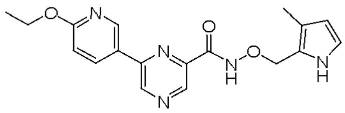 6-(6-ethoxypyridine-3-yl)-*N*-((3-methyl-1*H*-pyrrole-2-yl)methoxy)pyrazine-2-carboxamide | |
| 68 | 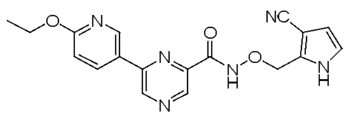 N-((3-cyan-1*H*-pyrrole-2-yl)methoxy)-6-(6-ethoxypyridine-3-yl)pyrazine-2-carboxamide | $^1$H-NMR, 400 MHz, DMSO-$d_6$ $\delta$ = 12.30 (br s, 1H), 12.11 (br s, 1H), 9.47 (s, 1H), 9.19 (d, J = 2.1 Hz, 1H), 9.03 (s, 1H), 8.70 (dd, J = 2.3, 8.7 Hz, 1H), 7.00 - 6.94 (m, 2H), 6.47 (d, J = 2.6 Hz, 1H), 5.01 (s, 2H), 4.40 (q, J = 7.0 Hz, 2H), 1.36 (t, J = 7.1 Hz, 3H). [M+H]$^+$: 365.4. |
| 69 | 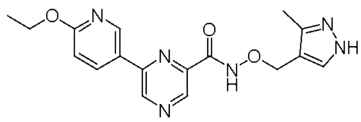 6-(6-ethoxypyridine-3-yl)-*N*-((3-methyl-1*H*-pyrazole-4-yl) methoxy)pyrazine-2-carboxamide | $^1$H-NMR, 400 MHz, DMSO-$d_6$ $\delta$ = 12.18 (s, 1H), 9.48 (s, 1H), 9.21 (d, J = 2.0 Hz, 1H), 9.05 (s, 1H), 8.73 (dd, J = 2.5, 8.8 Hz, 1H), 7.59 (s, 1H), 6.99 (d, J = 9.3 Hz, 1H), 4.84 (s, 2H), 4.41 (q, J = 7.1 Hz, 3H), 2.31 (s, 3H), 1.37 (t, J = 7.1 Hz, 3H). [M+H]$^+$: 355.4. |
| 70 | 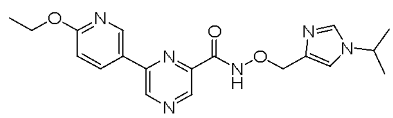 6-(6-ethoxypyridine-3-yl)-*N*-((1-isopropyl-1*H*-imidazole-4-yl)methoxy)pyrazine-2-carboxamide | $^1$H-NMR, 400 MHz, DMSO-$d_6$ $\delta$ = 12.25 (br s, 1H), 9.48 (s, 1H), 9.21 (d, J = 2.3 Hz, 1H), 9.04 (s, 1H), 8.75 (dd, J = 2.5, 8.8 Hz, 1H), 7.71 (d, J = 1.0 Hz, 1H), 7.39 (d, J = 0.8 Hz, 1H), 6.97 (d, J = 8.8 Hz, 1H), 4.84 (s, 2H), 4.49 - 4.31 (m, 3H), 1.42 - 1.33 (m, 9H). [M+H]$^+$: 383.1. |
| 71 | 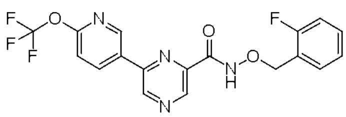 *N*-((2-fluorobenzyl)oxy)-6-(6-(trifluoromethoxy)pyridin e-3-yl)pyrazine-2-carboxamide | $^1$H-NMR, 400 MHz, DMSO-$d_6$ $\delta$ = 12.42 (s, 1H), 9.60 (s, 1H), 9.40 (d, J = 2.00 Hz, 1H), 9.15 (s, 1H), 9.02 (dd, J = 2.40, 8.40 Hz, 1H), 7.62-7.54 (m, 1H), 7.51 (d, J = 0.80 Hz, 1H), 7.48-7.46 (m, 1H), 7.45-7.43 (m, 2H), 5.08 (s, 2H), 3.32 (s, 3H). [M+H]$^+$: 409.1. |

(continued)

| Compound № | Structure | $^1$H-NMR / MS(m/z) [M+H]$^+$ |
|---|---|---|
| 72 | 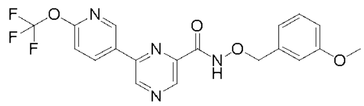 *N*-((3-methoxybenzyl)oxy)-6-(6-(trifluoromethoxy)pyridin e-3-yl)pyrazine-2-carboxamide | $^1$H-NMR, 400 MHz, DMSO-$d_6$ $\delta$ = 12.40 (s, 1H), 9.60 (s, 1H), 9.41 (d, J = 2.40 Hz, 1H), 9.16 (s, 1H), 9.02 (dd, J = 2.40, 8.60 Hz, 1H), 7.53 (d, J = 8.40 Hz, 1H), 7.33 (t, J = 8.00 Hz, 1H), 7.09-7.06 (m, 2H), 6.94 (dd, J = 2.40, 8.00 Hz, 1H), 4.99 (s, 2H), 3.78 (s, 3H). [M+H]$^+$: 421.1. |
| 73 | 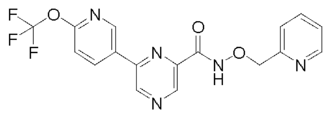 *N*-(pyridine-2-ylmethoxy)-6-(6-(trifluoromethoxy)pyridin e-3-yl)pyrazine-2-carboxamide | $^1$H-NMR, 400 MHz, DMSO-$d_6$ $\delta$ = 12.49 (s, 1H), 9.60 (s, 1H), 9.46 (t, J = 2.00 Hz, 1H), 9.18 (d, J = 10.40 Hz, 1H), 9.02 (dd, J = 2.40, 8.40 Hz, 1H), 8.58 (d, J = 4.00 Hz, 1H), 7.91-7.86 (m, 1H), 7.70-7.68 (m, 1H), 7.53 (d, J = 8.80 Hz, 1H), 7.40 (d, J = 4.80 Hz, 1H), 7.39-7.37 (m, 1H), 5.12 (s, 2H). [M+H]$^+$: 392.0. |
| 74 | 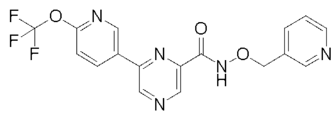 *N*-(pyridine-3-ylmetoxy)-6-(6-(trifluoromethoxy)pyridine -3-yl)pyrazine-2-carboxamide | $^1$H-NMR, 400 MHz, DMSO-$d_6$ $\delta$ = 12.43 (s, 1H), 9.59 (d, J = 9.20 Hz, 1H), 9.40 (d, J = 2.40 Hz, 1H), 9.17 (d, J = 13.60 Hz, 1H), 9.05 to 9.00 (m, 1H), 8.70 (d, J = 1.60 Hz, 1H), 8.58 (dd, J = 1.60, 4.80 Hz, 1H), 7.94 (dd, J = 2.00, Hz, 1H), 7.53 (d, J = 8.80 Hz, 1H), 7.47 to 7.44 (m, 1H), 5.06 (s, 2H). [M+H]$^+$: 392.1. |
| 75 | 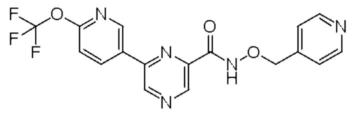 (*N*-(pyridine-4-ylmethoxy)-6-(6-(trifluoromethoxy)pyridine -3-yl)pyrazine-2-carboxamide | $^1$H-NMR, 400 MHz, DMSO-$d_6$ $\delta$ = 12.48 (s, 1H), 9.59 (s, 1H), 9.39 (d, J = 2.40 Hz, 1H), 9.15 (s, 1H), 9.00 (dd, J = 2.40, 8.40 Hz, 1H), 8.61 (dd, J = 1.60, 4.40 Hz, 1H), 7.54-7.51 (m, 3H), 5.09 (s, 2H). [M+H]$^+$: 392.2. |
| 76 | 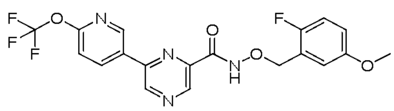 *N*-((2-fluoro-5-methoxybenzyl)oxy)-6-(6-(trifluoromethoxy) pyridine -3-yl)pyrazine-2-carboxamide | $^1$H-NMR, 400 MHz, DMSO-$d_6$ $\delta$ = 12.42 (s, 1H), 9.60 (s, 1H), 9.40 (d, J = 2.40 Hz, 1H), 9.16 (s, 1H), 9.02 (dd, J = 2.40, 8.40 Hz, 1H), 7.53 (d, J = 8.80 Hz, 1H), 7.20-7.14 (m, 2H), 6.99 (m, 1H), 5.05 (s, 2H), 3.76 (s, 3H). [M+H]$^+$: 439.1. |
| 77 | 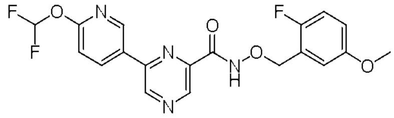 6-(6-(difluoromethoxy)pyridine -3-yl)-*N*-((2-fluoro-5-methoxybenzyl)oxy)pyrazin e-2-carboxamide | $^1$H-NMR, 400 MHz, DMSO-$d_6$ $\delta$ = 12.40 (s, 1H), 9.57 (s, 1H), 9.30 (d, J = 2.00 Hz, 1H), 9.12 (s, 1H), 8.93 (dd, J = 2.40, 8.80 Hz, 1H), 7.83 (t, J = 72.40 Hz, 1H), 7.32 (dd, J = 0.40, 8.60 Hz, 1H), 7.20-7.14 (m, 2H), 7.00-6.97 (m, 1H), 5.05 (s, 2H), 3.76 (s, 3H). [M+H]$^+$: 420.1 |

(continued)

| Compound No | Structure | $^1$H-NMR / MS(m/z) [M+H]$^+$ |
|---|---|---|
| 78 | 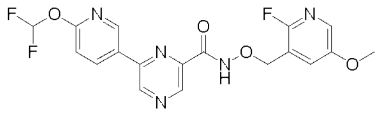 6-(6-(difluoromethoxy)pyridine -3-yl)-*N*-((2-fluoro-5-methoxypyridine-3-yl)methoxy)pyrazine-2-carboxamide | $^1$H-NMR, 400 MHz, DMSO-$d_6$ $\delta$ = 12.42 (br s, 1H), 9.57 (s, 1H), 9.29 (br d, J = 1.8 Hz, 1H), 9.11 (s, 1H), 8.92 (dd, J = 2.3, 8.6 Hz, 1H), 8.06 - 7.62 (m, 2H), 7.32 (d, J = 8.6 Hz, 1H), 5.06 (s, 2H), 3.84 (s, 3H). [M+H]$^+$: 422.1. |
| 79 | 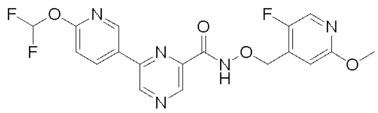 6-(6-(difluoromethoxy)pyridine -3-yl)-*N*-((5-fluoro-2-methoxypyridine-4-yl)methoxy)pyrazine-2-carboxamide | $^1$H-NMR, 400 MHz, DMSO-$d_6$ $\delta$ = 12.46 (br s, 1H), 9.57 (br s, 1H), 9.29 (br s, 1H), 9.12 (br s, 1H), 8.91 (br d, J = 8.2 Hz, 1H), 8.17 (s, 1H), 8.02 - 7.62 (m, 1H), 7.32 (br d, J = 8.6 Hz, 1H), 7.10 (br d, J = 3.2 Hz, 1H), 5.11 (br s, 2H), 3.85 (s, 3H). [M+H]$^+$: 422.0. |
| 80 | 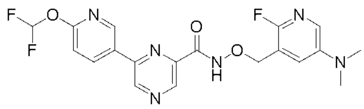 6-(6-(difluoromethoxy)pyridine -3-yl)-*N*-((5-(dimethylamino)-2-fluoropyridine-3-yl)methoxy)pyrazine-2-carboxamide | $^1$H-NMR, 400 MHz, DMSO-$d_6$ $\delta$ = 12.39 (s, 1H), 9.57 (s, 1H), 9.29 (s, 1H), 9.29 (s, 1H), 8.94-8.91 (m, 1H), 8.01 (s, 1H), 7.83 (s, 1H), 7.65-7.62 (m, 1H), 7.51-7.48 (m, 1H), 5.03 (s, 2H), 2.90 (s, 6H). [M+H]$^+$: 435.1. |
| 81 | 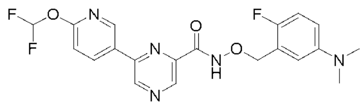 6-(6-(difluoromethoxy)pyridine -3-yl)-*N*-((5-(dimethylamino)-2-fluorobenzyl)oxy)pyrazine -2-carboxamide | $^1$H-NMR, 400 MHz, DMSO-$d_6$ $\delta$ = 12.38 (s, 1H), 9.57 (s, 1H), 9.30 (d, J = 2.40 Hz, 1H), 9.12 (s, 1H), 8.96-8.93 (m, 1H), 7.83 (s, 1H), 7.33-7.30 (m, 1H), 7.09-7.05 (m, 1H), 6.91 (m, 1H), 6.78-6.76 (m, 1H), 5.03 (s, 2H), 2.87 (s, 6H). [M+H]$^+$: 434.2. |
| 82 | 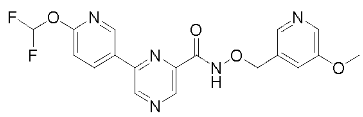 6-(6-(difluoromethoxy)pyridine -3-yl)-*N*-((5-methoxypyridine-3-yl)methoxy)pyrazine-2-carboxamide | $^1$H-NMR, 400 MHz, DMSO-$d_6$ $\delta$ = 12.42 (br s, 1H), 9.57 (s, 1H), 9.29 (d, J = 2.3 Hz, 1H), 9.12 (s, 1H), 8.93 (dd, J = 2.3, 8.7 Hz, 1H), 8.29 (dd, J = 2.2, 4.2 Hz, 2H), 8.05 - 7.63 (m, 1H), 7.59 - 7.52 (m, 1H), 7.33 (d, J = 8.6 Hz, 1H), 5.06 (s, 2H), 3.86 (s, 3H). [M+H]$^+$: 404.1. |
| 83 | 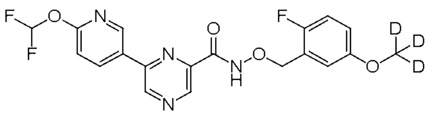 6-(6-(difluoromethoxy)pyridine -3-yl)-*N*-((2-fluoro-5-(methoxy-$d_3$)benzyl)oxy)pyrazine-2-carboxamide | $^1$H-NMR, 400 MHz, DMSO-$d_6$ $\delta$ = 12.40 (s, 1H), 9.56 (s, 1H), 9.29 (d, J = 2.3 Hz, 1H), 9.11 (s, 1H), 8.93 (dd, J = 2.5, 8.6 Hz, 1H), 7.82 (t, J = 72.5 Hz, 1H), 7.31 (d, J = 8.6 Hz, 1H), 7.21 - 7.10 (m, 2H), 6.97 (td, J = 3.7, 8.9 Hz, 1H), 5.04 (s, 2H). [M+H]$^+$: 423.9. |

(continued)

| Compound Nº | Structure | $^1$H-NMR / MS(m/z) [M+H]$^+$ |
|---|---|---|
| 84 | 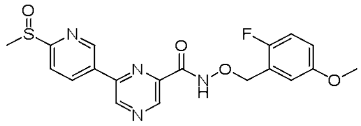 *N*-((2-fluoro-5-methoxybenzyl)oxy)-6-(6-(methylsulfinyl) pyridine-3-yl)pyrazine-2-carboxamide | $^1$H-NMR, 400 MHz, DMSO-$d_6$ $\delta$ = 12.45 (s, 1H), 9.67 to 9.64 (m, 2H), 9.18 (s, 1H), 9.09 to 9.07 (m, 1H), 8.10 to 8.08 (m, 1H), 7.21 to 7.14 (m, 2H), 7.00 to 6.97 (m, 1H), 5.06 (s, 2H), 3.76 (s, 3H), 2.88 (s, 3H). [M+H]$^+$: 417.2. |
| 85 | 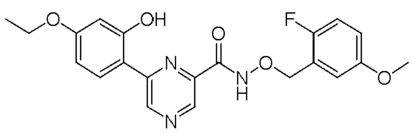 6-(4-ethoxy-2-hydroxyphenyl)-*N*-((2-fluoro-5-methoxybenzyl)oxy)pyrazine -2-carboxamide | $^1$H-NMR, 400 MHz, DMSO-$d_6$ $\delta$ = 12.22 (br s, 1H), 11.01 - 10.67 (m, 1H), 9.47 (s, 1H), 8.88 (s, 1H), 8.16 (d, J = 8.8 Hz, 1H), 7.20 - 7.07 (m, 2H), 7.00 - 6.90 (m, 1H), 6.63 - 6.47 (m, 2H), 5.02 (s, 2H), 4.06 (q, J = 7.0 Hz, 2H), 3.75 (s, 3H), 1.35 (t, J = 6.9 Hz, 3H). [M+H]$^+$: 414.4. |
| 86 | 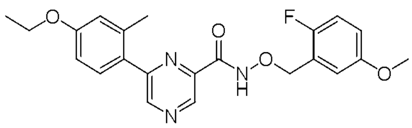 6-(4-ethoxy-2-methylphenyl)-*N*-((2-fluoro-5-methoxybenzyl)oxy)pyrazine -2-carboxamide | $^1$H-NMR, 400 MHz, DMSO-$d_6$ $\delta$ = 12.09 (br s, 1H), 9.03 (s, 1H), 8.99 (s, 1H), 7.56 (d, J = 8.4 Hz, 1H), 7.17 to 7.11 (m, 2H), 6.97 to 6.90 (m, 3H), 5.00 (s, 2H), 4.12 to 4.07 (q, J = 7.2 Hz, 2H), 3.74 (s, 3H), 2.37 (s, 3H), 1.35 (t, J = 7.2 Hz, 3H). [M+H]$^+$: 412.1. |
| 87 | 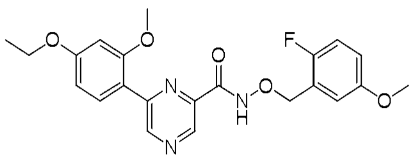 6-(4-ethoxy-2-methoxyphenyl)-*N*-((2-fluoro-5-methoxybenzyl)oxy)pyrazine -2-carboxamide | $^1$H-NMR, 400 MHz, DMSO-$d_6$ $\delta$ = 12.22 (s, 1H), 9.31 (s, 1H), 8.93(s, 1H), 8.14 (d, , J = 8.4 Hz, 1H), 7.19 to 7.13 (m, 2H), 6.99 to 6.94 (m, 1H), 6.73 to 6.69 (m, 2H), 5.02 (s, 2H), 4.17 to 4.12 (q, J = 7.2 Hz, 2H), 3.91 (s, 3 H), 3.75 (s, 3H), 1.37 (t, J = 6.8 Hz, 3H). [M+H]$^+$: 428.1. |
| 88 | 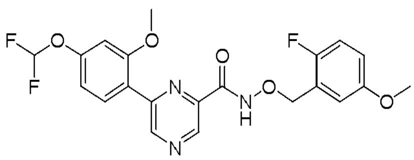 6-(4-(difluoromethoxy)-2-methoxyphenyl)-*N*-((2-fluoro-5-methoxybenzyl)oxy)pyrazine -2-carboxamide | $^1$H-NMR, 400 MHz, DMSO-$d_6$ $\delta$ = 12.26 (s, 1H), 9.33 (s, 1H), 9.01 (s, 1H), 8.19 (d, J = 8.8 Hz, 1H), 7.44 (t, J = 74 Hz, 1H), 7.19 to 7.13 (m, 2H) 7.04 (m, 1H), 6.99 to 6.94 (m, 2H), 5.02 (s, 2H), 3.93 (s, 3H), 3.75 (s, 3H). [M+H]$^+$: 449.9. |
| 89 | 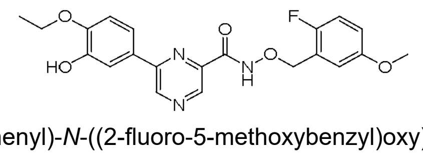 6-(4-ethoxy-3-hydroxyphenyl)-*N*-((2-fluoro-5-methoxybenzyl)oxy)pyrazine-2-carboxamide | |
| 90 | 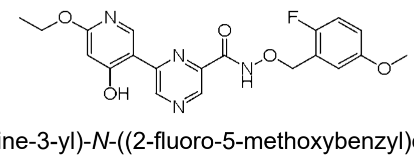 6-(6-ethoxy-4-hydroxypyridine-3-yl)-*N*-((2-fluoro-5-methoxybenzyl)oxy)pyrazine-2-carboxamide | |

(continued)

| Compound № | Structure | $^1$H-NMR / MS(m/z) $[M+H]^+$ |
|---|---|---|
| 91 | 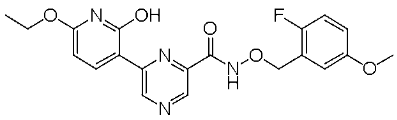 6-(6-ethoxy-2-hydroxypyridine-3-yl)-*N*-((2-fluoro-5-methoxybenzyl)oxy)pyrazine-2-carboxamide | |
| 92 | 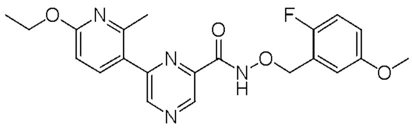 6-(6-ethoxy-2-methylpyridine-3-yl)-*N*-((2-fluoro-5-methoxybenzyl)oxy)pyrazine -2-carboxamide | $^1$H-NMR, 400 MHz, DMSO-$d_6$ $\delta$ = 12.15 (s, 1H), 9.06 (s, 2H), 8.01 (d, J = 8.4 Hz, 1H), 7.18-7.11 (m, 2H), 6.97-6.95 (m, 1H), 6.81-6.79 (d, J = 8.4 Hz, 1H), 5.01 (s, 2H), 4.40-4.34 (q, J = 7.2 Hz, 2H), 3.74 (s, 3H), 2.52-2.50 (m, 2 H), 1.35 (t, J = 7.2 Hz, 3H). $[M+H]^+$: 413.1. |
| 93 | 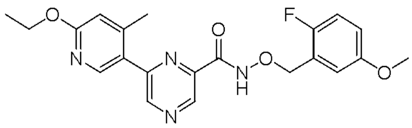 6-(6-ethoxy-4-methylpyridine-3-yl)-*N*-((2-fluoro-5-methoxybenzyl)oxy)pyrazine -2-carboxamide | $^1$H-NMR, 400 MHz, DMSO-$d_6$ $\delta$ = 12.14 (s, 1H), 9.08 (d, J = 6.4 Hz, 2H), 8.40 (s, 1H), 7.17-7.11 (m, 2H), 6.97-6.93 (m, 1H), 6.83 (s, 1H), 5.00 (s, 1H), 4.39-4.34 (q, J = 7.2 Hz, 2H), 3.74 (s, 3H), 2.39 (s, 3H), 1.36-1.30 (m, 3 H). $[M+H]^+$: 413.1. |
| 94 | 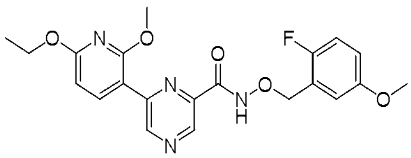 6-(6-ethoxy-2-methoxypyridine-3-yl)-*N*-((2-fluoro-5-methoxybenzyl)oxy)pyrazin e-2-carboxamide | $^1$H-NMR, 400 MHz, DMSO-$d_6$ $\delta$ = 12.12 (brs ,1H), 9.44 (s, 1H), 8.96 (s, 1H), 8.75 (d, J = 8.4Hz, 1H), 7.19-7.13 (m, 2H), 6.99-6.96 (m, 1H), 6.60 (d, J = 8.4Hz, 1H), 5.03 (s, 2H), 4.45-4.40 (q, J = 7.2Hz, 2H), 4.05 (s, 3H), 3.75(s, 3H), 1.38 (t, J = 7.2Hz, 3H). $[M+H]^+$: 429.0. |
| 95 | 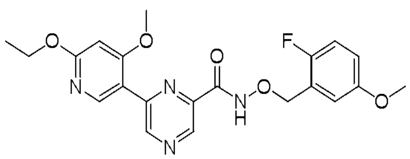 6-(6-ethoxy-4-methoxypyridine-3-yl)-*N*-((2-fluoro-5-methoxybenzyl)oxy)pyrazin e-2-carboxamide | $^1$H-NMR, 400 MHz, DMSO-$d_6$ $\delta$ = 12.18 (s, 1H), 9.13 (s, 2H), 8.22 (d, J = 8.4 Hz, 1H), 7.812 (t, J = 72.8 Hz, 1H), 7.18 to 7.11 (m, 3H), 6.98 to 6.94 (m, 1H), 5.01 (s, 2H), 3.74 (s, 3H), 2.55 (s, 3H), 1.23 (s, 3H). $[M+H]^+$: 435.0. |
| 96 | 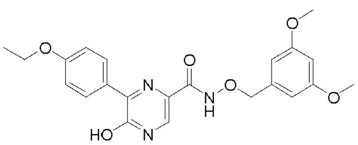 *N*-((3,5-dimethoxybenzyl)oxy)-6-(4-ethoxyphenyl)-5-hydro-xypyrazine-2-carboxamide | $^1$H-NMR, 400 MHz, DMSO-$d_6$ $\delta$ = 12.74 (s, 1H), 11.67 (s, 1H), 8.58 (dd, J = 6.8, 2.0 Hz, 2 H), 7.87 (s, 1H), 6.98 (m, 2 H), 6.65 (d, J = 2.0 Hz, 2H), 6.47 (t, J = 2.4 Hz, 1 H), 4.89 (s, 2H), 4.11 (q, J = 7.0 Hz, 2H), 3.76 (s, 6H), 1.37 (t, J = 6.8 Hz, 3H). $[M+H]^+$: 426.1. |

(continued)

| Compound № | Structure | $^1$H-NMR / MS(m/z) [M+H]$^+$ |
|---|---|---|
| 97 | 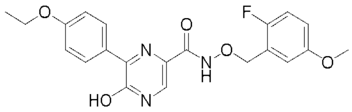 6-(4-ethoxyphenyl)-*N*-((2-fluoro-5-methoxybenzyl)oxy)-5-hydroxypyrazine-2-carboxamide | $^1$H-NMR, 400 MHz, DMSO-$d_6$ $\delta$ = 12.77 (s, 1H), 11.70 (s, 1H), 8.57 (m, 2.0 Hz, 2 H), 7.87 (s, 1H), 7.16 (m, 2H), 6.99 (m, 3H), 4.99 (s, 2H), 4.11 (q, J = 7.2 Hz, 2H), 3.76 (s, 3H), 1.67 (t, J = 6.8 Hz, 3H). [M+H]$^+$: 412.2. |
| 98 | 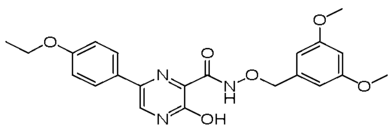 *N*-((3,5-dimethoxybenzyl)oxy)-6-(4-ethoxyphenyl)-3-hydroxypyrazine-2-carboxamide | $^1$H-NMR, 400 MHz, DMSO-$d_6$ $\delta$ = 8.39 (s, 1H), 7.79 (m, 2H), 6.94 (dd, J = 6.8, 2.0 Hz, 2H), 6.61 (s, 2H), 6.44 (s, 1H), 4.86 (s, 2H), 4.04 (q, J = 7.2 Hz, 2H), 3.75 (s, 6H), 1.34 (t, J = 7.2 Hz, 3H). [M+H]$^+$: 424.1. |
| 99 | 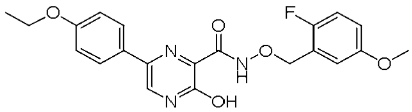 6-(4-ethoxyphenyl)-*N*-((2-fluoro-5-methoxybenzyl)oxy)-3-hydroxypyrazine-2-carboxamide | $^1$H-NMR, 400 MHz, DMSO-2 $d_6$ $\delta$ = 8.39 (s, 1H), 7.78 (m, 1H), 7.14 (m, 1H), 6.94 (m, 3H), 6.07 (s, 1H), 4.95 (s, 2H), 4.05 (q, J = 6.8 Hz, 2H), 3.75 (s, 3H), 1.34 (t, J = 7.2 Hz, 3H). [M+H]$^+$: 412.1. |
| 100 | 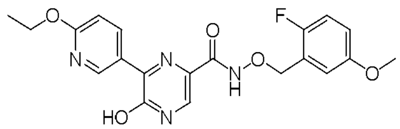 6-(6-ethoxypyridine-3-yl)-*N*-((2-fluoro-5-methoxybenzyl) oxy)-5-hydroxypyrazine-2-carboxamide | $^1$H-NMR, 400 MHz, DMSO-$d_6$ $\delta$ = 12.90 (s, 1H), 11.79 (s, 1H), 9.42 (s, 1H), 8.87 (m, 1H), 8.82 (s, 1H), 7.92 (s, 1H), 7.15 (m, 1H), 6.98 (m, 1H), 6.95 (d, J = 3.6 Hz, 1H), 4.98 (s, 2H), 4.39 (q, J = 7.2 Hz, 2H), 3.76 (s, 3H), 1.36 (t, J = 7.2 Hz, 3H). [M+H]$^+$: 413.0. |
| 101 | 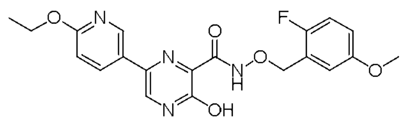 6-(6-ethoxypyridine-3-yl)-*N*-((2-fluoro-5-methoxybenzyl) oxy)-3-hydroxypyrazine-2-carboxamide | $^1$H-NMR, 400 MHz, DMSO-$d_6$ $\delta$ = 8.78 (s, 1H), 8.34 (s, 1H), 7.16 (m, 1H), 7.12 (m, 1H), 6.97 (m, 1H), 6.88 (m, 1H), 5.01 (s, 2H), 4.34 (q, J = 6.8 Hz, 2H), 3.76 (s, 3H), 1.36 (t, J = 7.2 Hz, 3H). [M+H]$^+$: 413.1. |
| 102 | 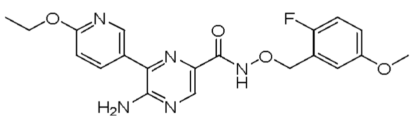 5-amino-6-(6-ethoxypyridine-3-yl)-*N*-((2-fluoro-5-methoxybenzyl)oxy)pyrazin e-2-carboxamide | $^1$H-NMR, 400 MHz, DMSO-$d_6$ $\delta$ = 11.57 (s, 1 H), 8.59 (d, J = 2.25 Hz, 1 H), 8.44 (s, 1 H) 8.11 (dd, J = 8.63, 2.50 Hz, 1 H), 7.07 - 7.19 (m, 2 H), 6.86 - 7.01 (m, 4 H), 4.95 (s, 2 H), 4.38 (q, J = 7.00 Hz, 2 H), 3.74 (s, 3 H), 1.35 (t, J = 7.07 Hz, 3 H). [M+H]$^+$: 414.1. |

(continued)

| Compound № | Structure | $^1$H-NMR / MS(m/z) [M+H]$^+$ |
|---|---|---|
| 103 | 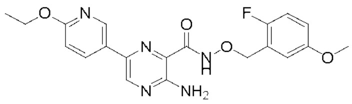 3-amino-6-(6-ethoxypyridine-3-yl)-*N*-((2-fluoro-5-methoxybenzyl)oxy)pyrazin e-2-carboxamide | $^1$H-NMR, 400 MHz, DMSO-$d_6$ $\delta$ = 12.02 (s, 1H), 8.96 (d, J = 2.1 Hz, 1H), 8.85 (s, 1H), 8.51 (dd, J = 2.4, 8.6 Hz, 1H), 7.52 (br s, 2H), 7.20 - 7.11 (m, 2H), 7.00 - 6.93 (m, 1H), 6.87 (d, J = 8.8 Hz, 1H), 5.00 (s, 2H), 4.35 (q, J = 7.0 Hz, 2H), 3.75 (s, 3H), 1.34 (t, J = 7.0 Hz, 3H). [M+H]$^+$: 414.1. |
| 104 | 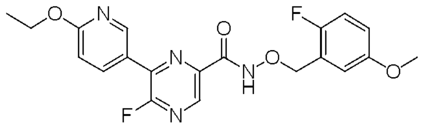 6-(6-ethoxypyridine-3-yl)-5-fluoro-*N*-((2-fluoro-5-methoxybenzyl)oxy)pyrazin e-2-carboxamide | $^1$H-NMR, 400 MHz, DMSO-$d_6$ $\delta$ = 12.31 (s, 1H), 9.03 (s, 1H), 8.74 (d, J = 1.4 Hz, 1H), 8.56 (dd, J = 1.3, 8.9 Hz, 1H), 7.21 - 7.10 (m, 2H), 7.03 - 6.93 (m, 2H), 5.03 (s, 2H), 4.42 (q, J = 7.0 Hz, 2H), 3.75 (s, 3H), 1.36 (t, J = 7.0 Hz, 3H). [M+H]$^+$: 417.0. |
| 105 | 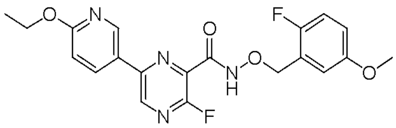 6-(6-ethoxypyridine-3-yl)-3-fluoro-*N*-((2-fluoro-5-methoxybenzyl)oxy)pyrazin e-2-carboxamide | $^1$H-NMR, 400 MHz, DMSO-$d_6$ $\delta$ = 12.21 (s, 1H), 9.17 - 8.97 (m, 2H), 8.55 (dd, J = 2.3, 8.7 Hz, 1H), 7.22 - 7.08 (m, 2H), 7.02 - 6.93 (m, 2H), 5.02 (s, 2H), 4.39 (q, J = 7.0 Hz, 2H), 3.75 (s, 3H), 1.36 (t, J = 7.0 Hz, 3H). [M+H]$^+$: 417.4. |
| 106 | 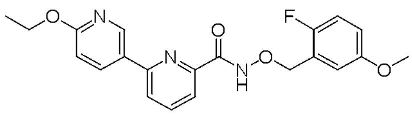 6'-ethoxy-*N*-((2-fluoro-5-methoxybenzyl)oxy)-[2,3'-bipyri-dine]-6-carboxamide | $^1$H-NMR, 400 MHz, DMSO-$d_6$ $\delta$ = 1.37 (s, 3 H) 3.76 (s, 3 H) 4.40 (q, J=7.05 Hz, 2 H) 5.03 (s, 2 H) 6.86 - 7.01 (m, 2 H) 7.09 - 7.23 (m, 2 H) 7.89 - 7.95 (m, 1 H) 8.06 (t, J=7.82 Hz, 1 H) 8.15 - 8.22 (m, 1 H) 8.67 (dd, J=8.76, 2.50 Hz, 1 H) 9.14 (d, J=2.13 Hz, 1 H) 12.14 (s, 1 H). [M+H]$^+$: 398.4. |
| 107 | 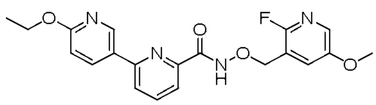 6'-ethoxy-*N*-((2-fluoro-5-methoxypyridine-3-yl)methoxy)-[2,3'-bipyridine]-6-carboxamide | $^1$H-NMR, 400 MHz, DMSO-$d_6$ $\delta$ = 1.36 (t, J=7.07 Hz, 3 H) 3.84 (s, 3 H) 4.39 (q, J=7.05 Hz, 2 H) 5.04 (s, 2 H) 6.92 (d, J=8.63 Hz, 1 H) 7.77 (dd, J=7.75, 3.00 Hz, 1 H) 7.86 - 7.94 (m, 2 H) 8.05 (t, J=7.82 Hz, 1 H) 8.12 - 8.23 (m, 1 H) 8.65 (dd, J=8.76, 2.50 Hz, 1 H) 9.13 (d, J=2.25 Hz, 1 H) 12.14 (s, 1 H). [M+H]$^+$: 399.4. |

(continued)

| Compound No | Structure | $^1$H-NMR / MS(m/z) [M+H]$^+$ |
|---|---|---|
| 108 | 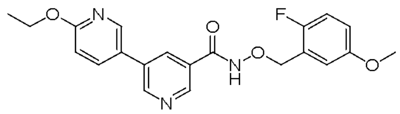 6'-ethoxy-*N*-((2-fluoro-5-methoxybenzyl)oxy)-[3,3'-bipyridine]-5-carboxamide | $^1$H-NMR, 400 MHz, DMSO-$d_6$ $\delta$ = 12.02 (br s, 1H), 9.04 (d, J = 1.4 Hz, 1H), 8.85 (d, J = 1.4 Hz, 1H), 8.58 (d, J = 2.3 Hz, 1H), 8.32 (br s, 1H), 8.07 - 8.15 (m, 1H), 7.08 - 7.20 (m, 2H), 6.91 - 7.02 (m, 2H), 5.00 (s, 2H), 4.36 (q, J = 7.0 Hz, 2H), 3.75 (s, 3H), 1.34 (t, J = 7.1 Hz, 3H). [M+H]$^+$: 398.0. |
| 109 | 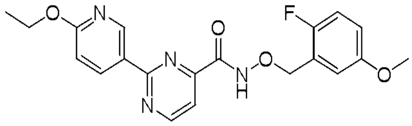 2-(6-ethoxypyridine-3-yl)-*N*-((2-fluoro-5-methoxybenzyl) oxy)pyrimid ine-4-carboxamide | $^1$H-NMR, 400 MHz, DMSO-$d_6$ $\delta$ = 12.47 (s, 1H), 9.38 (d, J = 2.3 Hz, 1H), 9.10 (d, J = 4.9 Hz, 1H), 8.81 (dd, J = 2.4, 8.8 Hz, 1H), 7.85 (d, J = 5.0 Hz, 1H), 7.21 - 7.10 (m, 2H), 7.01 - 6.91 (m, 2H), 5.04 (s, 2H), 4.41 (q, J = 7.0 Hz, 2H), 3.75 (s, 3H), 1.36 (t, J = 7.1 Hz, 3H). [M+H]$^+$: 399.4. |
| 110 | 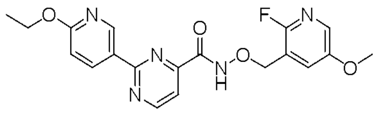 2-(6-ethoxypyridine-3-yl)-*N*-((2-fluoro-5-methoxypyridine-3-yl)methoxy)pyrimidine-4-carboxamide | $^1$H-NMR, 400 MHz, DMSO-$d_6$ $\delta$ = 1.21 - 1.58 (m, 2 H) 1.37 - 1.37 (m, 1 H) 3.77 - 3.88 (m, 3 H) 4.36 - 4.48 (m, 2 H) 5.06 (s, 2 H) 6.83 (d, J=2.38 Hz, 1 H) 7.62 - 8.11 (m, 2 H) 8.59 - 8.93 (m, 1 H) 8.68 - 8.90 (m, 1 H) 8.99 (s, 1 H) 9.51 (s, 1 H) 12.33 - 12.57 (m, 1 H). [M+H]$^+$: 400.0. |
| 111 | 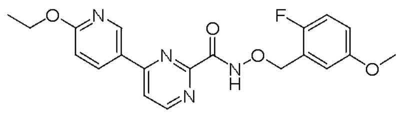 4-(6-ethoxypyridine-3-yl)-*N*-((2-fluoro-5-methoxybenzyl) oxy)pyrimid ine-2-carboxamide | $^1$H-NMR, 400 MHz, DMSO-$d_6$ $\delta$ = 9.06 (d, J = 2.3 Hz, 1H), 8.96 (br d, J = 5.3 Hz, 1H), 8.53 (dd, J = 2.5, 8.8 Hz, 1H), 8.23 (d, J = 5.4 Hz, 1H), 6.98 (d, J = 8.6 Hz, 1H), 4.40 (q, J = 7.0 Hz, 2H), 1.35 (t, J = 7.0 Hz, 3H). [M+H]$^+$: 398.1. |
| 112 | 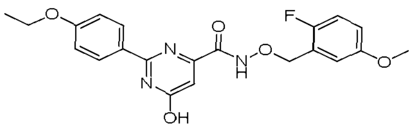 2-(4-ethoxyphenyl)-*N*-((2-fluoro-5-methoxybenzyl)oxy)-6-hydroxypyrimidine-4-carboxamide | $^1$H-NMR, 400 MHz, DMSO-$d_6$ $\delta$ = 12.82 (br s, 1H), 12.11 (s, 1H), 8.36 (br d, J = 8.8 Hz, 2H), 7.21 - 6.92 (m, 5H), 6.72 (br s, 1H), 5.00 (s, 2H), 4.14 (q, J = 6.9 Hz, 2H), 3.75 (s, 3H), 1.36 (t, J = 6.9 Hz, 3H). [M+H]$^+$: 414.1. |
| 113 | 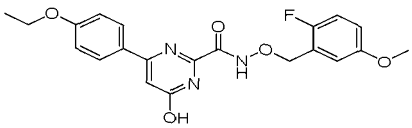 4-(4-ethoxyphenyl)-*N*-((2-fluoro-5-methoxybenzyl)oxy)-6-hydroxypyrimidine-2-carboxamide | $^1$H-NMR, 400 MHz, DMSO-$d_6$ $\delta$ = 12.83 (br dd, J = 1.9, 9.9 Hz, 1H), 12.11 (s, 1H), 8.36 (br d, J = 8.5 Hz, 2H), 7.21 - 7.10 (m, 2H), 7.05 (d, J = 8.9 Hz, 2H), 6.96 (td, J = 3.7, 8.8 Hz, 1H), 6.71 (br s, 1H), 5.00 (s, 2H), 4.14 (q, J = 6.9 Hz, 2H), 3.75 (s, 3H), 1.36 (t, J = 7.0 Hz, 3H). [M+H]$^+$: 414.1. |

(continued)

| Compound № | Structure | $^1$H-NMR / MS(m/z) [M+H]$^+$ |
|---|---|---|
| 114 | 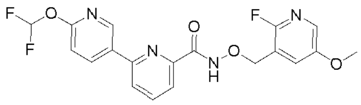 6'-(difluoromethoxy)-*N*-((2-fluoro-5-methoxypyridine-3-yl) methoxy)-[2,3'-bipyridine]-6-carboxamide | $^1$H-NMR, 400 MHz, DMSO-$d_6$ $\delta$ = 12.20 (br s, 1H), 9.23 (d, J = 2.3 Hz, 1H), 8.86 (dd, J = 2.4, 8.7 Hz, 1H), 8.28 (d, J = 8.0 Hz, 1H), 8.11 (t, J = 7.9 Hz, 1H), 8.00 - 7.95 (m, 1H), 7.93 - 7.88 (m, 1H), 7.81 - 7.74 (m, 1H), 7.62 (s, 1H), 7.25 (d, J = 8.8 Hz, 1H), 5.05 (s, 2H), 3.84 (s, 3H). [M+H]$^+$: 421.3. |
| 115 | 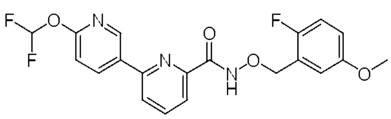 6'-(difluoromethoxy)-*N*-((2-fluoro-5-methoxybenzyl) oxy)-[2,3'-bipyridine]-6-carboxamide | $^1$H-NMR, 400 MHz, DMSO-$d_6$ $\delta$ = 12.20 (s, 1H), 9.24 (d, J = 2.3 Hz, 1H), 8.88 (dd, J = 2.4, 8.7 Hz, 1H), 8.27 (dd, J = 0.8, 8.0 Hz, 1H), 8.11 (t, J = 7.8 Hz, 1H), 8.01 - 7.57 (m, 2H), 7.30 - 7.09 (m, 3H), 6.96 (td, J = 3.7, 8.9 Hz, 1H), 5.03 (s, 2H), 3.75 (s, 3H). [M+H]$^+$: 420.3. |
| 116 | 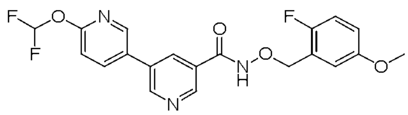 6'-(difluoromethoxy)-*N*-((2-fluoro-5-methoxybenzyl) oxy)-[3,3'-bipyridine]-5-carboxamide | $^1$H-NMR, 400 MHz, DMSO-$d_6$ $\delta$ = 11.93 - 12.12 (m, 1H), 9.09 (s, 1H), 8.89 (s, 1H), 8.70 (d, J = 2.4 Hz, 1H), 8.30 - 8.42 (m, 2H), 7.78 (t, J = 72.7 Hz, 1H), 7.27 (d, J = 8.5 Hz, 1H), 7.07 - 7.22 (m, 2H), 6.91 - 7.02 (m, 1H), 5.01 (s, 2H), 3.75 (s, 3H). [M+H]$^+$: 420.1 |
| 117 | 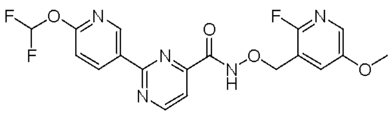 2-(6-(difluoromethoxy)pyridine -3-yl)-*N*-((2-fluoro-5-methoxypyridine-3-yl)methoxy)pyrimidine-4-carboxamide | $^1$H-NMR, 400 MHz, DMSO-$d_6$ $\delta$ = 3.84 (s, 3 H) 5.06 (s, 2 H) 7.29 (d, J=8.76 Hz, 1 H) 7.62 - 7.69 (m, 1 H) 7.72 - 8.07 (m, 3 H) 9.02 (dd, J=8.63, 2.38 Hz, 1 H) 9.17 (d, J=5.00 Hz, 1 H) 9.45 (d, J=2.13 Hz, 1 H) 12.55 (s, 1 H). [M+H]$^+$: 422.0. |
| 118 | 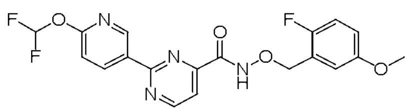 2-(6-(difluoromethoxy)pyridine -3-yl)-*N*-((2-fluoro-5-methoxybenzyl)oxy)pyrimid ine-4-carboxamide | $^1$H-NMR, 400 MHz, DMSO-$d_6$ $\delta$ = 12.55 (s, 1H), 9.46 (d, J = 2.0 Hz, 1H), 9.17 (d, J = 4.9 Hz, 1H), 9.04 (dd, J = 2.3, 8.6 Hz, 1H), 8.05 - 7.62 (m, 2H), 7.29 (d, J = 8.8 Hz, 1H), 7.22 - 7.09 (m, 2H), 7.03 - 6.93 (m, 1H), 5.05 (s, 2H), 3.76 (s, 3H). [M+H]$^+$: 421.4. |
| 119 | 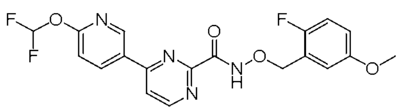 4-(6-(difluoromethoxy)pyridine -3-yl)-*N*-((2-fluoro-5-methoxybenzyl)oxy)pyrimid ine-2-carboxamide | $^1$H-NMR, 400 MHz, DMSO-$d_6$ $\delta$ = 12.31 (s, 1H), 9.28 (d, J = 2.1 Hz, 1H), 9.04 (d, J = 5.3 Hz, 1H), 8.88 (dd, J = 2.4, 8.8 Hz, 1H), 8.32 (d, J = 5.4 Hz, 1H), 7.83 (t, J = 72.4 Hz, 1H), 7.31 (d, J = 8.8 Hz, 1H), 7.13 - 7.19 (m, 2H), 6.96 (td, J = 3.7, 8.8 Hz, 1H), 5.03 (s, 2H), 3.75 (s, 3H). [M+H]$^+$: 421.0. |

(continued)

| Compound № | Structure | $^1$H-NMR / MS(m/z) [M+H]$^+$ |
|---|---|---|
| 120 | 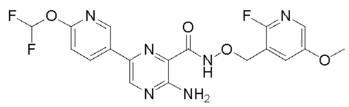 3-amino-6-(6-(difluoromethoxy)pyridine -3-yl)-*N*-((2-fluoro-5-methoxypyridine-3-yl)methoxy)pyrazine-2-carboxamide | $^1$H-NMR, 400 MHz, DMSO-$d_6$ $\delta$ = 12.20 (s, 1H), 9.24 (d, J = 2.3 Hz, 1H), 8.88 (dd, J = 2.4, 8.7 Hz, 1H), 8.27 (dd, J = 0.8, 8.0 Hz, 1H), 8.11 (t, J = 7.8 Hz, 1H), 7.98 (d, J = 1.5 Hz, 1H), 7.97 - 7.61 (m, 1H), 7.24 (d, J = 8.6 Hz, 1H), 7.20 - 7.11 (m, 2H), 6.96 (td, J = 3.7, 8.9 Hz, 1H), 5.03 (s, 2H), 3.75 (s, 3H). [M+H]$^+$: 437.4. |
| 121 | 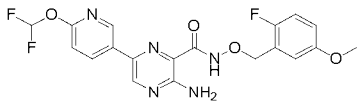 3-amino-6-(6-(difluoromethoxy)pyridine -3-yl)-*N*-((2-fluoro-5-methoxybenzyl)oxy)pyrazin e-2-carboxamide | $^1$H-NMR, 400 MHz, DMSO-$d_6$ $\delta$ = 12.09 (s, 1H), 9.07 (d, J = 2.1 Hz, 1H), 8.92 (s, 1H), 8.76 - 8.67 (m, 1H), 7.98 - 7.54 (m, 3H), 7.21 - 7.09 (m, 3H), 7.01 - 6.91 (m, 1H), 5.00 (s, 2H), 3.84 - 3.65 (m, 3H). [M+H]$^+$: 436.4. |
| 122 | 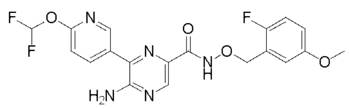 5-amino-6-(6-(difluoromethoxy)pyridine -3-yl)-*N*-((2-fluoro-5-methoxybenzyl)oxy)pyrazin e-2-carboxamide | $^1$H-NMR, 400 MHz, DMSO-$d_6$ $\delta$ = 11.61 (s, 1 H), 8.67 (d, J = 2.00 Hz, 1 H), 8.48 (s, 1 H), 8.32 (dd, J = 8.51, 2.50 Hz, 1 H), 7.80 (t, J = 72.73 Hz, 1 H), 7.03 - 7.24 (m, 5 H), 3.74 (s, 3 H), 6.94 (dt, J = 8.82, 3.72 Hz, 1 H), 4.95 (s, 2 H). [M+H]$^+$: 436.0. |
| 123 | 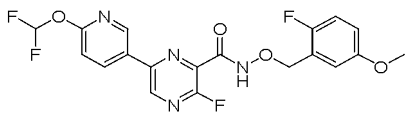 6-(6-(difluoromethoxy)pyridine -3-yl)-3-fluoro-*N*-((2-fluoro-5-methoxybenzyl)oxy)pyrazin e-2-carboxamide | $^1$H-NMR, 400 MHz, DMSO-$d_6$ $\delta$ = 12.26 (br s, 1H), 9.22 - 9.12 (m, 2H), 8.77 (dd, J = 2.4, 8.6 Hz, 1H), 7.82 (t, J = 72.5 Hz, 1H), 7.30 (d, J = 8.6 Hz, 1H), 7.22 - 7.09 (m, 2H), 6.98 (td, J = 3.6, 8.9 Hz, 1H), 5.02 (s, 2H), 3.75 (s, 3H). [M+H]$^+$: 439.3. |
| 124 | 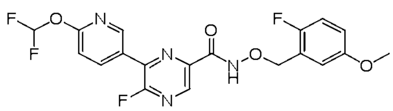 6-(6-(difluoromethoxy)pyridine -3-yl)-5-fluoro-*N*-((2-fluoro-5-methoxybenzyl)oxy)pyrazin e-2-carboxamide | $^1$H-NMR, 400 MHz, Methanol-$d_4$ $\delta$ = 9.01 (s, 1H), 8.83 (d, J = 1.6 Hz, 1H), 8.68 - 8.63 (m, 1H), 7.67 (t, J = 72.6 Hz, 1H), 7.19 - 7.13 (m, 2H), 7.07 - 7.00 (m, 1H), 6.92 (td, J = 3.6, 9.0 Hz, 1H), 5.09 (s, 2H), 3.79 (s, 3H). [M+H]$^+$: 439.4. |
| 125 | 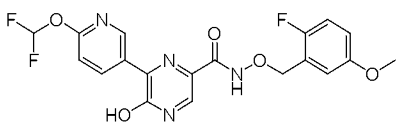 6-(6-(difluoromethoxy)pyridine-3-yl)-*N*-((2-fluoro-5-methoxybenzyl)oxy)-5-hydroxypyrazine-2-carboxamide | |

(continued)

| Compound № | Structure | $^1$H-NMR / MS(m/z) [M+H]$^+$ |
|---|---|---|
| 126 | 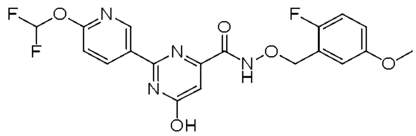 2-(6-(difluoromethoxy)pyridine -3-yl)-*N*-((2-fluoro-5-methoxybenzyl)oxy)-6-hydroxypyrimidine-4-carboxamide | $^1$H-NMR, 400 MHz, DMSO-$d_6$ $\delta$ = 13.34 - 12.96 (m, 1H), 12.22 (br s, 1H), 9.25 (br s, 1H), 8.88 (dd, J = 1.9, 8.7 Hz, 1H), 7.82 (t, J = 72.4 Hz, 1H), 7.28 (d, J = 8.8 Hz, 1H), 7.23 - 7.08 (m, 2H), 7.01 - 6.80 (m, 2H), 5.00 (s, 2H), 3.75 (s, 3H). [M+H]$^+$: 437.1. |
| 127 | 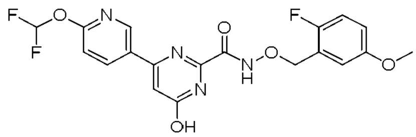 4-(6-(difluoromethoxy)pyridine -3-yl)-*N*-((2-fluoro-5-methoxybenzyl)oxy)-6-hydroxypyrimidine-2-carboxamide | $^1$H-NMR, 400 MHz, DMSO-$d_6$ $\delta$ = 3.75 (s, 3 H) 5.00 (s, 2 H) 6.85 (s, 1 H) 6.97 (dt, J = 8.88, 3.63 Hz, 1 H) 7.10 - 7.20 (m, 2 H) 7.27 (d, J = 8.76 Hz, 1 H) 7.82 (t, J = 72.35 Hz, 1 H) 8.88 (dd, J = 8.75, 2.38 Hz, 1 H) 9.25 (d, J = 2.00 Hz, 1 H) 12.13 - 12.30 (m, 1 H) 12.77 - 13.43 (m, 1 H). [M+H]$^+$: 437.1. |
| 128 | 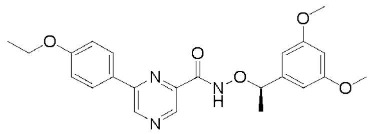 (*R**)-*N*-(1-(3,5-dimethoxyphenyl)ethoxy)-6-(4-ethoxyphenyl)pyrazine-2-carboxamide | $^1$H-NMR, 400 MHz, DMSO-$d_6$ $\delta$ = 11.94 (s, 1H), 9.39 (s, 1H), 8.95 (s, 1H), 8.31 (d, J = 8.80 Hz, 2H), 7.09 to 7.06 (m, 2H), 6.67 (d, J = 2.00 Hz, 2H), 6.43 (s, 1H), 5.14 (q, J = 6.40 Hz, 1H), 4.14 (q, J = 7.20 Hz, 2H), 3.75 (s, 6H), 1.51 (s, 3H), 1.35 (s, 3H). [M+H]$^+$: 424.0. |
| 129 | 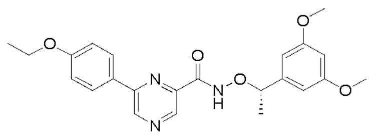 (*S**)-*N*-(1-(3,5-dimethoxyphenyl)ethoxy)-6-(4-ethoxyphenyl)pyrazine-2-carboxamide | $^1$H-NMR, 400 MHz, DMSO-$d_6$ $\delta$ = 11.93 (s, 1H), 9.39 (s, 1H), 8.95 (s, 1H), 8.31 (d, J = 8.80 Hz, 2H), 7.09 to 7.06 (m, 2H), 6.67 (d, J = 2.00 Hz, 2H), 6.43 (s, 1H), 5.14 (q, J = 6.40 Hz, 1H), 4.14 (q, J = 7.20 Hz, 2H), 3.75 (s, 6H), 1.51 (s, 3H), 1.35 (s, 3H). [M+H]$^+$: 424.0. |
| 130 | 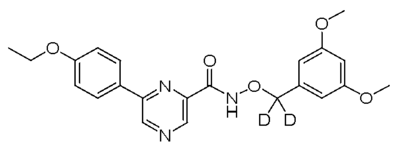 *N*-((3,5-dimethoxyphenyl)methoxy-d2)-6-(4-ethoxyphenyl) pyrazine-2-carboxamide | $^1$H-NMR, 400 MHz, DMSO-$d_6$ $\delta$ = 9.41 (s, 1H), 8.98 (s, 1H), 8.35 (d, J = 9.20 Hz, 2H), 7.09 (d, J = 8.80 Hz, 2H), 6.68 (s, 2H), 6.49 (s, 1H), 4.14 (q, J = 6.80 Hz, 2H), 3.76 (s, 6H), 1.38 (t, J = 7.20 Hz, 3H). [M+H]$^+$: 412.0. |
| 131 | 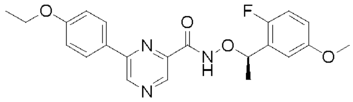 (*R*)*-6-(4-ethoxyphenyl)-*N*-(1-(2-fluoro-5-methoxyphenyl)ethoxy)pyrazine-2-carboxamide | |
| 132 | 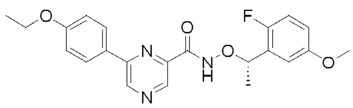 (*S**)-6-(4-ethoxyphenyl)-*N*-(1-(2-fluoro-5-methoxyphenyl)ethoxy)pyrazine-2-carboxamide | |

(continued)

| Compound № | Structure | $^1$H-NMR / MS(m/z) $[M+H]^+$ |
|---|---|---|
| 133 | 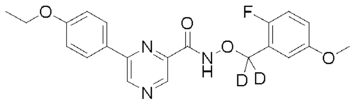 6-(4-ethoxyphenyl)-*N*-((2-fluoro-5-methoxyphenyl)methoxy-$d_2$)pyrazine-2-carboxamide | |
| 134 | 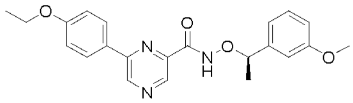 (*R**)-6-(4-ethoxyphenyl)-*N*-(1-(3-methoxyphenyl)ethoxy)pyrazine-2-carboxamide | |
| 135 | 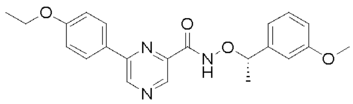 (*S**)-6-(4-ethoxyphenyl)-*N*-(1-(3-methoxyphenyl)ethoxy)pyrazine-2-carboxamide | |
| 136 | 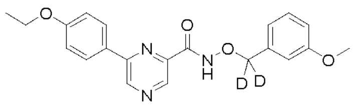 6-(4-ethoxyphenyl)-*N*-((3-methoxyphenyl)methoxy-$d_2$)pyrazine-2-carboxamide | |
| 137 | 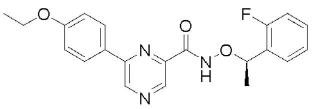 (*R**)-6-(4-ethoxyphenyl)-*N*-(1-(2-fluorophenyl)ethoxy)pyrazine-2-carboxamide | |
| 138 | 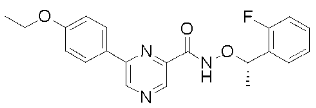 (*S**)-6-(4-ethoxyphenyl)-*N*-(1-(2-fluorophenyl)ethoxy)pyrazine-2-carboxamide | |
| 139 | 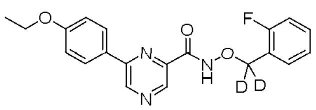 6-(4-ethoxyphenyl)-*N*-((2-fluorophenyl)methoxy-$d_2$)pyrazine-2-carboxamide | |
| 140 | 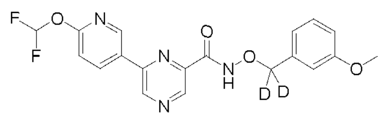 6-(6-(difluoromethoxy)pyridine -3-yl)-*N*-((3-methoxyphenyl) methoxy-$d_2$)pyrazine-2-carboxamide | $^1$H-NMR, 400 MHz, DMSO-$d_6$ $\delta$ = 12.38 (s, 1H), 9.57 (s, 1H), 9.30 (d, J = 2.3 Hz, 1H), 9.12 (s, 1H), 8.94 (dd, J = 2.4, 8.7 Hz, 1H), 7.83 (t, J = 72.5 Hz, 1H), 7.37 - 7.26 (m, 2H), 7.13 - 7.01 (m, 2H), 6.94 (dd, J = 2.3, 8.1 Hz, 1H), 3.77 (s, 3H). $[M+H]^+$: 405.1. |
| 141 | 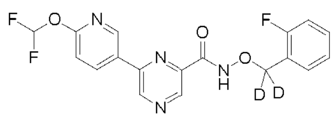 6-(6-(difluoromethoxy)pyridine -3-yl)-*N*-((2-fluorophenyl) methoxy-$d_2$)pyrazine-2-carboxamide | $^1$H-NMR, 400 MHz, DMSO-$d_6$ $\delta$ = 12.40 (br s, 1H), 9.56 (s, 1H), 9.30 (d, J = 2.1 Hz, 1H), 9.11 (s, 1H), 8.93 (dd, J = 2.4, 8.6 Hz, 1H), 7.82 (t, J = 72.5 Hz, 1H), 7.59 (dt, J = 1.6, 7.5 Hz, 1H), 7.45 (ddt, J = 1.8, 5.7, 7.7 Hz, 1H), 7.31 (d, J = 8.6 Hz, 1H), 7.29 - 7.20 (m, 2H). $[M+H]^+$: 393.1. |

(continued)

| Compound № | Structure | $^1$H-NMR / MS(m/z) [M+H]$^+$ |
|---|---|---|
| 142 | 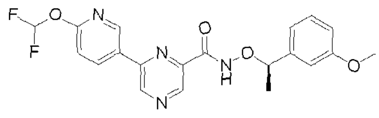 (*R**)-6-(6-(difluoromethoxy)pyridine -3-yl)-*N*-(1-(3-methoxyphenyl)ethoxy)pyra zine-2-carboxamide | $^1$H-NMR, 400 MHz, DMSO-$d_6$ $\delta$ = 12.14 - 11.99 (m, 1H) , 9.53 (s, 1H), 9.27 (d, J = 2.3 Hz, 1H), 9.08 (s, 1H), 8.89 (dd, J = 2.4, 8.7 Hz, 1H), 7.82 (t, J = 72.5 Hz, 1H), 7.33 - 7.23 (m, 2H), 7.12 - 7.02 (m, 2H), 6.93 - 6.82 (m, 1H), 5.17 (q, J = 6.4 Hz, 1H), 3.76 (s, 3H), 1.53 (d, J = 6.5 Hz, 3H). [M+H]$^+$: 417.4. |
| 143 | 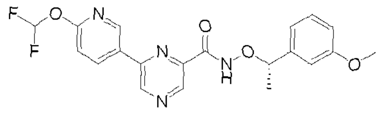 (*S**)-6-(6-(difluoromethoxy)pyridine -3-yl)-*N*-(1-(3-methoxyphenyl)ethoxy)pyra zine-2-carboxamide | $^1$H-NMR, 400 MHz, DMSO-$d_6$ $\delta$ = 12.06 (br s, 1H) , 9.52 (s, 1H) , 9.26 (d, J = 2.3 Hz, 1H) , 9.07 (s, 1H) , 8.88 (dd, J = 2.4, 8.6 Hz, 1H) , 7.81 (t, J = 72.5 Hz, 1H) , 7.33 - 7.21 (m, 2H) , 7.13 - 6.97 (m, 2H) , 6.87 (dd, J = 1.9, 8.2 Hz, 1H) , 5.16 (q, J = 6.5 Hz, 1H) , 3.76 (s, 3H) , 1.52 (d, J = 6.5 Hz, 3H). [M+H]$^+$: 417.4. |
| 144 | 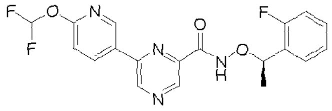 (*R**)-6-(6-(difluoromethoxy)pyridine -3-yl)-*N*-(1-(2-fluorophenyl)ethoxy)pyraz ine-2-carboxamide | $^1$H-NMR, 400 MHz, DMSO-$d_6$ $\delta$ = 12.14 (br s, 1H), 9.53 (s, 1H), 9.26 (d, J = 2.3 Hz, 1H), 9.07 (s, 1H), 8.88 (dd, J = 2.4, 8.6 Hz, 1H), 8.02 - 7.62 (m, 2H), 7.42 - 7.13 (m, 4H), 5.48 (q, J = 6.5 Hz, 1H), 1.56 (d, J = 6.5 Hz, 3H). [M+H]$^+$: 405.1. |
| 145 | 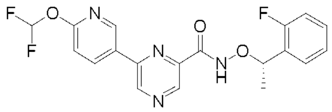 (*S**)-6-(6-(difluoromethoxy)pyridine -3-yl)-*N*-(1-(2-fluorophenyl)ethoxy)pyraz ine-2-carboxamide | $^1$H-NMR, 400 MHz, DMSO-$d_6$ $\delta$ = 12.14 (s, 1H) , 9.52 (s, 1H) , 9.26 (d, J = 2.3 Hz, 1H) , 9.07 (s, 1H) , 8.88 (dd, J = 2.5, 8.6 Hz, 1H) , 8.03 - 7.60 (m, 2H) , 7.42 - 7.12 (m, 4H) , 5.49 (q, J = 6.5 Hz, 1H) , 1.57 (d, J = 6.6 Hz, 3H). [M+H]$^+$: 405.1. |
| 146 | 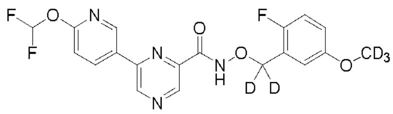 6-(6-(difluoromethoxy)pyridine -3-yl)-*N*-((2-fluoro-5-(methoxy-$d_3$)phenyl)methoxy-$d_2$)pyrazine-2-carboxamide | $^1$H-NMR, 400 MHz, DMSO-$d_6$ $\delta$ = 12.37 (br s, 1H), 9.54 (s, 1H), 9.27 (d, J = 2.3 Hz, 1H), 9.09 (s, 1H), 8.90 (dd, J = 2.4, 8.7 Hz, 1H), 8.05 - 7.53 (m, 1H), 7.29 (d, J = 8.8 Hz, 1H), 7.19 - 7.07 (m, 2H), 6.95 (td, J = 3.8, 8.8 Hz, 1H). [M+H]$^+$: 426.3. |

(continued)

| Compound № | Structure | $^1$H-NMR / MS(m/z) $[M+H]^+$ |
|---|---|---|
| 147 | 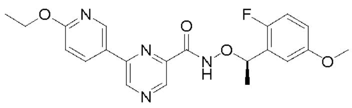<br>(*R**)-6-(6-ethoxypyridine-3-yl)-*N*-(1-(2-fluoro-5-methoxyphenyl)ethoxy)pyra zine-2-carboxamide | $^1$H-NMR, 400 MHz, DMSO-$d_6$ $\delta$ = 12.09 (s, 1 H), 9.45 (s, 1 H), 9.16 (d, J = 2.13 Hz, 1 H), 9.00 (s, 1 H) 8.66 (dd, J = 8.76, 2.38 Hz, 1 H), 7.24 (dd, J = 5.75, 3.25 Hz, 1 H), 7.09 (t, J = 9.44 Hz, 1 H), 6.96 (d, J = 8.75 Hz, 1 H), 6.88 (dt, J = 8.88, 3.63 Hz, 1 H), 5.46 (q, J = 6.46 Hz, 1 H), 4.40 (q, J = 7.00 Hz, 2 H), 3.78 (s, 3 H), 1.54 (d, J = 6.50 Hz, 3 H), 1.36 (t, J = 7.00 Hz, 3 H) .<br>$[M+H]^+$: 412.15. |
| 148 | 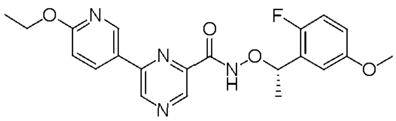<br>(*S**)-6-(6-ethoxypyridine-3-yl)-*N*-(1-(2-fluoro-5-methoxyphenyl)ethoxy)pyra zine-2-carboxamide | $^1$H-NMR, 400 MHz, DMSO-$d_6$ $\delta$ = 12.09 (s, 1 H), 9.45 (s, 1 H), 9.17 (d, J = 2.25 Hz, 1 H), 9.00 (s, 1 H), 8.66 (dd, J = 8.76, 2.50 Hz, 1 H), 7.24 (dd, J = 5.88, 3.13 Hz, 1 H), 7.09 (t, J = 9.38 Hz, 1 H), 6.97 (d, J = 8.76 Hz, 1 H), 6.88 (dt, J = 9.04, 3.61 Hz, 1 H), 5.46 (d, J = 6.50 Hz, 1 H), 4.40 (q, J = 7.05 Hz, 2 H), 3.78 (s, 3 H), 1.54 (d, J = 6.50 Hz, 3 H), 1.36 (t, J = 7.00 Hz, 3 H) .<br>$[M+H]^+$: 412.15. |
| 149 | 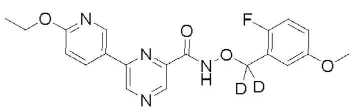<br>6-(6-ethoxypyridine-3-yl)-*N*-((2-fluoro-5-methoxyphenyl) methoxy-$d_2$)pyrazine-2-carboxamide | $^1$H-NMR, 400 MHz, DMSO-$d_6$ $\delta$ = 10.10 (s, 1 H), 9.27 (s, 1 H), 9.14 (s, 1 H), 8.76 (d, J = 2.25 Hz, 1 H), 8.15 (dd, J = 8.69, 2.56 Hz, 1 H), 7.03 - 7.08 (m, 1 H), 6.99 - 7.03 (m, 1 H), 6.84 - 6.91 (m, 2 H), 4.45 (q, J = 7.05 Hz, 2 H), 3.80 (s, 3 H), 1.44 (t, J = 7.07 Hz, 3 H).<br>$[M+H]^+$: 400.15. |
| 150 | 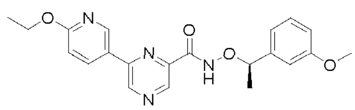<br>(*R**)-6-(6-ethoxypyridine-3-yl)-*N*-(1-(3-methoxyphenyl)ethoxy)pyrazine-2-carboxamide | |
| 151 | 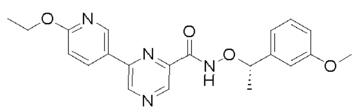<br>(*S**)-6-(6-ethoxypyridine-3-yl)-*N*-(1-(3-methoxyphenyl)ethoxy)pyrazine-2-carboxamide | |
| 152 | 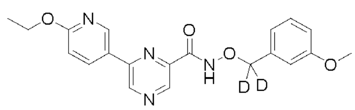<br>6-(6-ethoxypyridine-3-yl)-*N*-((3-methoxyphenyl)methoxy-$d_2$)pyrazine-2-carboxamide | |
| 153 | 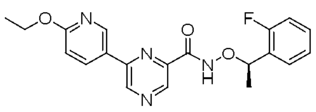<br>(*R**)-6-(6-ethoxypyridine-3-yl)-*N*-(1-(2-fluorophenyl)ethoxy)pyrazine-2-carboxamide | |

(continued)

| Compound Nº | Structure | $^1$H-NMR / MS(m/z) [M+H]$^+$ |
|---|---|---|
| 154 | 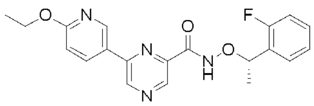 (*S**)-6-(6-ethoxypyridine-3-yl)-*N*-(1-(2-fluorophenyl)ethoxy)pyrazine-2-carboxamide | |
| 155 | 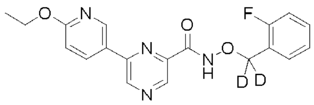 6-(6-ethoxypyridine-3-yl)-*N*-((2-fluorophenyl)methoxy-*d*$_2$)pyrazine-2-carboxamide | |
| *(R*) (S*) = Relative stereochemistry.* | | |

**COMPOUNDS IN FORMULA IB**

**EXAMPLE 2. 6-(6-ETHOXYPYRIDINE-3-IL)-N-((2-FLUORO-5-METHOXYBENZYLOXY-N-METHYLPYRAZINE-2-CARBOXAMIDE (156)**

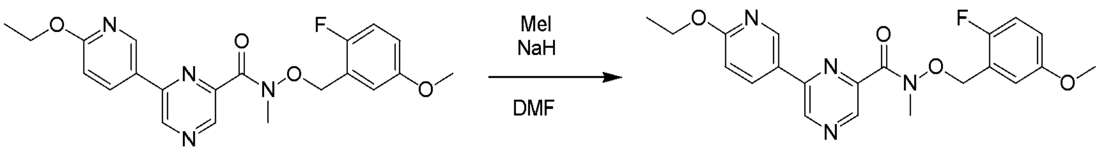


In a flask containing 3.57 mmol of 6-(6-ethoxypyridin-3-yl)-*N*-((2-fluoro-5-methoxybenzyl)oxy)-*N*-methylpyrazine-2-carboxamide, dissolved in 50 mL DMF, 4.28 mmol NaH (60% purity) at 0 °C were added. The mixture was heated to 20 °C for 2 hours, and then 9.86 mmol of iodomethane was added. Subsequently, the reaction mixture was kept in agitation at 20 °C for 2 hours. After total consumption of the starting reagent (monitored by CCF), the reaction mixture was diluted with a solution of $NH_4Cl$. The product was filtered and washed with ethanol (10 mL). 440 mg (30% yield) of the product of interest were obtained.

**COMPOUNDS -156 - 159**

**TABLE 12. COMPOUNDS OBTAINED FROM THE CORRESPONDING REACTANTS FOLLOWING THE METHOD IN EXAMPLE 2.**

| Compound No. | Structure | $^1$H-NMR / MS (m/z) [M+H]$^+$ |
|---|---|---|
| 156 | 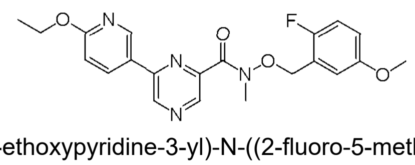 6-(6-ethoxypyridine-3-yl)-N-((2-fluoro-5-methoxybenzyl)oxy)-N-methylpyrazine-2-carboxamide | $^1$H-NMR, 400 MHz, DMSO-d$_6$ $\delta$ = 9.33 (s, 1H), 8.93 (d, J = 1.8 Hz, 1H), 8.65 (br s, 1H), 8.37 (dd, J = 2.1, 8.7 Hz, 1H), 6.96 (br d, J = 8.7 Hz, 2H), 6.77 (br s, 1H), 6.67 - 6.46 (m, 1H), 4.99 (br s, 2H), 4.39 (q, J = 7.0 Hz, 2H), 3.55 (br s, 3H), 3.42 (s, 3H), 1.35 (t, J = 7.0 Hz, 3H). [M+H]$^+$: 413.1. |

(continued)

| Compound No. | Structure | $^1$H-NMR / MS (m/z) [M+H]$^+$ |
|---|---|---|
| 157 | 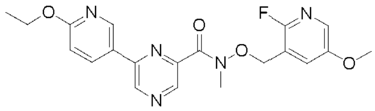 6-(6-ethoxypyridine-3-yl)-*N*-((2-fluoro-5-methoxypyridine-3-yl)methoxy)-*N*-methylpyrazine-2-carboxamide | $^1$H-NMR, 400 MHz, DMSO-$d_6$ $\delta$ = 9.33 (s, 1H), 8.91 (d, J = 1.3 Hz, 1H), 8.67 (br s, 1H), 8.36 (dd, J = 1.6, 8.7 Hz, 1H), 7.80 - 7.54 (m, 1H), 7.45 - 7.15 (m, 1H), 6.96 (d, J = 8.6 Hz, 1H), 5.01 (s, 2H), 4.40 (q, J = 7.0 Hz, 2H), 3.67 (br s, 3H), 3.43 (s, 3H), 1.36 (t, J = 7.1 Hz, 3H). [M+H]$^+$: 414.1. |
| 158 | 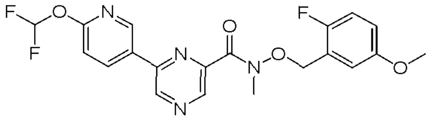 6-(6-(difluoromethoxy)pyridine-3-yl)-*N*-((2-fluoro-5-methoxybenzyl)oxy)-*N*-methylpyrazine-2-carboxamide | $^1$H-NMR, 400 MHz, DMSO-$d_6$ $\delta$ = 9.40 (s, 1H), 9.00 (d, J = 2.1 Hz, 1H), 8.72 (br s, 1H), 8.57 (dd, J = 2.3, 8.6 Hz, 1H), 7.81 (t, J = 72.5 Hz, 1H), 7.28 (d, J = 8.7 Hz, 1H), 7.03 - 6.51 (m, 3H), 4.98 (br s, 2H), 3.55 (br s, 3H), 3.43 (s, 3H). [M+H]$^+$: 435.1. |
| 159 | 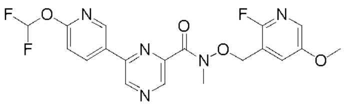 6-(6-(difluoromethoxy)pyridine-3-yl)-*N*-((2-fluoro-5-methoxypyridine-3-yl)methoxy)-*N*-methylpyrazine-2-carboxamide | $^1$H-NMR, 400 MHz, DMSO-$d_6$ $\delta$ = 9.41 (s, 1H), 8.99 (d, J = 1.9 Hz, 1H), 8.75 (br s, 1H), 8.56 (dd, J = 2.0, 8.6 Hz, 1H), 8.04 - 7.76 (m, 1H), 7.63 (s, 1H), 7.28 (d, J = 8.6 Hz, 2H), 5.00 (br s, 2H), 3.67 (br s, 3H), 3.44 (s, 3H). [M+H]$^+$: 436.1. |

**EXAMPLE 3. TESTS *IN VITRO:***

Biological assays were performed on Chinese hamster ovary (CHO) cells that express human sodium channels Nav 1.8 or Nav 1.7 in a stable manner.

The experimental protocol *voltage-clamp* using the whole cell configuration was established on the automated ScreenPatch® 384P platform (SP384PE, Nanion Technologies, Livingston, NJ) and the results were recorded with a Nanion 384-well Patch Clamp chip (NPC) (Nanion Technologies, Livingston, NJ).

Formula I compounds were diluted in eight concentrations in the extracellular solution composed of saline saline solution, buffered with HEPES (mM): NaCl, 137; KCl, 4; $CaCl_2$, 3,8; $MgCl_2$, 1; HEPES, 10; Glucose, 10; pH 7.4. The extracellular solution is composed of (mM) CsCl, 50; CsF, 90; $MgCl_2$, 5; EGTA, 5; HEPES, 10; pH 7.2. The duration of exposure of each compound with cells expressing Nav 1.7 or Nav 1.8 was at least five minutes and the assays were performed at room temperature.

The measurements of the sodium currents of Nav 1.8 and Nav 1.7 were obtained using the voltage protocol described below.

A holding voltage of -100 mV was established followed by an inactivation voltage step at -40 mV for 8 seconds, followed by a step of -100 mV for 20 ms, followed by a 20 ms step for 10 mV for Nav 1.8 or 0 mV for Nav 1.7 (TP1A) before returning to the holding voltage of -100 mV.

The protocol was repeated at a frequency of 0.05 Hz and the amplitude of the current was quantified throughout the recording of the TP1A phase. The variation in the amplitude of the peak current was evaluated according to the formula described below, after exposing the cells expressing the channels to each concentration of the different compounds:

$$\% \text{ Block} = (1 - (I_{TP1A,molecule} / I_{TP1A,basal}) \times 100\%,$$

wherein $I_{TP1A,basal}$ and $I_{TP1A,molecule}$ represent the peaks of sodium current input into TP1A before exposure to the compound and in the presence of the compound, respectively.

The decrease in the amplitude of the peak current, after the exposure of the cells to the compounds, was used to calculate the percentage of relative blockage of the channels in relation to the positive control according to the formula below:

$$\% \text{ Block}' = 100\% - ((\% \text{ Block} - \% \text{ CP}) * (100\% / (\%V - \% \text{ CP})),$$

wherein %V and %CP represent the means of the values of current inhibition with a vehicle (DMSO) and positive controls, respectively. The sodium currents of the positive control were considered as 100%.

$$\% \text{ Block}' = (100\% / [1 + ([\text{Test}] / CI_{50})^N],$$

wherein [Test] represents the concentration of the molecule evaluated, $IC_{50}$ is the concentration of the compound that generates half of the maximum inhibition, N is the Hill coefficient, and % Blockage' is the percentage of sodium channel current (Nav 1.8 and Nav 1.7) blocked at each concentration of the molecule evaluated. Data were obtained by nonlinear regression (*nonlinear least squares*) with XLfit for Excel (Microsoft, Redmond, WA).

The compounds were tested in at least one assay to obtain the value of $CI_{50}$. For compounds that were tested in two or more assays, the results are described as the mean of the $CI_{50}$ values.

Table 5 shows the efficacy *in vitro* of selected compounds against Nav1.8 and Nav1.7. $CI_{50}$ values less than 500 nM are represented with the legend (+++); $CI_{50}$ values between 500 nM and 1000 nM are represented with the legend (++), $CI_{50}$ values greater than 1000 nM are demonstrated with the symbology (+).

**TABLE 13. $CI_{50}$ VALUES OF THE COMPOUNDS OF THIS INVENTION IN NAV 1.8 AND NAV 1.7 CHANNELS.**

| Compound № | Nav1.8 $IC_{50}$ (nM) | Nav1.7 $IC_{50}$ (nM) |
|---|---|---|
| 1 | + | + |
| 2 | +++ | + |
| 3 | +++ | +++ |
| 4 | +++ | ++ |
| 5 | +++ | + |
| 6 | + | + |
| 7 | + | + |
| 8 | + | + |
| 9 | +++ | + |
| 10 | +++ | + |
| 11 | +++ | ++ |
| 12 | +++ | + |
| 13 | +++ | + |
| 14 | +++ | + |
| 15 | +++ | +++ |
| 16 | + | + |
| 17 | +++ | + |
| 18 | +++ | ++ |
| 19 | + | + |
| 20 | + | + |
| 21 | + | + |
| 22 | + | + |
| 23 | + | + |
| 24 | + | + |
| 25 | ++ | + |
| 26 | +++ | ++ |
| 27 | +++ | + |

(continued)

| Compound № | Nav1.8 $IC_{50}$ (nM) | Nav1.7 $IC_{50}$ (nM) |
|---|---|---|
| 28 | +++ | + |
| 29 | + | + |
| 30 | +++ | + |
| 31 | ++ | + |
| 32 | ++ | + |
| 33 | +++ | + |
| 34 | + | + |
| 35 | +++ | + |
| 36 | +++ | + |
| 37 | +++ | + |
| 38 | +++ | + |
| 39 | +++ | + |
| 40 | +++ | + |
| 41 | +++ | + |
| 42 | +++ | + |
| 43 | + | + |
| 44 | + | + |
| 45 | + | + |
| 46 | + | + |
| 47 | + | + |
| 48 | + | + |
| 49 | + | + |
| 50 | + | + |
| 51 | + | + |
| 52 | + | + |
| 53 | + | + |
| 54 | + | + |
| 55 | +++ | ++ |
| 56 | +++ | ++ |
| 57 | + | + |
| 58 | + | + |
| 59 | + | + |
| 60 | +++ | ++ |
| 61 | +++ | + |
| 62 | + | + |
| 63 | + | + |
| 64 | + | + |
| 65 | + | + |
| 66 | + | + |

(continued)

| Compound № | Nav1.8 $IC_{50}$ (nM) | Nav1.7 $IC_{50}$ (nM) |
|---|---|---|
| 67 | +++ | + |
| 68 | + | + |
| 69 | + | + |
| 70 | +++ | ++ |
| 71 | +++ | +++ |
| 72 | +++ | +++ |
| 73 | +++ | + |
| 74 | +++ | + |
| 75 | +++ | +++ |
| 76 | +++ | ++ |
| 77 | + | + |
| 78 | + | + |
| 79 | + | + |
| 80 | ++ | + |
| 81 | + | + |
| 82 | + | + |
| 83 | + | + |
| 84 | +++ | + |
| 85 | + | + |
| 86 | +++ | + |
| 87 | +++ | + |
| 88 | + | + |
| 89 | +++ | + |
| 90 | ++ | + |
| 91 | + | + |
| 92 | +++ | + |
| 93 | + | + |
| 94 | + | + |
| 95 | ++ | + |
| 96 | +++ | +++ |
| 97 | +++ | + |
| 98 | + | + |
| 99 | +++ | + |
| 100 | +++ | + |
| 101 | + | + |
| 102 | +++ | + |
| 103 | ++ | + |
| 104 | +++ | + |
| 105 | ++ | + |

(continued)

| Compound № | Nav1.8 $IC_{50}$ (nM) | Nav1.7 $IC_{50}$ (nM) |
|---|---|---|
| 106 | + | + |
| 107 | + | + |
| 108 | + | + |
| 109 | +++ | + |
| 110 | +++ | ++ |
| 111 | + | + |
| 112 | +++ | + |
| 113 | +++ | + |
| 114 | ++ | + |
| 115 | + | + |
| 116 | +++ | + |
| 117 | + | + |
| 118 | +++ | + |
| 119 | +++ | + |
| 120 | + | + |
| 121 | +++ | + |
| 122 | + | + |
| 123 | + | + |
| 124 | + | + |
| 125 | + | + |

From these results, the blocking activity of Nav 1.7 and/or 1.8 is proven, whose application is readily performed by those versed in the technique in pharmaceutical compositions, which may comprise one or more of the aforementioned Formula I compounds, kits, in addition to uses in the treatment of pain-related pathologies.

In particular, these results indicate the possibility of using Formula (I) compounds in the preparation of drugs for the treatment of conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that several modifications can be made by a person skilled in the art without departing from the scope of this invention. Consequently, the present invention should not be regarded as limited to the illustrative specifications described in this application.

## Claims

1. COMPOUND, **characterized by** being of Formula (I)

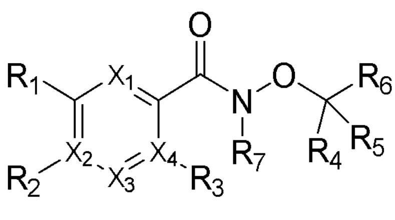

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, COH, or nitrogen wherein
- when at least one of the substituents $X_2$, $X_4$ is nitrogen or CH, the corresponding R ($R_2$, $R_3$) is null,
- $R_1$ is:

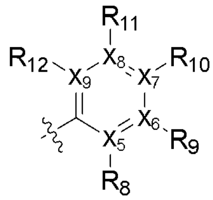
,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_8$-$R_{12}$) is null,
- $R_8$, $R_9$, $R_{10}$, $R_{11}$, and $R_{12}$ are independently selected from the group consisting of hydrogen, halogen, linear or branched $C_1$-$C_6$ alkyl, linear or branched $C_1$-$C_6$ alkoxy, haloalkoxy $C_1$-$C_6$ linear or branched, hydroxy, azetidinyloxy, azetidinylalkoxy $C_1$-$C_3$, alkylazetidinyloxy $C_1$-$C_6$, alkylazetidinylalkoxy $C_1$-$C_6$, alkylsulfinyl $C_1$-$C_6$, alkoxy $C_1$-$C_2$ containing but not limited to one or more isotopically enriched atoms such as hydrogen isotope $^2H$;
- $R_2$ and $R_3$ are independently selected from the group consisting of hydrogen, halogen, amine, hydroxy, $C_1$-$C_6$ alkoxy linear or branched, alkyl $C_1$-$C_6$ linear or branched;
- $R_4$ and $R_5$ are independently selected from the group consisting of hydrogen, deuterium, haloalkyl $C_1$-$C_6$ linear or branched, alkyl $C_1$-$C_6$ linear or branched;
- $R_6$ is selected from the group consisting of aryl, pyridinyl, or substituted pyridinyl, or $R_{13}$, or $R_{14}$, wherein
- $R_{13}$ is:

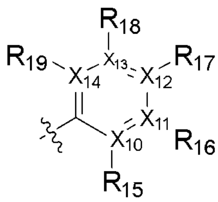
,

wherein:

- $X_{10}$, $X_{11}$, $X_{12}$, $X_{13}$ and $X_{14}$ are independently selected from the group consisting of carbon, CH or nitrogen;
- when at least one of the substituents $X_{10}$-$X_{14}$ is nitrogen or CH, the corresponding R ($R_{13}$-$R_{19}$) is null,
- $R_{13}$ $R_{16}$, $R_{17}$, $R_{18}$, and $R_{19}$ are independently selected from the group consisting of hydrogen, halogen, hydroxy, alkyl linear or branched $C_1$-$C_4$, cycloalkyl $C_3$-$C_7$, $C_1$-$C_6$ alkoxy linear or branched, cycloalkoxy $C_3$-$C_6$, hydroxyalkyl $C_{1}$-$C_6$ linear or branched, aminoalkyl $C_1$-$C_6$ linear or branched, alkoxy $C_1$-$C_2$ containing one or more isotopically enriched atoms, such as hydrogen isotope $^2H$, but not limited to the same;
- $R_{14}$ is selected from the group consisting of:

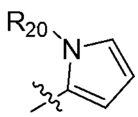
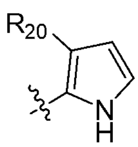
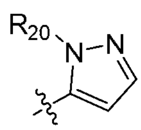
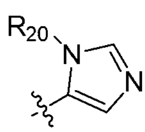
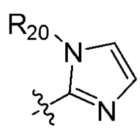

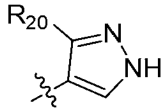 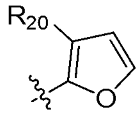 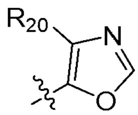 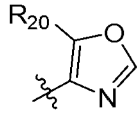 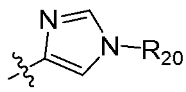

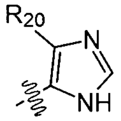 ,

wherein $R_{20}$ is independently selected from the group consisting of hydrogen, halogen, hydroxy, hydroxyalkyl $C_{1-}C_6$ linear or branched, alkyl $C_1$-$C_6$ linear or branched, alkoxy $C_1$-$C_6$ linear or branched, cycloalkoxy $C_3$-$C_7$ saturated or unsaturated, haloalkoxy $C_1$-$C_6$ linear or branched, nitrile, carboxyl, carboxyalkyl ester $C_1$-$C_6$, amine, *N*-alkylamine, *N,N*-dialkylamine, aminoalkyl $C_{1-}C_6$ linear or branched, sulfonamide, alkylsulfonylamide $C_1$-$C_6$, *N*,,*N*-dialkylsulfonylamide $C_1$-$C_6$, sulfonyl, alkylsulfonyl $C_1$-$C_6$, sulfinyl, alkylsulfinyl $C_1$-$C_6$, azetidiniloxy, azetidinylalkoxy $C_1$-$C_6$, linear or branched alkoxy $C_1$-$C_6$, hydroxyazetidinyl;

- $R_7$, is independently selected from the group consisting of hydrogen and alkyl $C_1$-$C_6$ linear or branched.

**2.** COMPOUND in accordance with claim 1, **characterized by** be selected from the group consisting of:

*N*-((2-fluoro-5-methoxybenzyl)oxy)-6-(4-hydroxyphenyl)pyrazine-2-carboxamide (Compound 1);
*N*-((2-fluoro-5-methoxybenzyl)oxy)-6-(4-methoxyphenyl)pyrazine-2-carboxamide (Compound 2);
*N*-((2-fluoro-5-methoxybenzyl)oxy)-6-(4-isopropoxyphenyl)pyrazine-2-carboxamide (Compound 3);
6-(4-ethoxyphenyl)-*N*-((2-fluorobenzyl)oxy)pyrazine-2-carboxamide (Compound 4);
6-(4-ethoxyphenyl)-*N*-((3-methoxybenzyl)oxy)pyrazine-2-carboxamide (Compound 5);
6-(4-ethoxyphenyl)-*N*-((2-hydroxybenzyl)oxy)pyrazine-2-carboxamide (Compound 6);
6-(4-ethoxyphenyl)-*N*-((3-hydroxybenzyl)oxy)pyrazine-2-carboxamide (Compound 7);
6-(4-ethoxyphenyl)-*N*-((5-methoxypyridine-3-yl)methoxy)pyrazine-2-carboxamide (Compound 8);
6-(4-ethoxyphenyl)-*N*-((2-fluoro-5-methoxybenzyl)oxy)pyrazine-2-carboxamide (Compound 9);
6-(4-ethoxyphenyl)-*N*-((2-fluoro-5-isopropoxybenzyl)oxy)pyrazine-2-carboxamide (Compound 10);
*N*-((2-chloro-5-methoxybenzyl)oxy)-6-(4-ethoxyphenyl)pyrazine-2-carboxamide (Compound 11);
6-(4-ethoxyphenyl)-*N*-((2-fluoro-5-methoxypyridine-3-yl)methoxy)pyrazine-2-carboxamide (Compound 12);
6-(4-ethoxyphenyl)-*N*-((5-fluoro-2-methoxypyridine-4-yl)methoxy)pyrazine-2-carboxamide (Compound 13);
6-(4-ethoxyphenyl)-*N*-((2-fluoro-5-hydroxybenzyl)oxy)pyrazine-2-carboxamide (Compound 14);
*N*-((2-cyclopropyl-5-methoxybenzyl)oxy)-6-(4-ethoxyphenyl)pyrazine-2-carboxamide (Compound 15);
6- (4-ethoxyphenyl)-*N*-((2-hydroxy-5-methoxybenzyl)oxy)pyrazine-2-carboxamide (Compound 16);
*N*-((5-(1-aminoethyl)-2-fluorobenzyl)oxy)-6-(4-ethoxyphenyl)pyrazine-2-carboxamide (Compound 17);
6-(4-ethoxyphenyl)-*N*-((5-methoxy-2-methylpyridine-3-yl)methoxy)pyrazine-2-carboxamide (Compound 18);
6-(4-ethoxyphenyl)-*N*-((5-methoxy-2-methylbenzyl)oxy)pyrazine-2-carboxamide (Compound 19);
*N*-((3,5-dimethoxybenzyl)oxy)-6-(4-ethoxyphenyl)pyrazine-2-carboxamide (Compound 20);
*N*-((3,5-dimethylbenzyl)oxy)-6-(4-ethoxyphenyl)pyrazine-2-carboxamide (Compound 21);
6-(4-(azetidine-3-yloxy)phenyl)-*N*-((2-fluoro-5-methoxybenzyl)oxy)pyrazine-2-carboxamide (Compound 22);
6-(4-(azetidine-3-ylmethoxy)phenyl)-*N*-((2-fluoro-5-methoxybenzyl)oxy)pyrazine-2-carboxamide (Compound 23);
6-(4-(2-(azetidine-3-yl)ethoxy)phenyl)-*N*-((2-fluoro-5-methoxybenzyl)oxy)pyrazine-2-carboxamide (Compound 24);
*N*-((2-fluoro-5-methoxybenzyl)oxy)-6-(4-((1-methylazetidine-3-yl)oxy)phenyl)pyrazine-2-carboxamide (Compound 25);
*N*-((2-fluoro-5-methoxybenzyl)oxy)-6-(4-((1-methylazetidine-3-yl)methoxy)phenyl)pyrazine-2-carboxamide (Compound 26);
*N*-((2-fluoro-5-methoxybenzyl)oxy)-6-(4-(2-(1-methylazetidine-3-yl)ethoxy)phenyl)pyrazine-2-carboxamide (Compound 27);
6-(4-(difluoromethoxy)phenyl)-*N*-((5-methoxypyridine-3-yl)methoxy)pyrazine-2-carboxamide (Compound 28);
*N*-((2-fluoro-5-methoxybenzyl)oxy)-6-(4-(methylsulfinyl)phenyl)pyrazine-2-carboxamide (Compound 29);
*N*-((2-fluoro-5-methoxybenzyl)oxy)-6-(6-methoxypyridine-3-yl)pyrazine-2-carboxamide (Compound 30);
*N*-((2-fluoro-5-methoxybenzyl)oxy)-6-(6-isopropoxypyridine-3-yl)pyrazine-2-carboxamide (Compound 31);

6-(6-ethoxypyridine-3-yl)-*N*-((2-fluorobenzyl)oxy)pyrazine-2-carboxamide (Compound 32);
6-(6-ethoxypyridine-3-yl)-*N*-((3-methoxybenzyl)oxy)pyrazine-2-carboxamide (Compound 33);
6-(6-ethoxypyridine-3-yl)-*N*-((2-hydroxybenzyl)oxy)pyrazine-2-carboxamide (Compound 34);
6-(6-ethoxypyridine-3-yl)-*N*-((3-hydroxybenzyl)oxy)pyrazine-2-carboxamide (Compound 35);
6-(6-ethoxypyridine-3-yl)-*N*-((5-methoxypyridine-3-yl)methoxy)pyrazine-2-carboxamide (Compound 36);
6-(6-ethoxypyridine-3-yl)-*N*-((2-fluoro-5-methoxybenzyl)oxy)pyrazine-2-carboxamide (Compound 37);
6-(6-ethoxypyridine-3-yl)-*N*-((2-fluoro-5-methoxypyridine-3-yl)methoxy)pyrazine-2-carboxamide (Compound 38);
6-(6-ethoxypyridine-3-yl)-*N*-((5-fluoro-2-methoxypyridine-4-yl)methoxy)pyrazine-2-carboxamide (Compound 39);
*N*-((5-(dimethylamino)-2-fluorobenzyl)oxy)-6-(6-ethoxypyridine-3-yl)pyrazine-2-carboxamide (Compound 40);
*N*-((5-(dimethylamino)-2-fluoropyridine-3-yl)methoxy)-6-(6-ethoxypyridine-3-yl)pyrazine-2-carboxamide (Compound 41);
*N*-((5-(dimethylamino)-2-fluoropyridine-3-yl)methoxy)-6-(6-ethoxypyridine-3-yl)pyrazine-2-carboxamide (Compound 42);
6-(6-ethoxypyridine-3-yl)-*N*-((2-fluoro-5-hydroxybenzyl)oxy)pyrazine-2-carboxamide (Compound 43);
6-(6-ethoxypyridine-3-yl)-*N*-((2-hydroxy-5-methoxybenzyl)oxy)pyrazine-2-carboxamide (Compound 44);
*N*-((5-(1-aminoethyl)-2-fluorobenzyl)oxy)-6-(6-ethoxypyridine-3-yl)pyrazine-2-carboxamide (Compound 45);
6-(6-ethoxypyridin-3-yl)-*N*-((5-methoxy-2-methylpyridine-3-yl)methoxy)pyrazine-2-carboxamide (Compound 46);
6-(6-ethoxypyridine-3-yl)-*N*-((5-methoxy-2-methylbenzyl)oxy)pyrazine-2-carboxamide (Compound 47);
6-(6-(ethoxy-d5)pyridine-3-yl)-*N*-((2-fluoro-5-methoxybenzyl)oxy)pyrazine-2-carboxamide (Compound 48);
6-(6-(ethoxy-2,2,2-d3)pyridine-3-yl)-*N*-((2-fluoro-5-methoxybenzyl)oxy)pyrazine-2-carboxamide (Compound 49);
6-(6-(ethoxy-1,1-d2)pyridine-3-yl)-*N*-((2-fluoro-5-methoxybenzyl)oxy)pyrazine-2-carboxamide (Compound 50);
6-(6-(ethoxy-1,1-d2)pyridine-3-yl)-*N*-((2-fluoro-5-methoxybenzyl)oxy)pyrazine-2-carboxamide (Compound 51);
6-(6-(ethoxy-2,2,2-d3)pyridine-3-yl)-*N*-((2-fluoro-5-(methoxy-d3)benzyl)oxy)pyrazine-2-carboxamide (Compound 52);
6-(6-(ethoxy-d5)pyridine-3-yl)-*N*-((2-fluoro-5-(methoxy-d3)benzyl)oxy)pyrazine-2-carboxamide (Compound 53);
6-(6-(ethoxy-d5)pyridine-3-yl)-*N*-((2-fluoro-5-(methoxy-d3)phenyl)methoxy-d2)pyrazine-2-carboxamide (Compound 54);
6-(6-ethoxypyridine-3-yl)-*N*-((2-fluoro-5-(methoxy-d3)benzyl)oxy)pyrazine-2-carboxamide (Compound 55);
6-(6-ethoxypyridine-3-yl)-*N*-((2-fluoro-5-(methoxy-d3)benzyl)oxy)pyrazine-2-carboxamide (Compound 56);
6-(6-ethoxypyridine-3-yl)-*N*-((5-methyloxazole-4-yl)methoxy)pyrazine-2-carboxamide (Compound 57);
6-(6-ethoxypyridine-3-yl)-*N*-((5-methyloxazole-4-yl)methoxy)pyrazine-2-carboxamide (Compound 58);
6-(6-ethoxypyridine-3-yl)-*N*-((4-methyl-1H-imidazole-5-yl)methoxy)pyrazine-2-carboxamide (Compound 59);
6-(6-ethoxypyridine-3-yl)-*N*-((1-methyl-1H-pyrrole-2-yl)methoxy)pyrazine-2-carboxamide (Compound 60);
6-(6-ethoxypyridine-3-yl)-*N*-((1-ethyl-1H-imidazole-2-yl)methoxy)pyrazine-2-carboxamide (Compound 61);
6-(6-ethoxypyridine-3-yl)-*N*-((1-ethyl-1H-pyrazole-5-yl)methoxy)pyrazine-2-carboxamide (Compound 62);
6-(6-ethoxypyridine-3-yl)-*N*-((1-ethyl-1H-pyrazole-5-yl)methoxy)pyrazine-2-carboxamide (Compound 63);
6-(6-ethoxypyridine-3-yl)-*N*-((1-ethyl-1H-pyrazole-5-yl)methoxy)pyrazine-2-carboxamide (Compound 64);
6-(6-ethoxypyridine-3-yl)-*N*-((4-methyloxazole-5-yl)methoxy)pyrazine-2-carboxamide (Compound 65);
6-(6-ethoxypyridine-3-yl)-*N*-((3-methoxyfuran-2-yl)methoxy)pyrazine-2-carboxamide (Compound 66);
6-(6-ethoxypyridine-3-yl)-*N*-((3-methyl-1H-pyrrole-2-yl)methoxy)pyrazine-2-carboxamide (Compound 67);
6-(6-ethoxypyridine-3-yl)-*N*-((3-methyl-1H-pyrrole-2-yl)methoxy)pyrazine-2-carboxamide (Compound 68);
6-(6-ethoxypyridine-3-yl)-*N*-((3-methyl-1H-pyrazole-4-yl)methoxy)pyrazine-2-carboxamide (Compound 69);
*N*-((2-fluorobenzyl)oxy)-6-(6-(trifluoromethoxy)pyridine-3-yl)pyrazine-2-carboxamide (Compound 70);
*N*-((3-methoxybenzyl)oxy)-6-(6-(trifluoromethoxy)pyridine-3-yl)pyrazine-2-carboxamide (Compound 71);
*N*-(pyridine-2-ylmethoxy)-6-(6-(trifluoromethoxy)pyridine-3-yl)pyrazine-2-carboxamide (Compound 72);
*N*-(pyridine-3-ylmethoxy)-6-(6-(trifluoromethoxy)pyridine-3-yl)pyrazine-2-carboxamide (Compound 73);
*N*-(pyridine-4-ylmethoxy)-6-(6-(trifluoromethoxy)pyridine-3-yl)pyrazine-2-carboxamide (Compound 74);
*N*-((2-fluoro-5-methoxybenzyl)oxy)-6-(6-(trifluoromethoxy)pyridine-3-yl)pyrazine-2-carboxamide (Compound 75);
6-(6-(difluoromethoxy)pyridine-3-yl)-*N*-((2-fluoro-5-methoxybenzyl)oxy)pyrazine-2-carboxamide (Compound 76);
6-(6-(difluoromethoxy)pyridine-3-yl)-*N*-((2-fluoro-5-methoxybenzyl)oxy)pyrazine-2-carboxamide (Compound 77);

6-(6-(difluoromethoxy)pyridine-3-yl)-*N*-((5-fluoro-2-methoxypyridine-4-yl)methoxy)pyrazine-2-carboxamide (Compound 78);
6-(6-(difluoromethoxy)pyridine-3-yl)-*N*-((5-fluoro-2-methoxypyridin-4-yl)methoxy)pyrazine-2-carboxamide (Compound 79);
6-(6-(difluoromethoxy)pyridine-3-yl)-*N*-((5-(dimethylamino)-2-fluorobenzyl)oxy)pyrazine-2-carboxamide (Compound 80);
6-(6-(difluoromethoxy)pyridine-3-yl)-*N*-((3-(1-hydroxyethyl)benzyl)oxy)pyrazine-2-carboxamide (Compound 81);
6-(6-(difluoromethoxy)pyridin-3-yl)-*N*-((2-fluoro-5-(methoxy-d3)benzyl)oxy)pyrazine-2-carboxamide (Compound 82);
*N*-((2-fluoro-5-methoxybenzyl)oxy)-6-(6-(methylsulfinyl)pyridine-3-yl)pyrazine-2-carboxamide (Compound 83);
6-(4-ethoxy-2-hydroxyphenyl)-*N*-((2-fluoro-5-methoxybenzyl)oxy)pyrazine-2-carboxamide (Compound 84);
6-(4-ethoxy-3-hydroxyphenyl)-*N*-((2-fluoro-5-methoxybenzyl)oxy)pyrazine-2-carboxamide (Compound 85);
6-(6-ethoxy-4-hydroxypyridine-3-yl)-*N*-((2-fluoro-5-methoxybenzyl)oxy)pyrazine-2-carboxamide (Compound 86);
6-(6-ethoxy-2-oxo-1,2-dihydropyridine-3-yl)-*N*-((2-fluoro-5-methoxybenzyl)oxy)pyrazine-2-carboxamide (Compound 87);
*N*-((3,5-dimethoxybenzyl)oxy)-6-(4-ethoxyphenyl)-5-hydroxypyrazine-2-carboxamide (Compound 88);
6-(4-ethoxyphenyl)-*N*-((2-fluoro-5-methoxybenzyl)oxy)-5-hydroxypyrazine-2-carboxamide (Compound 89);
*N*-((3,5-dimethoxybenzyl)oxy)-6-(4-ethoxyphenyl)-3-hydroxypyrazine-2-carboxamide (Compound 90);
6-(4-ethoxyphenyl)-*N*-((2-fluoro-5-methoxybenzyl)oxy)-3-hydroxypyrazine-2-carboxamide (Compound 91);
6-(6-ethoxypyridine-3-yl)-*N*-((2-fluoro-5-methoxybenzyl)oxy)-5-hydroxypyrazine-2-carboxamide (Compound 92);
6-(6-ethoxypyridine-3-yl)-*N*-((2-fluoro-5-methoxybenzyl)oxy)-3-hydroxypyrazine-2-carboxamide (Compound 93);
5-amino-6-(6-ethoxypyridine-3-yl)-*N*-((2-fluoro-5-methoxybenzyl)oxy)pyrazine-2-carboxamide (Compound 94);
3-amino-6-(6-ethoxypyridine-3-yl)-*N*-((2-fluoro-5-methoxybenzyl)oxy)pyrazine-2-carboxamide (Compound 95);
6-(6-ethoxypyridine-3-yl)-5-fluoro-*N*-((2-fluoro-5-methoxybenzyl)oxy)pyrazine-2-carboxamide (Compound 96);
6-(6-ethoxypyridine-3-yl)-3-fluoro-*N*-((2-fluoro-5-methoxybenzyl)oxy)pyrazine-2-carboxamide (Compound 97);
6'-ethoxy-*N*-((2-fluoro-5-methoxybenzyl)oxy)-[2,3'-bipyridine]-6-carboxamide (Compound 98);
6'-ethoxy-*N*-((2-fluoro-5-methoxypyridine-3-yl)methoxy)-[2,3'-bipyridine]-6-carboxamide (Compound 99);
6'-ethoxy-*N*-((2-fluoro-5-methoxybenzyl)oxy)-[3,3'-bipyridine]-5-carboxamide (Compound 100);
2-(6-ethoxypyridine-3-yl)-*N*-((2-fluoro-5-methoxybenzyl)oxy)pyrimidine-4-carboxamide (Compound 101);
2-(6-ethoxypyridine-3-yl)-*N*-((2-fluoro-5-methoxypyridine-3-yl)methoxy)pyrimidine-4-carboxamide (Compound 102);
4-(6-ethoxypyridine-3-yl)-*N*-((2-fluoro-5-methoxybenzyl)oxy)pyrimidine-2-carboxamide (Compound 103);
4-(6-ethoxypyridine-3-yl)-*N*-((2-fluoro-5-methoxybenzyl)oxy)pyrimidine-2-carboxamide (Compound 104);
4-(6-ethoxypyridine-3-yl)-*N*-((2-fluoro-5-methoxybenzyl)oxy)pyrimidine-2-carboxamide (Compound 105);
6'-(difluoromethoxy)-*N*-((2-fluoro-5-methoxypyridine-3-yl)methoxy)-[2,3'-bipyridine]-6-carboxamide (Compound 106);
6'-(difluoromethoxy)-*N*-((2-fluoro-5-methoxypyridine-3-yl)methoxy)-[2,3'-bipyridine]-6-carboxamide (Compound 107);
6'-(difluoromethoxy)-*N*-((2-fluoro-5-methoxypyridine-3-yl)methoxy)-[2,3'-bipyridine]-6-carboxamide (Compound 108);
6'-(difluoromethoxy)-*N*-((2-fluoro-5-methoxybenzyl)oxy)-[3,3'-bipyridine]-5-carboxamide (Compound 109);
2-(6-(difluoromethoxy)pyridine-3-yl)-*N*-((2-fluoro-5-methoxypyridine-3-yl)methoxy)pyrimidine-4-carboxamide (Compound 110);
2-(6-(difluoromethoxy)pyridine-3-yl)-*N*-((2-fluoro-5-methoxypyridine-3-yl)methoxy)pyrimidine-4-carboxamide (Compound 111);
2-(6-(difluoromethoxy)pyridine-3-yl)-*N*-((2-fluoro-5-methoxypyridine-3-yl)methoxy)pyrimidine-4-carboxamide (Compound 112);
3-amino-6-(6-(difluoromethoxy)pyridine-3-yl)-*N*-((2-fluoro-5-methoxypyridine-3-yl)methoxy)pyrazine-2-carboxamide (Compound 113);
3-amino-6-(6-(difluoromethoxy)pyridine-3-yl)-*N*-((2-fluoro-5-methoxypyridine-3-yl)methoxy)pyrazine-2-carboxamide (Compound 114);
5-amino-6-(6-(difluoromethoxy)pyridine-3-yl)-*N*-((2-fluoro-5-methoxybenzyl)oxy)pyrazine-2-carboxamide

(Compound 115);
6-(6-(difluoromethoxy)pyridine-3-yl)-3-fluoro-*N*-((2-fluoro-5-methoxybenzyl)oxy)pyrazine-2-carboxamide (Compound 116);
6-(6-(difluoromethoxy)pyridine-3-yl)-5-fluoro-*N*-((2-fluoro-5-methoxybenzyl)oxy)pyrazine-2-carboxamide (Compound 117);
3-(6-(difluoromethoxy)pyridine-3-yl)-*N*-((2-fluoro-5-methoxybenzyl)oxy)-1,2,4-triazine-5-carboxamide (Compound 118);
5-(6-(difluoromethoxy)pyridine-3-yl)-*N*-((2-fluoro-5-methoxybenzyl)oxy)-1,2,4-triazine-3-carboxamide (Compound 119);
*N*-((2-(3,5-dimethoxyphenyl)propan-2-yl)oxy)-6-(4-ethoxyphenyl)pyrazine-2-carboxamide (Compound 120);
*N*-(1-(3,5-dimethoxyphenyl)cyclopropoxy)-6-(4-ethoxyphenyl)pyrazine-2-carboxamide (Compound 121);
(*R*)-*N*-(1-(3,5-dimethoxyphenyl)ethoxy)-6-(4-ethoxyphenyl)pyrazine-2-carboxamide (Compound 122);
(*S*)-*N*-(1-(3,5-dimethoxyphenyl)ethoxy)-6-(4-ethoxyphenyl)pyrazine-2-carboxamide (Compound 123);
(*S*)-*N*-(1-(3,5-dimethoxyphenyl)ethoxy)-6-(4-ethoxyphenyl)pyrazine-2-carboxamide (Compound 124);
(*R*)-6-(4-ethoxyphenyl)-N-(1-(2-fluoro-5-methoxyphenyl)ethoxy)pyrazine-2-carboxamide (Compound 125);
(*S*)-6-(4-ethoxyphenyl)-*N*-(1-(2-fluoro-5-methoxyphenyl)ethoxy)pyrazine-2-carboxamide (Compound 126);
6-(4-ethoxyphenyl)-*N*-((2-fluoro-5-methoxyphenyl)methoxy-d2)pyrazine-2-carboxamide (Compound 127);
(*R*)-6-(4-ethoxyphenyl)-*N*-(1-(3-methoxyphenyl)ethoxy)pyrazine-2-carboxamide (Compound 128);
(*S*)-6-(4-ethoxyphenyl)-*N*-(1-(3-methoxyphenyl)ethoxy)pyrazine-2-carboxamide (Compound 129);
6-(4-ethoxyphenyl)-*N*-((3-methoxyphenyl)methoxy-d2)pyrazine-2-carboxamide (Compound 130);
(*R*)-6-(4-ethoxyphenyl)-*N*-(1-(2-fluorophenyl)ethoxy)pyrazine-2-carboxamide (Compound 131);
(*S*)-6-(4-ethoxyphenyl)-*N*-(1-(2-fluorophenyl)ethoxy)pyrazine-2-carboxamide (Compound 132);
6-(4-ethoxyphenyl)-*N*-((2-fluorophenyl)methoxy-d2)pyrazine-2-carboxamide (Compound 133);
6-(6-(difluoromethoxy)pyridin-3-yl)-*N*-((2-fluoro-5-(methoxy-d3)phenyl)methoxy-d2)pyrazine-2-carboxamide (compound 134);
(*R*)-6-(6-ethoxypyridine-3-yl)-*N*-(1-(2-fluoro-5-methoxyphenyl)ethoxy)pyrazine-2-carboxamide (Compound 135);
(*S*)-6-(6-ethoxypyridine-3-yl)-*N*-(1-(2-fluoro-5-methoxyphenyl)ethoxy)pyrazine-2-carboxamide (Compound 136);
6-(6-ethoxypyridine-3-yl)-*N*-((2-(2-fluoro-5-methoxyphenyl)propan-2-yl)oxy)pyrazine-2-carboxamide (Compound 137);
6-(6-ethoxypyridine-3-yl)-*N*-(1-(2-fluoro-5-methoxyphenyl)cyclopropoxy)pyrazine-2-carboxamide (Compound 138);
6-(6-ethoxypyridine-3-yl)-*N*-(1-(2-fluoro-5-methoxyphenyl)cyclopropoxy)pyrazine-2-carboxamide (Compound 139);
(*R*)-6-(6-ethoxypyridine-3-yl)-*N*-(1-(3-methoxyphenyl)ethoxy)pyrazine-2-carboxamide (Compound 140);
(*S*)-6-(6-ethoxypyridine-3-yl)-*N*-(1-(3-methoxyphenyl)ethoxy)pyrazine-2-carboxamide (Compound 141);
6-(6-ethoxypyridine-3-yl)-*N*-((3-methoxyphenyl)methoxy-d2)pyrazine-2-carboxamide (Compound 142);
(*R*)-6-(6-ethoxypyridine-3-yl)-*N*-(1-(2-fluorophenyl)ethoxy)pyrazine-2-carboxamide (Compound 143);
(*S*)-6-(6-ethoxypyridine-3-yl)-*N*-(1-(2-fluorophenyl)ethoxy)pyrazine-2-carboxamide (Compound 144);
6-(6-ethoxypyridine-3-yl)-*N*-((2-fluorophenyl)methoxy-d2)pyrazine-2-carboxamide (Compound 145);
(*R*)-6-(6-ethoxypyridine-3-yl)-*N*-(1-(2-fluoro-5-methoxyphenyl)ethoxy-2,2,2-d3)pyrazine-2-carboxamide (Compound 146);
(*S*)-6-(6-ethoxypyridine-3-yl)-*N*-(1-(2-fluoro-5-methoxyphenyl)ethoxy-2,2,2-d3)pyrazine-2-carboxamide (Compound 147);
6-(6-ethoxypyridine-3-yl)-*N*-((2-fluoro-5-methoxybenzyl)oxy)-*N*-methylpyrazine-2-carboxamide (Compound 148);
6-(6-ethoxypyridine-3-yl)-*N*-((2-fluoro-5-methoxypyridine-3-yl)methoxy)-*N*-methylpyrazine-2-carboxamide (Compound 149);
6-(6-(difluoromethoxy)pyridine-3-yl)-*N*-((2-fluoro-5-methoxybenzyl)oxy)-*N*-methylpyrazine-2-carboxamide (Compound 150);
6-(6-(difluoromethoxy)pyridine-3-yl)-*N*-((2-fluoro-5-methoxypyridine-3-yl)methoxy)-*N*-methylpyrazine-2-carboxamide (Compound 151);
6-(6-ethoxypyridine-3-yl)-N-((3-methoxyphenyl)methoxy-$d_2$)pyrazine-2-carboxamide (Compound 152);
(R*)-6-(6-ethoxypyridine-3-yl)-N-(1-(2-fluorophenyl)ethoxy)pyrazine-2-carboxamide (Compound 153);
(S*)-6-(6-ethoxypyridine-3-yl)-N-(1-(2-fluorophenyl)ethoxy)pyrazine-2-carboxamide (Compound 154);
6-(6-ethoxypyridine-3-yl)-N-((2-fluorophenyl)methoxy-d2)pyrazine-2-carboxamide (Compound 155);
6-(6-ethoxypyridine-3-yl)-N-((2-fluoro-5-methoxybenzyl)oxy)-N-methylpyrazine-2-carboxamide (Compound

156);
6-(6-ethoxypyridine-3-yl)-N-((2-fluoro-5-methoxypyridin-3-yl)methoxy)-N-methylpyrazine-2-carboxamide (Compound 157);
6-(6-(difluoromethoxy)pyridine-3-yl)-N-((2-fluoro-5-methoxybenzyl)oxy)-N-methylpyrazine-2-carboxamide (Compound 158); and
6-(6-(difluoromethoxy)pyridine-3-yl)-N-((2-fluoro-5-methoxypyridine-3-yl)methoxy)-N-methylpyrazine-2-carboxamide (Compound 159).

**3.** COMPOUND, according to claim 1 or 2, **characterized by** being a blocker of voltage-gated sodium channels Nav 1.7 and/or Nav 1.8.

**4.** PHARMACEUTICAL COMPOSITION, **characterized by** comprising a therapeutically effective amount of one or more compounds of Formula (I) or of a pharmaceutically acceptable salt, hydrate, solvate, and isomer thereof, as defined in any of claims 1 to 3, and one or more pharmaceutically acceptable excipients.

**5.** PHARMACEUTICAL COMPOSITION, according to claim 4, **characterized by** being formulated as an oral, sublingual, nasal, parenteral, injectable, submuscular, topical, transdermal, ocular, or rectal composition.

**6.** USE OF FORMULA (I) COMPOUND(S), as defined in any of claims 1 to 3, **characterized by** being to prepare a drug to treat pathologies related to neuropathic pain.

**7.** USE, according to claim 6, **characterized by** said pathologies being selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**8.** METHOD OF TREATMENT, PREVENTION, RELIEF, SUPPRESSION AND/OR CONTROL of pathologies related to neuropathic pain **characterized by the** administration of an effective amount of at least one compound of Formula (I) or a pharmaceutically acceptable salt, hydrate, solvate and isomer thereof, as defined in any of claims 1 to 3.

**9.** METHOD, according to claim 8, **characterized by** being for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**10.** METHOD, according to claim 8 or 9, **characterized by** administration of at least one compound of Formula (I) being selected from the group comprising oral, sublingual, nasal, parenteral, injectable, submuscular, topical, transdermal, ocular, and rectal routes.

**11.** PROCESS OF OBTAINING GENERAL FORMULA COMPOUND (I), as defined in any of the claims 1 to 3, **characterized by** comprising the steps:

(a) formation of the Formula III intermediate:

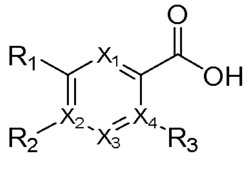

Formula III

from the hydrolysis reaction of a Formula IV intermediate:

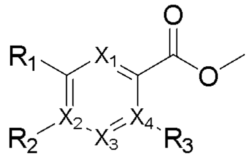

Formula IV

(b) obtaining Formula I compound;

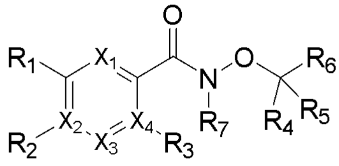

Formula (I)

from the reaction with the Formula II intermediates:

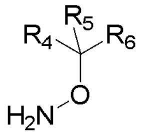

Formula II

and Formula III, with or without the presence of a catalyst and a suitable solvent;
wherein,

- $X_1$, $X_2$, $X_3$, and $X_4$ are independently selected from the group consisting of carbon, CH, COH, or nitrogen; wherein:
- when at least one of the substituents $X_2$, $X_4$ is nitrogen or CH, the corresponding R ($R_2$, $R_3$) is null,
- $R_1$ is:

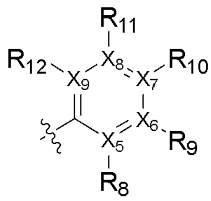

,

wherein:

- $X_5$, $X_6$, $X_7$, $X_8$, and $X_9$ are independently selected from the group consisting of carbon, CH, or nitrogen; wherein:

  - when at least one of the substituents $X_5$-$X_9$ is nitrogen or CH, the corresponding R ($R_8$-$R_{12}$) is null; and
  - $R_8$, $R_9$, $R_{10}$, $R_{11}$, and $R_{12}$ are independently selected from the group consisting of hydrogen, halogen, $C_1$-$C_6$ linear or branched alkyl, $C_1$-$C_6$ linear or branched alkoxy, hydroxy, haloalkoxy $C_1$-$C_6$ linear or branched, azetidinyloxy, azetidinylalkoxy $C_1$-$C_6$, alkylazetidinyloxy $C_1$-$C_6$, alkylsulfinyl, alkoxy $C_1$-$C_2$ containing one or more isotopically enriched atoms, such as hydrogen isotopes $^2H$, but not limited to the same;
  - $R_2$ and $R_3$ are independently selected from the group consisting of hydrogen, halogen, amine, hydroxy, $C_1$-$C_6$ alkoxy, and $C_1$-$C_4$ alkyl
  - $R_4$ and $R_5$ are independently selected from the group consisting of hydrogen, deuterium, haloalkyl $C_1$-$C_6$ linear or branched, alkyl $C_1$-$C_6$ linear or branched;
  - $R_6$ is selected from the group consisting of aryl, pyridinyl, or substituted pyridinyl, or $R_{13}$ or $R_{14}$:

    - $R_{13}$ is:

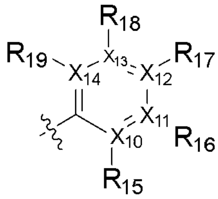

,

wherein:

- $X_{10}$, $X_{11}$, $X_{12}$, $X_{13}$, and $X_{14}$ are independently selected from the group consisting of carbon, CH, or nitrogen; wherein:

  - when at least one of the substituents $X_{10}$-$X_{14}$ is N or CH, the corresponding R ($R_{15}$-$R_{19}$) is null; and
  - $R_{15}$, $R_{16}$, $R_{17}$, $R_{18}$, and $R_{19}$ are independently selected from the group consisting of hydrogen, halogen, hydroxy, linear or branched $C_1$-$C_6$ alkyl, cycloalkyl $C_3$-$C_7$, alkoxy $C_1$-$C_6$ linear or branched, $C_3$-$C_6$ cycloalkoxy, hydroxyalkyl $C_{1-}C_6$ linear or branched, $C_1$-$C_6$ aminoalkyl linear or branched, alkoxy $C_1$-$C_2$ containing one or more isotopically enriched atoms, such as hydrogen isotope $^2H$, but not limited to the same;
  - $R_{14}$ is selected from the group consisting of:

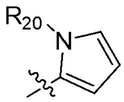
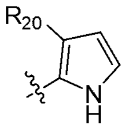
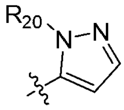
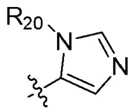
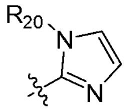

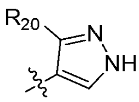
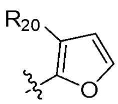
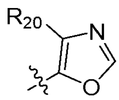
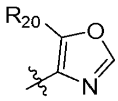
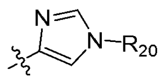

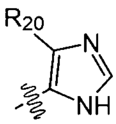

,

wherein $R_{20}$ is independently selected from the group consisting of hydrogen, halogen, hydroxy, hydroxyalkyl $C_{1-}C_6$ linear or branched, alkyl $C_1$-$C_6$ linear or branched, alkoxy $C_1$-$C_6$ linear or branched, cycloalkoxy $C_3$-$C_7$ saturated or unsaturated, haloalkoxy $C_1$-$C_6$ linear or branched, nitrile, carboxyl, carboxyalkyl ester $C_1$-$C_6$, amine, *N*-alkylamine, *N,N*-dialkylamine, aminoalkyl $C_{1-}C_6$ linear or branched, sulfonamide, alkylsulfonylamide $C_1$-$C_6$, *N,N*-dialkylsulfonylamide $C_1$-$C_6$, sulfonyl, alkyl sulfonyl $C_1$-$C_6$, sulfinyl, alkyl sulfinyl $C_1$-$C_6$, azetidinyloxy, azetidinylalkoxy $C_1$-$C_3$, linear or branched alkoxy $C_1$-$C_6$, hydroxyzetidinyl; and
- $R_7$, is independently selected from the group consisting of hydrogen and alkyl $C_1$-$C_6$ linear or branched.

**12.** PROCESS, according to claim 11, **characterized by** further comprising a compound formation step (c) of Formula (I) where $R_7$ is linear or branched $C_{1-}C_6$ alkyl from the nucleophilic substitution reaction of a compound obtained in step (b), with linear or branched $C_{1-}$ $C_6$ alkyl halides in the presence of inorganic base and polar aprotic solvents.

**13.** PROCESS, according to any of claims 11 to 12, **characterized by** in step (b) said catalyst being selected from concentrated hydrochloric acid, acetic acid, trifluoroacetic acid, formic acid, or combinations of these and said solvent being selected from dimethylformamide, dimethylsulfoxide, alcohols, or combinations thereof.

**14.** PROCESS, according to any of claims 12 to 13, **characterized by** in step (c) said inorganic base being selected from $K_2CO_3$ or NaH.

**15.** KIT, **characterized by** comprising a pharmaceutical composition as defined in claim 4 and an application device.

## INTERNATIONAL SEARCH REPORT

International application No.
**PCT/BR2024/050027**

### A. CLASSIFICATION OF SUBJECT MATTER

***C07C 233/64***(2006.01)i; **C07C 15/00**(2006.01)i; ***C07C 15/12***(2006.01)i; **C07D 213/00**(2006.01)i; **C07D 401/00**(2006.01)i; **C07D 403/00**(2006.01)i; **A61K 31/166**(2006.01)i; **A61K 31/435**(2006.01)i; **A61K 31/495**(2006.01)i; ***A61K 31/609***(2006.01)i; ***A61P 25/02***(2006.01)i; ***A61P 25/04***(2006.01)i; **A61P 29/00**(2006.01)i

CPC: C07C 233/64, C07C 15/00, C07C 15/12, C07D 213/00, C07D 401/00; C07D 403/00, A61K 31/166, A61K 31/435, A61K 31/495, A61K 31/609; A61P 25/02, A61P 25/04, A61P 29/00

According to International Patent Classification (IPC) or to both national classification and IPC

### B. FIELDS SEARCHED

Minimum documentation searched (classification system followed by classification symbols)

C07C 233/64; C07C 15/00; C07C 15/12; C07D 213/00; C07D 401/00; C07D 403/00; A61K 31/166; A61K 31/435; A61K 31/495; A61K 31/609; A61P 25/02; A61P 25/04; A61P 29/00

CPC: C07C 233/64, C07C 15/00, C07C 15/12, C07D 213/00, C07D 401/00; C07D 403/00, A61K 31/166, A61K 31/435, A61K 31/495, A61K 31/609; A61P 25/02, A61P 25/04, A61P 29/00

Documentation searched other than minimum documentation to the extent that such documents are included in the fields searched

Database INPI-BR; Periódicos Capes

Electronic data base consulted during the international search (name of data base and, where practicable, search terms used)

Derwent Innovation; REGISTRY, CAPLUS, MARPAT (STN®); ESPACENET

### C. DOCUMENTS CONSIDERED TO BE RELEVANT

| Category* | Citation of document, with indication, where appropriate, of the relevant passages | Relevant to claim No. |
|---|---|---|
| X | WO 2012129562 A2 (SCRIPPS RESEARCH INST [US]) 27 September 2012 (2012-09-27) See abstract; paragraphs 0002, 0076 and 0284; claims. | 1-7, 11-15 |
| X | WO 2015048547 A2 (RIGEL PHARMACEUTICALS INC [US]) 02 April 2015 (2015-04-02) See abstract; description, paragraphs 0214-0234; claims. | 1-7, 11-15 |
| X | WO 03051366 A2 (ABBOTT LAB [US]) 26 June 2003 (2003-06-26) See abstract; description, pages 25 and 26; claims. | 1-7, 11-15 |
| X<br>A | WO 2006006569 A1 (NIHON NOHYAKU CO LTD [JP]) 19 January 2006 (2006-01-19) See abstract and claims. | 1-5, 11-15<br>6-7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **29 April 2024** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Rodinelli OLIVEIRA** |
| Telephone No. **(55 21) 3037-3742, 3037-3984** | Telephone No. **+55 21 3037 4528 - 3037 3319** |

Form PCT/ISA/210 (second sheet) (July 2022)

**INTERNATIONAL SEARCH REPORT**

International application No.
**PCT/BR2024/050027**

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2012151567 A1 (ST JUDE CHILDRENS RES HOSPITAL) 08 November 2012 (2012-11-08)<br>See abstract; description, pages 155, 158, 159, 160, 161, 168 and 198; claims. | 1-5, 11-15<br>6-7 |
| X<br>A | WO 2006065204 A1 (ASTRAZENECA AB [SE]) 22 June 2006 (2006-06-22)<br>See abstract and claims. | 1-5, 11-15<br>6-7 |
| X<br>A | WO 0151456 A2 (TULARIK INC [US]) 19 July 2001 (2001-07-19)<br>See abstract and claims. | 1-5, 11-15<br>6-7 |
| X<br>A | WO 2008096005 A1 (BASF SE [DE]) 14 August 2008 (2008-08-14)<br>See abstract and claims. | 1-5, 11-15<br>6-7 |
| X<br>A | WO 2010099279 A1 (DOW AGROSCIENCES LLC [US]) 02 September 2010 (2010-09-02)<br>See abstract and claims. | 1-5, 11-15<br>6-7 |
| X<br>A | JP 2004203871 A (YAMANOUCHI PHARMA CO LTD) 22 July 2004 (2004-07-22)<br>See abstract and claims. | 1-5, 11-15<br>6-7 |
| X<br>A | US 4607053 A (SQUIBB & SONS INC [US]) 19 August 1986 (1986-08-19)<br>See abstract and claims. | 1-5, 11-15<br>6-7 |
| X<br>A | CN 108503501 A (UNIV ZHEJIANG TECHNOLOGY) 07 September 2018 (2018-09-07)<br>See abstract and claims. | 1-5, 11-15<br>6-7 |
| A | WO 2020138271 A1 (RAQUALIA PHARMA INC [JP]) 02 July 2020 (2020-07-02)<br>The whole document | 1-7, 11-15 |
| A | WO 2018235851 A1 (RAQUALIA PHARMA INC [JP]) 27 December 2018 (2018-12-27)<br>The whole document | 1-7, 11-15 |
| A | Lopes, A.B.; Miguez, E.; Kümmerle, A.E.; Rumjanek, V.M.; Fraga, C.A.M.; Barreiro, E.J. Characterization of Amide Bond Conformers for a Novel Heterocyclic Template of N-acylhydrazone Derivatives. Molecules 2013, 18, 11683-11704. https://doi.org/10.3390/molecules181011683 | 1-7, 11-15 |
| A | da Silva YK, Augusto CV, de Castro Barbosa ML, de Albuquerque Melo GM, de Queiroz AC, de Lima Matos Freire Dias T, Júnior WB, Barreiro EJ, Lima LM, Alexandre-Moreira MS. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893. | 1-7, 11-15 |
| A | US 2008300240 A1 (OMEROS CORP [US]) 04 December 2008 (2008-12-04)<br>The whole document | 1-7, 11-15 |

**INTERNATIONAL SEARCH REPORT**

International application No.
**PCT/BR2024/050027**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:

Claims 8-10 contain subject matter that falls under the provisions of PCT Rule 39.1(iv) concerning methods for treatment of the human or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/BR2024/050027**

| Patent document cited in search report | | | Publication date (day/month/year) | Patent family member(s) | | | Publication date (day/month/year) |
|---|---|---|---|---|---|---|---|
| WO | 2012129562 | A2 | 27 September 2012 | WO | 2012129562 | A3 | 31 January 2013 |
| | | | | US | 2017112843 | A1 | 27 April 2017 |
| | | | | US | 10166237 | B2 | 01 January 2019 |
| | | | | US | 2014113012 | A1 | 24 April 2014 |
| | | | | US | 9464065 | B2 | 11 October 2016 |
| WO | 2015048547 | A2 | 02 April 2015 | WO | 2015048547 | A3 | 18 June 2015 |
| | | | | US | 2015087673 | A1 | 26 March 2015 |
| WO | 03051366 | A2 | 26 June 2003 | WO | 03051366 | A3 | 25 March 2004 |
| | | | | US | 2003187026 | A1 | 02 October 2003 |
| | | | | US | 2003199511 | A1 | 23 October 2003 |
| | | | | US | 6831175 | B2 | 14 December 2004 |
| WO | 2006006569 | A1 | 19 January 2006 | NONE | | | |
| WO | 2012151567 | A1 | 08 November 2012 | US | 2014079666 | A1 | 20 March 2014 |
| | | | | US | 9328075 | B2 | 03 May 2016 |
| WO | 2006065204 | A1 | 22 June 2006 | EP | 1831170 | A1 | 12 September 2007 |
| | | | | EP | 1831170 | A4 | 14 October 2009 |
| | | | | JP | 2008523139 | A | 03 July 2008 |
| | | | | US | 2008287399 | A1 | 20 November 2008 |
| WO | 0151456 | A2 | 19 July 2001 | WO | 0151456 | A3 | 20 December 2001 |
| | | | | AT | E376996 | T1 | 15 November 2007 |
| | | | | AU | 3972501 | A | 24 July 2001 |
| | | | | CA | 2397575 | A1 | 19 July 2001 |
| | | | | DE | 60131138 | D1 | 13 December 2007 |
| | | | | DE | 60131138 | T2 | 14 August 2008 |
| | | | | EP | 1246795 | A2 | 09 October 2002 |
| | | | | EP | 1246795 | B1 | 31 October 2007 |
| | | | | ES | 2293980 | T3 | 01 April 2008 |
| | | | | JP | 2003519676 | A | 24 June 2003 |
| | | | | US | 2002045749 | A1 | 18 April 2002 |
| | | | | US | 6780858 | B2 | 24 August 2004 |
| | | | | US | 2004235911 | A1 | 25 November 2004 |
| | | | | US | 7053234 | B2 | 30 May 2006 |
| | | | | US | 2006270651 | A1 | 30 November 2006 |
| | | | | US | 7148259 | B1 | 12 December 2006 |
| WO | 2008096005 | A1 | 14 August 2008 | AR | 065288 | A1 | 27 May 2009 |
| | | | | AT | E542421 | T1 | 15 February 2012 |
| | | | | AU | 2008212764 | A1 | 14 August 2008 |
| | | | | AU | 2008212764 | B2 | 29 November 2012 |
| | | | | BR | PI0807864 | A2 | 17 June 2014 |
| | | | | BR | PI0807864 | A8 | 08 March 2016 |
| | | | | BR | PI0807864 | B1 | 02 February 2021 |
| | | | | CA | 2675627 | A1 | 14 August 2008 |
| | | | | CA | 2675627 | C | 22 April 2014 |
| | | | | CL | 2008000395 | A1 | 04 July 2008 |
| | | | | CR | 10984 | A | 15 October 2009 |
| | | | | CY | 1112576 | T1 | 10 February 2016 |
| | | | | DK | 2117297 | T3 | 21 May 2012 |
| | | | | EA | 200901081 | A1 | 26 February 2010 |
| | | | | EA | 017040 | B1 | 28 September 2012 |

Form PCT/ISA/210 (patent family annex) (July 2022)

**INTERNATIONAL SEARCH REPORT**
**Information on patent family members**

International application No.
**PCT/BR2024/050027**

| Patent document cited in search report | Publication date (day/month/year) | Patent family member(s) | Publication date (day/month/year) |
|---|---|---|---|
| | | EC SP099615 A | 30 October 2009 |
| | | EP 2117297 A1 | 18 November 2009 |
| | | EP 2117297 B1 | 25 January 2012 |
| | | ES 2378909 T3 | 19 April 2012 |
| | | HR P20120198 T1 | 31 March 2012 |
| | | JP 2010518050 A | 27 May 2010 |
| | | JP 5523838 B2 | 18 June 2014 |
| | | KR 20090118962 A | 18 November 2009 |
| | | KR 101488034 B1 | 30 January 2015 |
| | | MX 2009007733 A | 08 October 2009 |
| | | PE 20081754 A1 | 31 January 2009 |
| | | PL 2117297 T3 | 31 July 2012 |
| | | PT 2117297 E | 08 February 2012 |
| | | RS 52230 B | 31 October 2012 |
| | | SI 2117297 T1 | 30 March 2012 |
| | | TW 200901889 A | 16 January 2009 |
| | | UA 97973 C2 | 10 April 2012 |
| | | US 2010113543 A1 | 06 May 2010 |
| | | US 8859607 B2 | 14 October 2014 |
| | | ZA 200906209 B | 24 November 2010 |
| WO 2010099279 A1 | 02 September 2010 | AR 075635 A1 | 20 April 2011 |
| | | BR PI1008770 A2 | 25 August 2015 |
| | | BR PI1008770 B1 | 21 February 2017 |
| | | BR PI1008770 B8 | 31 July 2018 |
| | | CA 2750539 A1 | 02 September 2010 |
| | | EP 2421832 A1 | 29 February 2012 |
| | | EP 2421832 B1 | 18 December 2013 |
| | | MX 2011009024 A | 29 September 2011 |
| | | PL 2421832 T3 | 30 May 2014 |
| | | US 2010222221 A1 | 02 September 2010 |
| | | US 8536331 B2 | 17 September 2013 |
| | | US 2013324412 A1 | 05 December 2013 |
| | | US 8889694 B2 | 18 November 2014 |
| JP 2004203871 A | 22 July 2004 | NONE | |
| US 4607053 A | 19 August 1986 | AU 4254685 A | 21 November 1985 |
| | | AU 586973 B2 | 03 August 1989 |
| | | DE 3587460 D1 | 26 August 1993 |
| | | DE 3587460 T2 | 17 February 1994 |
| | | DK 217385 D0 | 15 May 1985 |
| | | DK 217385 A | 18 November 1985 |
| | | EP 0161939 A2 | 21 November 1985 |
| | | EP 0161939 A3 | 08 October 1986 |
| | | EP 0161939 B1 | 21 July 1993 |
| | | ES 8609201 A1 | 01 September 1986 |
| | | ES 8704872 A1 | 16 April 1987 |
| | | ES 8800129 A1 | 16 October 1987 |
| | | ES 8802378 A1 | 16 May 1988 |
| | | ES 8706604 A1 | 01 July 1987 |
| | | ES 8706605 A1 | 01 July 1987 |
| | | ES 8706606 A1 | 01 July 1987 |

Form PCT/ISA/210 (patent family annex) (July 2022)

**INTERNATIONAL SEARCH REPORT**
**Information on patent family members**

International application No.
**PCT/BR2024/050027**

| Patent document cited in search report | Publication date (day/month/year) | Patent family member(s) | Publication date (day/month/year) |
|---|---|---|---|
| | | FI 851970 L | 18 November 1985 |
| | | GR 851203 B | 25 November 1985 |
| | | HU T37742 A | 28 February 1986 |
| | | HU 200594 B | 28 July 1990 |
| | | IL 75223 A | 31 May 1988 |
| | | KR 850008660 A | 21 December 1985 |
| | | KR 910000855 B1 | 11 February 1991 |
| | | NZ 212099 A | 29 August 1989 |
| | | PH 21267 A | 28 September 1987 |
| CN 108503501 A | 07 September 2018 | CN 108503501 B | 21 September 2021 |
| WO 2020138271 A1 | 02 July 2020 | BR 112021008995 A2 | 10 August 2021 |
| | | CA 3120268 A1 | 02 July 2020 |
| | | CN 113164461 A | 23 July 2021 |
| | | EP 3902544 A1 | 03 November 2021 |
| | | JP 2022515630 A | 21 February 2022 |
| | | KR 20210107698 A | 01 September 2021 |
| | | MX 2021007425 A | 05 August 2021 |
| | | TW 202038950 A | 01 November 2020 |
| | | US 2022048892 A1 | 17 February 2022 |
| WO 2018235851 A1 | 27 December 2018 | AU 2018289731 A1 | 14 November 2019 |
| | | AU 2018289731 B2 | 18 November 2021 |
| | | BR 112019021736 A2 | 05 May 2020 |
| | | CA 3059687 A1 | 27 December 2018 |
| | | CN 110612285 A | 24 December 2019 |
| | | CN 110612285 B | 04 April 2023 |
| | | DK 3487839 T3 | 18 January 2021 |
| | | EP 3487839 A1 | 29 May 2019 |
| | | EP 3487839 A4 | 18 March 2020 |
| | | EP 3487839 B1 | 23 December 2020 |
| | | ES 2851004 T3 | 02 September 2021 |
| | | HU E053460 T2 | 28 June 2021 |
| | | JP 6592702 B1 | 23 October 2019 |
| | | JP 2019532911 A | 14 November 2019 |
| | | KR 20200015888 A | 13 February 2020 |
| | | KR 102577137 B1 | 11 September 2023 |
| | | MX 2019012635 A | 11 December 2019 |
| | | PH 12019502850 A1 | 28 September 2020 |
| | | PL 3487839 T3 | 14 June 2021 |
| | | PT 3487839 T | 11 February 2021 |
| | | RU 2019133530 A | 20 July 2021 |
| | | RU 2019133530 A3 | 04 August 2021 |
| | | RU 2768149 C2 | 23 March 2022 |
| | | SG 11201909793Y A | 28 November 2019 |
| | | TW 201904944 A | 01 February 2019 |
| | | TW I769266 B | 01 July 2022 |
| | | US 2020016135 A1 | 16 January 2020 |
| | | US 11154544 B2 | 26 October 2021 |
| | | ZA 201906888 B | 24 February 2021 |
| US 2008300240 A1 | 04 December 2008 | US 7786139 B2 | 31 August 2010 |
| | | US 2011021509 A1 | 27 January 2011 |

Form PCT/ISA/210 (patent family annex) (July 2022)

**INTERNATIONAL SEARCH REPORT**
**Information on patent family members**

International application No.
**PCT/BR2024/050027**

| Patent document cited in search report | Publication date (day/month/year) | Patent family member(s) | Publication date (day/month/year) |
|---|---|---|---|
| | | US 8278327 B2 | 02 October 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B


### Non-patent literature cited in the description


- **SCHAIBLE.** *Langenbecks Arch. Surg.*, 2004, vol. 389, 237
- **SMITH.** *Pain.*, 2020, vol. 161 (1), S127
- **CAVALLI.** *Int. J. Immunopathol. Pharmacol.*, 2019, vol. 33, 2058738419838383
- **BOUHASSIRA.** *Rev Neurol (Paris).*, 2019, vol. 175 (1-2), 16
- **SCHOLZ.** *Nature Neurosci.*, 2002, vol. 5, 1062
- **COSTIGAN.** *Annu. Rev. Neurosci.*, 2009, vol. 32 (1)
- **DWORKIN.** *Clin. J. Pain*, 2002, vol. 18 (6), 343
- **DUCREUX.** *Brain*, 2006, vol. 129, 963
- **PAK.** *Curr. Pain Headache Rep.*, 2018, vol. 22 (2), 9
- **KUSHNAREV.** *Expert Opin. Investig. Drugs*, 2020, vol. 29 (3), 259
- **EMERY.** *Expert Opin. Ther. Targets*, 2016, vol. 20 (8), 975
- **CATTERALL.** *Nat. Chem. Biol.*, 2020, vol. 16, 1314
- **WISEDCHAISRI.** *Cell.*, 2019, vol. 178 (4), 993
- **CLAIRFEUILLE.** *Science*, 2019, vol. 363, 1302
- **LERA-RUIZ.** *J. Med. Chem.*, 2015, vol. 58 (18), 7093
- **BAGAL.** *J. Med. Chem.*, 2013, vol. 56 (3), 593
- **BAGAL.** *Channels*, 2015, vol. 9 (6), 360
- **LAW.** *Drug Discovery Today*, 2019, vol. 24 (7), 1389
- **BAGAL.** *Channels (Austin)*, 2015, vol. 9 (6), 360
- **VETTER.** *Pharmacology & Therapeutics*, 2017, vol. 172, 73
- **AHUJA.** *Science*, 2015, vol. 350 (6267), 1491
- **KINGWELL.** *Nat. Rev. Drug Discov.*, 2019, vol. 18, 321
- **SAFINA.** *J. Med. Chem.*, 2021, vol. 64, 2953
- **LUO.** *J. Med. Chem.*, 2019, vol. 62, 831
- **BANKAR**. *Cell Reports*, 2018, vol. 24, 3133
- **BROWN.** *Bioorg. Med. Chem.*, 2019, vol. 27 (1), 230
- **PAYNE.** *Br. J. Pharmacol.*, 2015, vol. 172 (10), 2654
- **BAGAL.** *Med. Chem. Lett.*, 2015, vol. 6 (6), 650
- **KORT.** *J. Med. Chem.*, 2008, vol. 51, 407
- **ZHANG.** *Neuropharmacology*, 2010, 59, 102, 207
- **BAGAL**. *Channels (Austin)*, 2015, vol. 9 (6), 360
- **KORNECOOK.** *J. Pharmacol. Exp. Ther.*, 2017, vol. 362, 146
- **DEUIS.** *Neuropharmacology*, 2017, 127, 87, 108
- **MCKERRALL.** *Bioorganic & Medicinal Chemistry Lett.*, 2018, vol. 28, 3141
- **BAGAL**. *Bioorganic & Medicinal Chemistry Lett.*, 2014, vol. 24, 3690